(19) Europäisches Patentamt
European Patent Office
Office européen des brevets

(11) Veröffentlichungsnummer: **0 270 947 B1**

(12) # EUROPÄISCHE PATENTSCHRIFT

(45) Veröffentlichungstag der Patentschrift: **19.05.93**

(51) Int. Cl.[5]: **C07C 217/00**, C07C 261/04, C07C 229/00, C07C 233/00, C07C 255/00, C07C 275/00, C07C 317/00

(21) Anmeldenummer: **87117549.3**

(22) Anmeldetag: **27.11.87**

Die Akte enthält technische Angaben, die nach dem Eingang der Anmeldung eingereicht wurden und die nicht in dieser Patentschrift enthalten sind.

(54) **Substituierte basische 2–Aminotetraline.**

(30) Priorität: **10.12.86 DE 3642192**
**01.06.87 DE 3718317**

(43) Veröffentlichungstag der Anmeldung:
**15.06.88 Patentblatt 88/24**

(45) Bekanntmachung des Hinweises auf die Patenterteilung:
**19.05.93 Patentblatt 93/20**

(84) Benannte Vertragsstaaten:
**AT BE CH DE ES FR GB GR IT LI NL SE**

(56) Entgegenhaltungen:
**EP–A– 0 026 848**
**EP–A– 0 161 604**
**EP–A– 0 236 930**
**FR–A– 2 323 388**
**FR–A– 2 391 189**

**CHEMICAL ABSTRACTS, Band 106, Nr. 5, 2. Februar 1987, Seite 94, Spalte 1, Abstract Nr. 28103s, Columbus, Ohio, USA; M.B. EMERIT et al.: "Irreversible blockade of central 5–HT1A receptor binding sites by the photoaffinity probe 8–methoxy–3'–NAP–amino–PAT"**

(73) Patentinhaber: **BAYER AG**

**W–5090 Leverkusen 1 Bayerwerk(DE)**

(72) Erfinder: **Schohe, Rudolf, Dr.**
**Pahlkestrasse 5**
**W–5600 Wuppertal(DE)**
Erfinder: **Glaser, Thomas, Dr.**
**Koesliner Strasse 21a**
**W–5064 Roesrath(DE)**
Erfinder: **Traber, Jörg, Dr.**
**Loewenburgstrasse 12**
**W–5204 Lohmar 21(DE)**
Erfinder: **Allen, George S., Prof. Dr.**
**Vanderbilt University Dept. Neurological Surgery**
**Nashville Tennessee 37 232(US)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die Erfindung betrifft substituierte basische 2-Amino-tetraline, Verfahren zu ihrer Herstellung und ihre Verwendung in Arzneimitteln.

Aus der EP-A1-41 488 ist bekannt, daß am Stickstoff mono- oder dialkylsubstituierte 2-Aminotetraline auf das Zentralnervensystem wirken.

Ebenso ist bekannt [Biochem. Pharmacol. 34 (6), 883 - 92], daß 2-(N-2'-Chloropropyl-N-propyl)amino-8-methoxy-1,2,3,4-tetrahydronaphthalin Hydrochlorid irreversibel an verschiedenen 5-HT-Rezeptor-Typen bindet.

Darüberhinaus ist 2-(N-3-Hydroxypropyl-N-propyl)amino-8-methoxy-1,2,3,4-tetrahydronaphthalin als Zwischenprodukt zur Herstellung von ZNS-wirksamen Stoffen bekannt [Eur. J. Pharm. 127, 67 - 81, 1986].

In ER 2 391 189 werden 2-Amino-tetraline beschrieben, deren Aminogruppe durch eine Benzoyloxy-alkylengruppe substituiert ist. Die Stoffe haben antispasmodische Wirkung auf die glatte Muskulatur.

EP 236 930 offenbart $5-HT_{1A}$-agonistische 2-Aminotetraline, deren Aminogruppe durch eine Saccharinylalkylgruppe substituiert ist.

Es wurden neue substituierte basische 2-Aminotetraline der allgemeinen Formel (I)

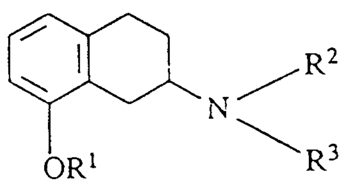

(I)

worin

$R^1$ - für Wasserstoff oder Methyl steht,

$R^2$ - für Wasserstoff, Methyl, Ethyl, Propyl, Isopropyl, Acetyl oder Propionyl steht,

und

$R^3$ - für Chinuclidin oder eine Gruppe der Formel
$-(CH_2)_a-R^4$ , $CH_2-CH=CH-(CH_2)_b-R^4$,
$-CH_2-C{\equiv}C-(CH_2)_b-R^4$,

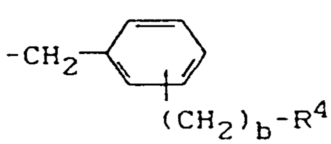

oder

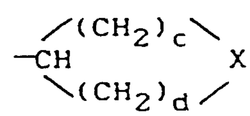

steht,

worin

a - eine Zahl 1 bis 6 bedeutet,

b - eine Zahl 0, 1 oder 2 bedeutet,

c - eine Zahl 1 oder 2 bedeutet,

d - die Zahl 2 bedeutet,

X - die Gruppe $NR^5$ bedeutet, wobei

$R^5$ - für Wasserstoff, Methyl, Ethyl, Propyl, Isopropyl, Butyl, Isobutyl, Phenyl, Benzyl, Methoxycarbonyl, Ethoxycarbonyl, Propoxycarbonyl, Isopropoxycarbonyl, Methylsulfonyl, Ethylsulfonyl oder Carbamoyl steht,

und

$R^4$ - Cyano oder
- eine Gruppe der Formel
$-OR^6$, $-COOR^7$, $-CONR^8R^9$, $-SO_mR^{10}$,
$-NR^{11}R^{12}$,

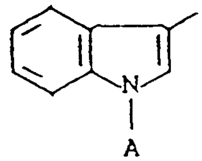

oder

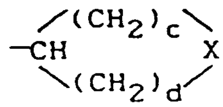

bedeutet,

wobei

c,d und X die oben angegebene Bedeutung haben,

A – für Wasserstoff, Methylsulfonyl, Phenylsulfonyl, Tolylsulfonyl Methoxycarbonyl oder Ethoxycarbonyl steht,

$R^6$ – für Wasserstoff, Methyl, Ethyl, Propyl, Isopropyl, Butyl, Isobutyl, Methoxycarbonyl, Ethoxycarbonyl, Propoxycarbonyl, Isopropoxycarbonyl, Butoxycarbonyl, Isobutoxycarbonyl, Tetrahydronaphthalin-1-yl oder 1,2,3-Benzothiadiazol-6-yl steht,

$R^7$ – für Wasserstoff, Methyl, Ethyl, Propyl, Isopropyl, Butyl oder Isobutyl steht,

$R^8$ und $R^9$ gleich oder verschieden sind

und

– für Wasserstoff, Methyl, Ethyl, Propyl, Isopropyl, Butyl oder Isobutyl stehen,

$R^{10}$ – für Methyl, Ethyl, Propyl, Isopropyl, Butyl, Isobutyl, oder

– für gegebenenfalls durch Fluor, Chlor, Methyl, Ethyl, Propyl oder Isopropyl substituiertes Phenyl steht,

m – für eine Zahl 0, 1 oder 2 steht,

$R^{11}$ und $R^{12}$ gleich oder verschieden sind,

und

– für Wasserstoff, Methyl, Ethyl, Propyl, Isopropyl, Butyl, Isobutyl, oder

– für gegebenenfalls durch Fluor, Chlor, Methyl oder Methoxy subsituiertes Phenyl, oder

– für eine Gruppe $-COR^{13}$ oder $-SO_2R^{14}$ stehen,

worin

$R^{13}$ – eine Gruppe $NHR^{15}$ bedeutet, oder

– Methyl, Ethyl, Propyl, Isopropyl, Methoxy, Ethoxy, Propoxy, Isopropoxy, oder

– gegebenenfalls durch Methyl, Methoxy, Fluor oder Chlor substituiertes Phenyl, Benzyl, Benzyloxy, Thienyl, Furyl, Pyridyl, Pyrimidyl, Chinolyl oder Isochinolyl bedeutet,

$R^{14}$ – gegebenenfalls durch Fluor, Chlor, Methoxycarbonyl, Ethoxycarbonyl, Propoxycarbonyl, Isopropoxycarbonyl, Butoxycarbonyl oder Isobutoxycarbonyl substituiertes Methyl, Ethyl, Propyl, Isopropyl, Butyl oder Isobutyl bedeutet, oder

– gegebenenfalls durch Methyl, Ethyl, Propyl, Isopropyl, Methoxy, Fluor oder Chlor substituiertes Phenyl, Thienyl, Furyl, Pyridyl, Pyrimidyl, Chinolyl oder Isochinolyl bedeutet, oder

– eine Gruppe $NR^8R^9$ bedeutet,

wobei

$R^8$ und $R^9$ die oben angegebene Bedeutung haben,

und

$R^{15}$ – gegebenenfalls durch Fluor oder Chlor substituiertes Methyl, Ethyl, Propyl, Isopropyl, Butyl, Isobutyl, Pentyl, Isopentyl, Hexyl oder Isohexyl bedeutet, oder

– Phenyl bedeutet, das durch Fluor, Chlor, Methyl oder Methoxy substituiert sein kann,

oder

$R^{11}$ und $R^{12}$ gemeinsam mit dem Stickstoffatom einen Ring der Reihe

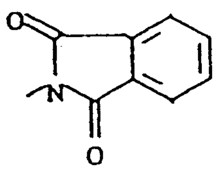, 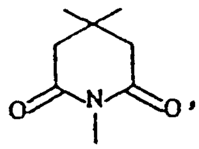, 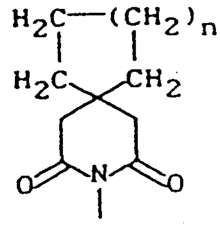,

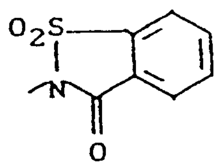, 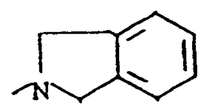, 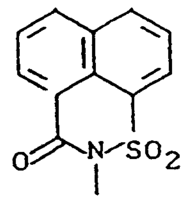

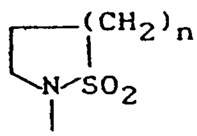, 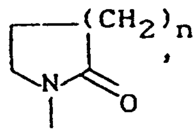, 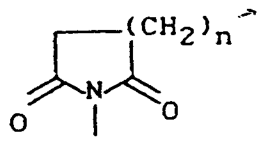

oder 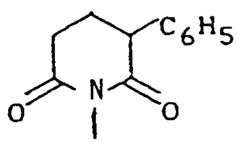

bilden,
worin

n − eine Zahl 1 oder 2 bedeutet

oder

in welcher

$R^2$ und $R^3$ gemeinsam mit dem Stickstoffatom eine Gruppe der Formel

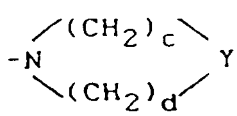

bilden,
worin

c und d die oben angegebene Bedeutung haben,
und

Y − eine Gruppe der Formel $NR^5$ oder $CH(CH_2)_e-NHR^5$ bedeutet,
wobei

$R^5$ die oben angegebene Bedeutung hat
und

e − für eine Zahl 1 oder 2 steht

wobei jedoch

$R^3$ nicht 3 − Hydroxypropyl bedeutet, wenn

$R^1$ − für Methyl und $R^2$ für Propyl steht,

$R^3$ nicht 2 − Methylthioethyl bedeutet, wenn

$R^1$ − für Wasserstoff oder Methyl und $R^2$ für Wasserstoff, Propyl oder Propionyl steht,

und

$R^2$ − nicht für Wasserstoff oder Methyl steht,

wenn

$R^1$ − für Wasserstoff oder Methyl steht und

$R^3$ – für einen Rest der Formel

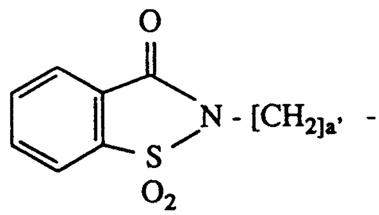

steht
worin
a' eine Zahl 2 bis 5 bedeutet,

und deren Salze gefunden.

Die erfindungsgemäßen Stoffe zeigen überraschenderweise eine gute Wirkung auf das Zentralnervensystem und können zu therapeutischen Behandlung von Menschen und Tieren verwendet werden.

Die erfindungsgemäßen Stoffe haben mehrere asymmetrische Kohlenstoffatome und können daher in verschiedenen stereochemischen Formen existieren. Darüberhinaus können Verbindungen mit einer Sulfoxidgruppe ebenfalls in unterschiedlichen stereochemischen Formen existieren. Sowohl die einzelnen Isomeren, als auch deren Mischungen sind Gegenstand der Erfindung. Beispielsweise seien folgende isomeren Formen der substituierten basischen 2-Aminotetraline genannt:

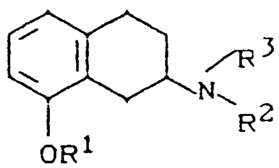

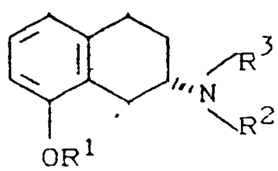

Die erfindungsgemäßen substituierten basischen 2-Aminotetraline können auch in Form ihrer Salze vorliegen. Im allgemeinen seien hier Salze mit anorganischen oder organischen Säuren genannt.

Im Rahmen der vorliegenden Erfindung werden physiologisch unbedenkliche Salze bevorzugt. Physiologisch unbedenkliche Salze der substituierten basischen 2-Aminotetraline können Salze der erfindungsgemäßen Stoffe mit Mineralsäuren, Carbonsäuren oder Sulfonsäuren sein. Besonders bevorzugt sind beispielsweise Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Oxalsäure, Malonsäure, Bernsteinsäure, Maleinsäure, Fumarsäure, Äpfelsäure, Weinsäure, Zitronensäure oder Benzoesäure.

Bevorzugt werden Verbindungen der allgemeinen Formel (I), in welcher

$R^1$ – für Wasserstoff oder Methyl steht,

$R^2$ – für Wasserstoff, Methyl, Propyl, Acetyl oder Propionyl steht,

und

$R^3$ – für Chinuclidin oder eine Gruppe der Formel
$-(CH_2)_a-R^4$ , $-CH_2-CH=CH-(CH_2)_b-R^4$,
$-CH_2-C\equiv C-(CH_2)_b-R^4$,

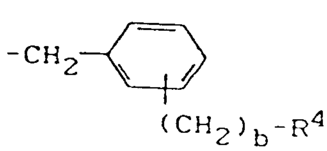 oder 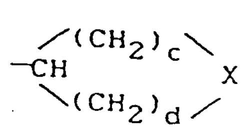

steht,
worin
a – eine Zahl 1 bis 6 bedeutet,
b – eine Zahl 0 oder 1 bedeutet,
c – die Zahl 2 bedeutet,
d – die Zahl 2 bedeutet,
x – die Gruppe $NR^5$ bedeutet,
wobei
$R^5$ – für Wasserstoff, Methyl oder Methoxycarbonyl steht,
$R^4$ – Cyano oder
– eine Gruppe der Formel
$-OR^6$, $-COOR^7$, $-CONR^8R^9$, $SO_mR^{10}$ oder
$NR^{11}R^{12}$,

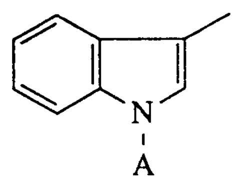

bedeutet,
wobei
A – für Wasserstoff oder Tolylsulfonyl steht,
$R^6$ – für Wasserstoff, Methyl, Methoxycarbonyl, Ethoxycarbonyl, Tetrahydronaphthalin-1-yl oder 1,2,3-1,2,3-Benzothiadiazol-6-yl steht,
$R^7$ – für Methyl oder Ethyl steht,
$R^8$ und $R^9$ gleich oder verschieden sind und
– für Wasserstoff, Methyl oder Ethyl stehen,
$R^{10}$ – für Phenyl steht,
m – für eine Zahl 0, 1 oder 2 steht,
$R^{11}$ und $R^{12}$ gleich oder verschieden sind,
und
– für Wasserstoff, Methyl oder Ethyl, oder
– für eine Gruppe $-COR^{13}$ oder $SO_2R^{14}$ stehen,
worin
$R^{13}$ – eine Gruppe $NHR^{15}$ bedeutet,
oder
Pyridyl oder Adamantyl bedeutet,
$R^{14}$ – gegebenenfalls durch Chlor, Methoxycarbonyl oder Ethoxycarbonyl, substituiertes Methyl, Ethyl, Propyl, Isopropyl oder Butyl bedeutet, oder
– gegebenenfalls durch Methyl, oder Fluor substituiertes Phenyl bedeutet, oder
– eine Gruppe $NR^8R^9$ bedeutet,
wobei
$R^8$ und $R^9$ die oben angegebene Bedeutung haben,
und
$R^{15}$ – gegebenenfalls durch Chlor substituiertes Butyl oder Hexyl bedeutet,
oder
$R^{11}$ und $R^{12}$ gemeinsam mit dem Stickstoffatom einen Ring der Reihe

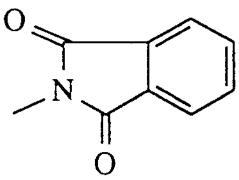 , 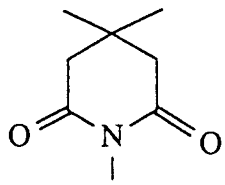 , 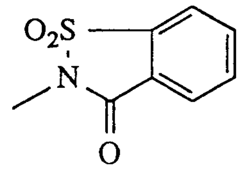 ,

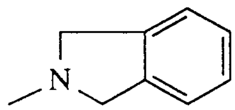 , 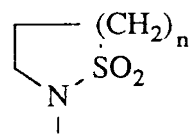 oder 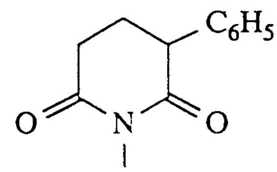

bilden,
worin
n – eine Zahl 1 oder 2 bedeutet,
oder
in welcher
$R^2$ und $R^3$ gemeinsam mit dem Stickstoffatom eine Gruppe der Formel

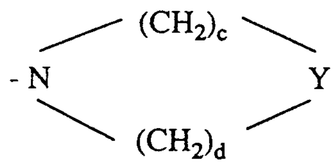

bilden,
worin
c und d die oben angegebene Bedeutung haben,
und
Y – eine Gruppe der Formel $NR^5$ oder $CH(CH_2)_e-NHR^5$ bedeutet,
wobei
$R^5$ die oben angegebene Bedeutung hat
und
e – für eine Zahl 1 oder 2 steht
wobei jedoch
$R^3$ nicht 3-Hydroxypropyl bedeutet, wenn
$R^1$ – für Methyl und $R^2$ für Propyl steht,
$R^3$ nicht 2-Methylthioethyl bedeutet, wenn
$R^1$ – für Wasserstoff oder Methyl und $R^2$ für Wasserstoff, Propyl oder Propionyl steht,
und
$R^2$ – nicht für Wasserstoff oder Methyl steht und
wenn
$R^1$ – für Wasserstoff oder Methyl steht und
$R^3$ – für einen Rest der Formel

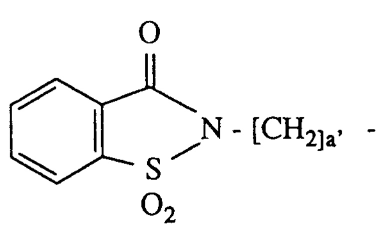

steht
worin
a' eine Zahl 2 bis 5 bedeutet,
und deren Salze.

Besonders bevorzugt sind Verbindungen der allgemeinen Formel (I), die einen basischen Stickstoff enthalten. Unter basischem Stickstoff werden Stickstoffgruppen, z.B. Aminogruppen verstanden, die nicht desaktiviert sind. Eine Stickstoffgruppe kann durch elektronenanziehende Gruppen desaktiviert werden. Solche desaktivierende Gruppen können Acyl oder Sulfonylgruppen sein, die am Stickstoff gebunden sind. Hierzu gehören bevorzugt Alkyl-, Aryl- oder Aralkylcarbonylgruppen, Alkyl-, Aryl- oder Aralkylsulfonyl- oder -sulfamoylgruppen, Carboxy, Carbamoyl oder Alkoxy-, Aryloxy- oder Aralkoxycarbonylgruppen. Beispielhaft seien folgende substituierte basische 2-Aminotetraline genannt:

N-6-Chlorhexyl-N'-{3-[N-(8-methoxy-1,2,3,4-tetrahydro-2-naphthyl)-N-propyl]-amino}propylharnstoff
8-Methoxy-2-(N-propyl-N-(3-phthalimidoyl-propyl))amino-1,2,3,4-tetrahydronaphthalin
2-(2-Ethoxycarbonylamido-ethyl)amino-8-methoxy-1,2,3,4-tetrahydronaphthalin
2-[N-(Diethylcarbonamidoethyl)-N-propyl]amino-8-methoxy-1,2,3,4-tetrahydronaphthalin
2-{N-[3-(4-Fluorbenzolsulfonamido)propyl]-N-propyl}amino-8-methoxy-1,2,3,4-tetrahydronaphthalin
2-[N-(3-Aminopropyl)-N-propyl]amino-8-methoxy-1,2,3,4-tetrahydronaphthalin
8-Methoxy-2-[N-(2-toluolsulfonamidoethyl)-N-propyl]amino-1,2,3,4-tetrahydronaphthalin
2-{4-[N-(8-Methoxy-1,2,3,4-tetrahydronaphthalin-2-yl)-N-propyllamino-butyl}-1,2-benzisothiazol-3(2H)-on-1,1-dioxid
2-[N-(2-Methansulfonamido-ethyl)-N-propyl]amino-8-methoxy-1,2,3,4-tetrahydronaphthalin
2-[N-(2-Aminoethyl)-N-propyl]amino-8-methoxy-1,2,3,4-tetrahydronaphthalin
2-(N-Ethoxycarbonylmethyl-N-propyl)amino-8-methoxy-1,2,3,4-tetrahydronaphthalin
2-(N-Cyanomethyl-N-propyl)amino-8-methoxy-1,2,3,4-tetrahydronaphthalin
N-{2-[N-(8-Methoxy-1,2,3,4-tetrahydronaphthalin-2-yl)-N-propyl]amino}ethyl-N'-phenylharnstoff
8-Methoxy-2-[N-propyl-N-(2-nicotinoylamino-ethyl)]amino-1,2,3,4-tetrahydronaphthalin
2-{N-[2-(3-Chlorpropylsulfonamido)ethyl]-N-propyl}amino-8-methoxy-1,2,3,4-tetrahydronaphthalin
2-{N-[2-(4-Chlorbutylsulfonamido)ethyl]-N-propyl}amino-8-methoxy-1,2,3,4-tetrahydronaphthalin
2-[N-(2-Dimethylaminosulfonylamido)ethyl-N-propyl]amino-8-methoxy-1,2,3,4-tetrahydronaphthalin
2-[N-(2-Cyanoethyl)-N-propyl]amino-8-methoxy-1,2,3,4-tetrahydronaphthalin
2-[N-(2-Carbonamido-ethyl)-N-propyl]amino-8-methoxy-1,2,3,4-tetrahydronaphthalin
2-[N-(2-Ethyl-carbonyldioxy-ethyl)-N-propyl]amino-8-methoxy-1,2,3,4-tetrahydronaphthalin
2-{2-[N-Propyl-N-(8-methoxy-1,2,3,4-tetrahydronaphthalin-2-yl)]amino}ethyl-perhydrothiazin-1,1-dioxid
2-{2-[N-propyl-N-(8-methoxy-1,2,3,4-tetrahydronaphthalin-2-yl)]amino}ethyl-isothiazolidin-1,1-dioxid
2-(4-Methylpiperazin-1-yl)-8-methoxy-1,2,3,4-tetrahydronaphthalin
2-(N-Chinuclidin-3-yl-N-propyl)amino-8-methoxy-1,2,3,4-tetrahydronaphthalin
2-(N-Diethylcarbonamidomethyl-N-propyl)amino-8-methoxy-1,2,3,4-tetrahydronaphthalin
2-[N-(2-Methoxycarbonylamido-ethyl)-N-propyl]amino-8-methoxy-1,2,3,4-tetrahydronaphthalin
2-(2-Diethylaminoethyl)amino-8-methoxy-1,2,3,4-tetrahydronaphthalin
2-[N-(2-Methylaminoethyl)-N-propyl]amino-8-methoxy-1,2,3,4-tetrahydronaphthalin
2-[N-(2-Diethylaminoethyl)-N-propyl]amino-8-methoxy-1,2,3,4-tetrahydronaphthalin
2-(1-Ethoxycarbonyl-piperidin-4-yl)amino-8-methoxy-1,2,3,4-tetrahydronaphthalin
2-(4-Ethoxycarbonylaminomethyl)piperidin-1-yl-8-methoxy-1,2,3,4-tetrahydronaphthalin
2-(3-Dimethylaminopropyl)amino-8-methoxy-1,2,3,4-tetrahydronaphthalin
2-[N-(3-Dimethylaminopropyl)-N-propyl]amino-8-methoxy-1,2,3,4-tetrahydronaphthalin
2-(1-Methylpiperidin-4-yl)amino-8-methoxy-1,2,3,4-tetrahydronaphthalin
2-[N-(3-Dimethylaminopropyl)-N-propionyl]amino-8-methoxy-1,2,3,4-tetrahydronaphthalin
2-[N-(2-Diethylaminoethyl)-N-acetyl]amino-8-methoxy-1,2,3,4-tetrahydronaphthalin
N-6-Chlorhexyl-N'-{3-[N-(8-methoxy-1,2,3,4-tetrahydro-2-naphthyl)-N-propyl]-amino}propylharnstoff Hydrochlorid

8 – Methoxy – 2 – [N – propyl – N – (3 – phthalimidoyl – propyl)]amino – 1,2,3,4 – tetrahydronaphthalin Hydro – chlorid
2 – (2 – Ethoxycarbonylamido – ethyl)amino – 8 – methoxy – 1,2,3,4 – tetrahydronaphthalin Hydrochlorid
2 – [N – (Diethylcarbonamidoethyl) – N – propyl]amino – 8 – methoxy – 1,2,3,4 – tetrahydronaphthalin Hydro – chlorid
2 – {N – [3 – (4 – Fluorbenzolsulfonamido)propyl] – N – propyl}amino – 8 – methoxy – 1,2,3,4 – tetrahydronaphthalin Hydrochlorid
8 – Methoxy – 2 – [N – (2 – toluolsulfonamidoethyl) – N – propyl]amino – 1,2,3,4 – tetrahydronaphthalin Hydro – chlorid
2 – [N – (2 – Methansulfonamido – ethyl) – N – propyl]amino – 8 – methoxy – 1,2,3,4 – tetrahydronaphthalin Hydrochlorid
2 – [N – (2 – Aminoethyl) – N – propyl]amino – 8 – methoxy – 1,2,3,4 – tetrahydronaphthalin Dihydrochlorid
2 – (N – Cyanomethyl – N – propyl)amino – 8 – methoxy – 1,2,3,4 – tetrahydronaphthalin Hydrochlorid
N – {2 – [N – (8 – Methoxy – 1,2,3,4 – tetrahydronaphthalin – 2 – yl) – N – propyl]amino}ethyl – N' – phenylharnstoff Hydrochlorid
8 – Methoxy – 2 – [N – propyl – N – (2 – nicotinoylamino – ethyl)]amino – 1,2,3,4 – tetrahydronaphthalin Hydro – chlorid
2 – {N – [2 – (3 – Chlorpropylsulfonamido)ethyl] – N – propyl}amino – 8 – methoxy – 1,2,3,4 – tetrahydronaphthalin Dihydrochlorid
2 – {N – [2 – (4 – Chlorbutylsulfonamido)ethyl] – N – propyl}amino – 8 – methoxy – 1,2,3,4 – tetrahydronaphthalin Hydrochlorid
2 – [N – (2 – Dimethylaminosulfonamido)ethyl – N – propyl]amino – 8 – methoxy – 1,2,3,4 – tetrahydronaphthalin Hydrochlorid
2 – [N – (2 – Cyanoethyl) – N – propyl]amino – 8 – methoxy – 1,2,3,4 – tetrahydronaphthalin Hydrochlorid
2 – [N – (2 – Carbonamido – ethyl) – N – propyl]amino – 8 – methoxy – 1,2,3,4 – tetrahydronaphthalin Hydro – chlorid
2 – [N – (2 – Ethyl – carbonyldioxy – ethyl) – N – propyl]amino – 8 – methoxy – 1,2,3,4 – tetrahydronaphthalin Hydrochlorid
2 – {2 – [N – Propyl – N – (8 – methoxy – 1,2,3,4 – tetrahydronaphthalin – 2 – yl)]amino}ethyl – perhydrothiazin – 1,1 – dioxid Hydrochlorid
2 – {2 – [N – Propyl – N – (8 – methoxy – 1,2,3,4 – tetrahydronaphthalin – 2 – yl)]amino}ethyl – isothiazolidin – 1,1 – dioxid Hydrochlorid
2 – (4 – Methylpiperazin – 1 – yl) – 8 – methoxy – 1,2,3,4 – tetrahydronaphthalin Hydrochlorid
2 – (N – Chinuclidin – 3 – yl – N – propyl)amino – 8 – methoxy – 1,2,3,4 – tetrahydronaphthalin Dihydrochlorid
2 – (N – Diethylaminocarbonamidomethyl – N – propyl)amino – 8 – methoxy – 1,2,3,4 – tetrahydronaphthalin Dihydrochlorid
2 – [N – (2 – Methoxycarbonylamido – ethyl) – N – propyl]amino – 8 – methoxy – 1,2,3,4 – tetrahydronaphthalin Hydrochlorid
2 – (2 – Diethylaminoethyl)amino – 8 – methoxy – 1,2,3,4 – tetrahydronaphthalin Hydrochlorid
2 – [N – (2 – Methylaminomethyl) – N – propyl]amino – 8 – methoxy – 1,2,3,4 – tetrahydronaphthalin Dihydro – chlorid
2 – [N – (2 – Diethylaminoethyl) – N – propyl)amino – 8 – methoxy – 1,2,3,4 – tetrahydronaphthalin Dihydro – chlorid
2 – (1 – Carbethoxypiperidin – 4 – yl)amino – 8 – methoxy – 1,2,3,4 – tetrahydronaphthalin Dihydrochlorid
2 – (4 – Ethoxycarbonylamido – methyl)piperidin – 1 – yl – 8 – methoxy – 1,2,3,4 – tetrahydronaphthalin Hydro – chlorid
2 – (3 – Dimethylaminopropyl)amino – 8 – methoxy – 1,2,3,4 – tetrahydronaphthalin Hydrochlorid
2 – [N – (3 – Dimethylaminopropyl) – N – propyl)amino – 8 – methoxy – 1,2,3,4 – tetrahydronaphthalin Dih – ydrochlorid
2 – (1 – Methylpiperidin – 4 – yl)amino – 8 – methoxy – 1,2,3,4 – tetrahydronaphthalin Dihydrochlorid
2 – [N – (3 – Dimethylaminopropyl) – N – propionyl]amino – 8 – methoxy – 1,2,3,4 – tetrahydronaphthalin Dih – ydrochlorid
2 – [N – (2 – Diethylaminoethyl) – N – acetyl]amino – 8 – methoxy – 1,2,3,4 – tetrahydronaphthalin Hydrochlorid

Weiterhin wurde ein Verfahren zur Herstellung der erfindungsgemäßen substituierten basischen 2 – Amino – tetralinen der allgemeinen Formel (I)

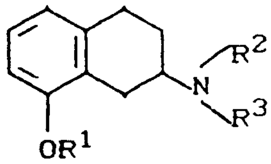

(I),

in welcher

$R^1$, $R^2$ und $R^3$ die angegebene Bedeutung haben,

und deren Salze

gefunden, das dadurch gekennzeichnet ist, daß man Tetralone der allgemeinen Formel (II)

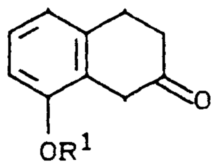

(II)

in welcher

$R^1$ – die angegebene Bedeutung hat,

mit Aminen der allgemeinen Formel (III)

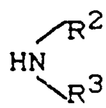

(III),

in welchen

$R^2$ und $R^3$ die angegebene Bedeutung haben,

$R^2$ jedoch nicht für Acyl stehen darf,

in inerten Lösemitteln, gegebenenfalls in Anwesenheit von Hilfsstoffen umsetzt,

dann die Zwischenprodukte in inerten Lösemitteln reduziert,

dann im Fall der Herstellung der Acylverbindungen ($R^2$ = Acyl) mit einem Acylierungsmittel umsetzt,

dann gegebenenfalls funktionelle Gruppen durch Reduktion, Hydrolyse, Oxidation oder Umsetzung mit elektrophilen Reagenzien in andere funktionelle Gruppen überführt

und dann im Fall der Herstellung der Salze, mit der entsprechenden Säure umsetzt.

Das erfindungsgemäße Verfahren kann für den Fall der Umsetzung mit cyclischen Aminen nach Reaktionsschema (a) und für den Fall der Umsetzung mit offenkettigen Aminen nach Reaktionsschema (b) beschrieben werden:

a)

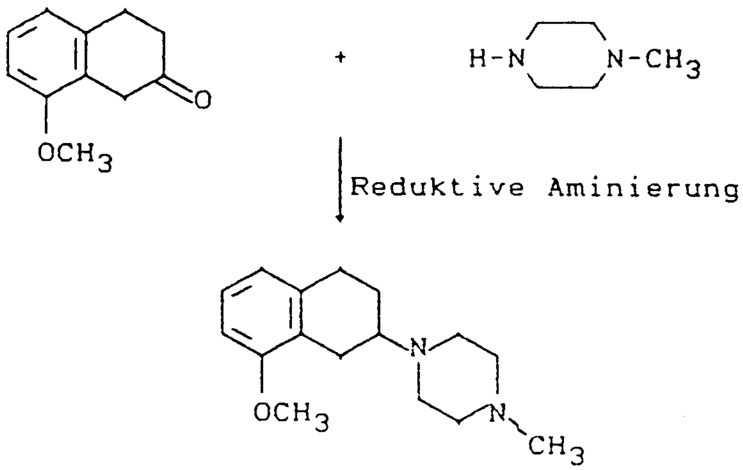


b)

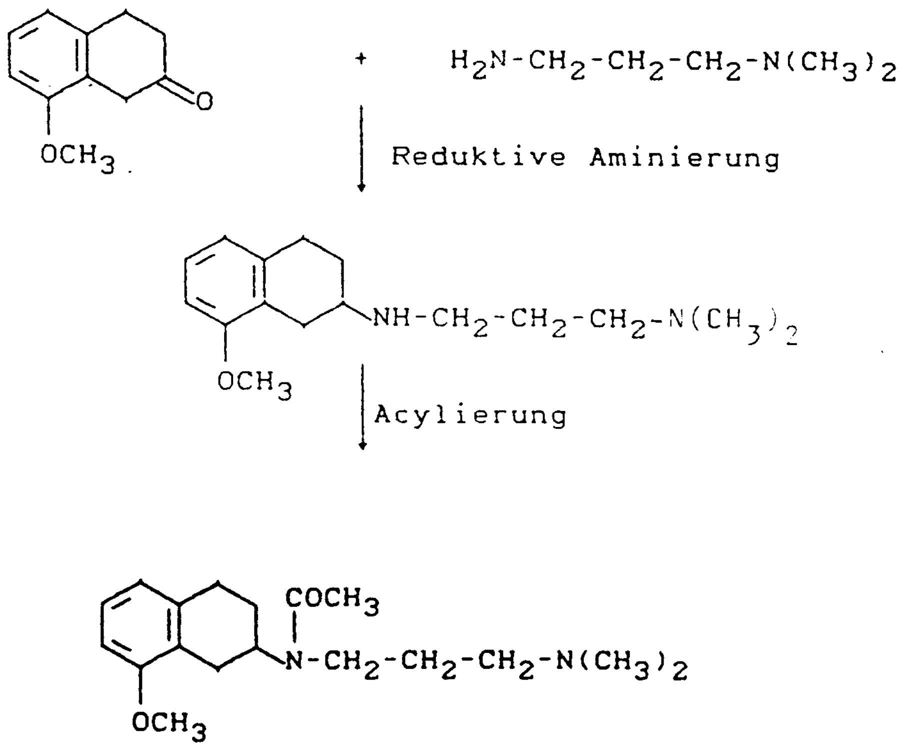


Die als Ausgangsmaterialien verwendeten Tetralone der allgemeinen Formel (II) sind bekannt oder können nach bekannten Methoden hergestellt werden (P.A. Robins, J. Walker, J. Chem. Soc. 1958, 409; Cornforth et al. J. Chem. Soc. 1942, 689].

Die als Ausgangsstoffe verwendeten Amine der allgemeinen Formel (III) sind bekannt oder können nach bekannten Methoden hergestellt werden [Houben-Weyl's "Methoden der organischen Chemie" Bd XI/1 und XI/2].

Im Fall der Reaktion mit primären Aminen sind die Zwischenprodukte Schiff'sche Basen, und im Fall der Reaktion mit sekundären Aminen sind die Zwischenprodukte Enamine oder Immoniumsalze.

Die Herstellung der Zwischenverbindungen durch Umsetzung der Tetralone (II) mit Aminen (III) erfolgt in inerten organischen Lösemitteln, gegebenenfalls in Anwesenheit eines Katalysators und gegebenenfalls in Anwesenheit eines wasserbindenden Mittels.

Das erfindungsgemäße Verfahren kann in zwei Schritten, d.h. mit Isolierung der Zwischenprodukte durchgeführt werden. Ebenso ist es möglich das erfindungsgemäße Verfahren als Eintopfverfahren durchzuführen.

Als inerte Lösemittel eignen sich hierbei die üblichen organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Butylmethylether, Dioxan, Tetrahydrofuran, Glykoldimethylether oder Diethylenglykoldimethylether, oder Halogenkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen, oder Amide wie Dimethylformamid oder Hexamethylphosphorsäuretriamid, oder Essigsäure.

Außerdem ist es möglich, Gemische der genannten Lösemittel zu verwenden.

Als Katalysatoren werden im allgemeinen Protonensäuren verwendet. Hierzu gehören bevorzugt anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit $C_1-C_4$-Alkylresten oder mit Arylresten wie beispielsweise Methansulfonsäure, Ethansulfonsäure, Benzolsulfonsäure oder Toluolsulfonsäure.

Das bei der Reaktion gebildete Wasser kann gegebenenfalls im Gemisch mit dem verwendeten Lösemittel während oder nach der Reaktion z.B. durch Destillation oder durch Zugabe von wasserbindenden Mitteln, wie beispielsweise Phosphorpentoxid oder bevorzugt durch Molekularsieb, entfernt werden.

Die Reaktion wird im allgemeinen in einem Temperaturbereich von 0°C bis +150°C, bevorzugt von +20°C bis +100°C durchgeführt.

Bei dem azeotropen Abdestillieren des bei der Reaktion gebildeten Wassers mit den verwendeten Lösemitteln, arbeitet man bevorzugt bei der Siedetemperatur des Azeotrops.

Die Umsetzung kann bei normalem, erhöhtem und bei erniedrigtem Druck durchgeführt werden (z.B. 0,5 - 5 bar). Im allgemeinen arbeitet man bei Normaldruck.

Bei der Durchführung der Reaktion werden im allgemeinen die Ausgangsstoffe in einem molaren Verhältnis von Tetralon (II) zu Amin (III) von 0,5 : 2 bis 1 : 2 eingesetzt. Bevorzugt verwendet man molare Mengen der Reaktanden.

Die Reduktion der Zwischenprodukte erfolgt entweder durch Wasserstoff in Wasser oder inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle oder Platin, oder aber mit Hydriden in inerten Lösemitteln, gegebenenfalls in Anwesenheit eines Katalysators.

Bevorzugt wird die Reaktion mit Hydriden, wie komplexen Borhydriden oder Aluminiumhydriden, durchgeführt. Besonders bevorzugt werden hierbei Natriumborhydrid, Lithiumaluminiumhydrid oder Natriumcyanoborhydrid eingesetzt.

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether oder Diethylenglykoldimethylether, oder Amide wie Hexamethylphosphorsäuretriamid, oder Dimethylformamid oder Essigsäure. Ebenso ist es möglich, Gemische der genannten Lösemittel zu verwenden.

Als Katalysatoren bei der Reduktion werden im allgemeinen Protonensäuren verwendet. Hierzu gehören bevorzugt anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit $C_1-C_4$-Alkylresten oder mit Arylresten wie beispielsweise Methansulfonsäure, Ethansulfonsäure, Benzolsulfonsäure oder Toluolsulfonsäure.

Bei der Durchführung des erfindungsgemäßen Verfahrens hat es sich als günstig erwiesen, die Umsetzung der Tetralone (II) mit den Aminen (III) in einem inerten Lösemittel, bevorzugt in Essigester oder in Alkoholen wie beispielsweise Methanol, Ethanol, Propanol oder Isopropanol, oder deren Gemische in Anwesenheit von anorganischen oder organischen Säuren, wie beispielsweise Chlorwasserstoffsäure oder Essigsäure, und in Anwesenheit eines Reduktionsmittels, bevorzugt von komplexen Hydriden wie beispielsweise Natriumborhydrid oder Natriumcyanoborhydrid, gegebenenfalls in Anwesenheit eines wasserentziehenden Mittels, bevorzugt von Molsieb, als Eintopfverfahren durchzuführen.

In diesem Fall wird die Reaktion in einem Temperaturbereich von 0°C bis +150°C, bevorzugt von 0°C bis +100°C bei Normaldruck durchgeführt. Es ist ebenso möglich, die Reaktion bei Unterdruck oder bei Überdruck (z.B im Bombenrohr) durchzuführen.

Wird das erfindungsgemäße Verfahren als Eintopfreaktion durchgeführt, hat es sich als günstig erwiesen, das Amin in einem bis zu 10-fachen, bevorzugt in einem bis zu 5-fachen Überschuß über das Tetralon einzusetzen.

Die Acylierung erfolgt im allgemeinen in inerten Lösemitteln durch Umsetzung der erfindungsgemäßen Verbindungen mit $R^2$ = H, mit Acylierungsmittel, bevorzugt reaktiven Carbonsäurederivaten, gegebenenfalls in Anwesenheit von Basen.

Inerte Lösemittel sind hierbei im allgemeinen Wasser oder organische Lösemittel, die sich unter den Reaktionsbedingungen nicht verändern. Hierzu gehören bevorzugt Alkohole wie Methanol, Ethanol, Propanol

oder Isopropanol, oder Ether wie Diethylether, Tetrahydrofuran, Dioxan oder Glykoldimethylether, oder Kohlenwasserstoffe wie Benzol, Toluol, Xylol oder Erdölfraktionen, oder Halogenkohlenwasserstoffe wie Methylenchlorid, Chloroform oder Tetrachlorkohlenstoff, oder Carbonsäuren wie Essigsäure oder Propionsäure, oder Carbonsäureanhydride wie Propionsäureanhydrid oder Acetanhydrid. Ebenso ist es möglich, Gemische der genannten Lösemittel einzusetzen.

Reaktive Carbonsäurederivate sind im allgemeinen Carbonsäurehalogenide oder Carbonsäureanhydride. Bevorzugt sind hierbei aliphatische Carbonsäurebromide, -chloride oder -anhydride. Besonders bevorzugt sind Essigsäurechlorid, Essigsäurebromid, Essigsäureanhydrid, Propionsäurechlorid, Propionsäurebromid, Propionsäureanhydrid.

Als Basen können übliche basische Verbindungen für basische Reaktionen eingesetzt werden. Hierzu gehören vorzugsweise Alkali- oder Erdalkalihydroxide oder -carbonate wie beispielsweise Lithiumhydroxid, Natriumhydroxid, Kaliumhydroxid oder Bariumhydroxid, Natriumcarbonat oder Kaliumcarbonat, oder Alkalialkoholate wie beispielsweise Natriummethanolat, Natriumethanolat, Kaliummethanolat oder Kaliumethanolat.

Die Acylierung wird im allgemeinen in einem Temperaturbereich von -20 °C bis +100 °C, bevorzugt von 0 °C bis +50 °C, bei Normaldruck durchgeführt.

Im Rahmen der vorliegenden Erfindung entsprechen die disubstituierten 2-Aminotetraline (Ia) der allgemeinen Formel

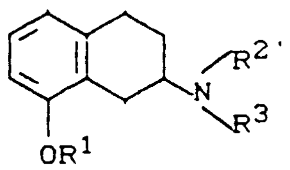

(Ia)

in welcher

$R^1$ und $R^3$ die angegebene Bedeutung haben

und

$R^{2'}$ für Alkyl steht.

Im Rahmen der vorliegenden Erfindung entsprechen die alkylsubstituierten 2-Aminotetraline (Ib) der allgemeinen Formel

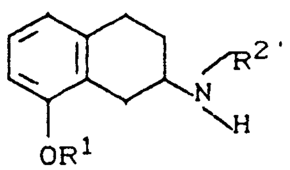

(Ib)

in welcher

$R^1$ die oben angegebene Bedeutung hat

und

$R^{2'}$ für Alkyl steht.

Im Rahmen der vorliegenden Erfindung entsprechen die monosubstituierten basischen 2-Aminotetraline (Ic) der Formel

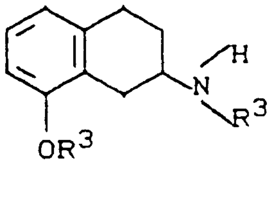

(Ic)

in welcher

$R^1$ und $R^3$ die angegebene Bedeutung haben.

Es wurde ein weiteres Verfahren für die Herstellung der Verbindungen der allgemeinen Formel (Ia) ausgehend von den erfindungsgemäßen Verbindungen der Formeln (Ib) und (Ic) gefunden, wobei die Ausgangsverbindungen (Ib) und (Ic) nach dem oben angegebenen erfindungsgemäßen Verfahren der reduktiven Aminierung erhalten werden.

Die Verbindungen der allgemeinen Formel (Ia)

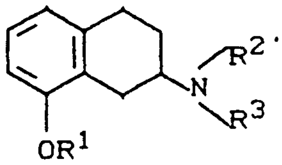

(Ia)

in welcher

$R^1$ – für Wasserstoff oder Alkyl steht,

$R^{2'}$ – für Alkyl steht,

und

$R^3$ – die angegebene Bedeutung hat,

und deren Salze

können hergestellt werden, indem man

[A] alkylsubstituierte 2 – Aminotetraline der allgemeinen Formel (Ib)

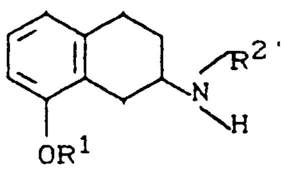

(Ib)

in welcher

$R^1$ und $R^{2'}$ die oben angegebene Bedeutung haben,

mit Halogenverbindungen der allgemeinen Formel (IV)

Hal – $R^3$ (IV)

in welcher

$R^3$ die oben angegebene Bedeutung hat

und

Hal – für Halogen, bevorzugt für Chlor, Brom oder Iod steht,

in inerten Lösemitteln, in Anwesenheit von Basen, gegebenenfalls in Gegenwart von Reaktionsbeschleunigern umsetzt,

dann gegebenenfalls funktionelle Gruppen durch Reduktion, Hydrolyse, Oxidation oder Umsetzung mit elektrophilen Reagenzien in andere funktionelle Gruppen überführt,

und dann in Fall der Herstellung der Salze mit entsprechenden Säuren umsetzt,

oder indem man

[B] monosubstituierte basische 2 – Aminotetraline der allgemeinen Formel (Ic)

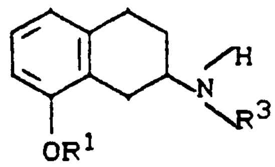

(Ic)

in welcher

$R^1$ und $R^3$ die oben angegebene Bedeutung haben,
mit Verbindungen der allgemeinen Formel (V)

$D - R^{2'}$ (V)

in welcher
$R^{2'}$ die oben angegebene Bedeutung hat
und
D für einen Carbonylsauerstoff steht,
in inerten Lösemitteln, gegebenenfalls in Gegenwart eines Katalysators umsetzt,
dann die erhaltenen Zwischenprodukte in inerten Lösemitteln reduziert,
dann gegebenenfalls funktionelle Gruppen durch Reduktion, Hydrolyse, Oxidation oder Umsetzung mit elektrophilen Reagenzien in andere funktionelle Gruppen überführt,
und dann im Fall der Herstellung der Salze mit entsprechenden Säuren umsetzt.

Je nach Art der verwendeten Ausgangsstoffe lassen sich die beiden Verfahrensvarianten A und B durch folgende Formelschemata erläutern:

[A]

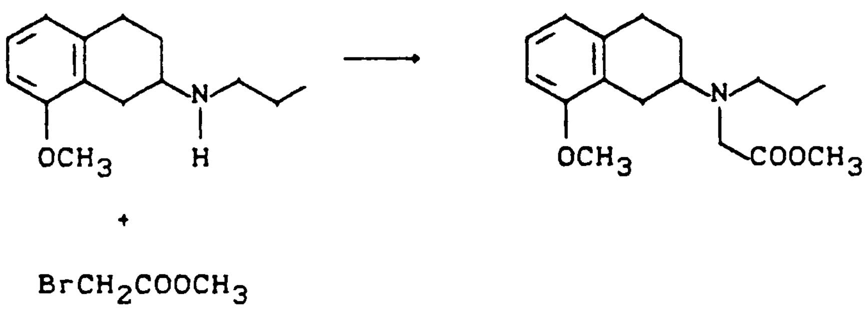

[B]

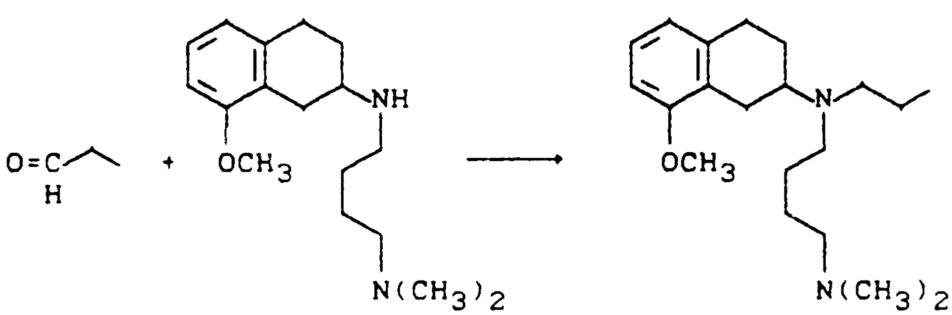

Verfahrensvariante A:

Als Lösemittel können hier die üblichen organischen Lösemittel verwendet werden, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether oder Butylmethylether, oder Ketone wie Aceton oder Butanon, oder Amide wie Dimethylformamid oder Hexamethylphosphorsäuretriamid, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin, Ebenso können Gemische der genannten Lösemittel verwendet werden.

Als Basen eignen sich die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Triethylamin, Picolin oder N-Methylpiperidin, oder Amide wie Natriumamid oder Lithiumdiisopropylamid, oder metallorganische Verbindungen wie Butyllithium oder Phenyllithium.

Die Umsetzung wird im allgemeinen in einem Temperaturbereich von 0°C bis +150°C, bevorzugt von Raumtemperatur bis +80°C durchgeführt.

Die Reaktion wird im allgemeinen bei Normaldruck durchgeführt. Es ist jedoch ebenso möglich, die Reaktion bei erhöhtem oder erniedrigtem Druck durchzuführen.

Als Reaktionsbeschleuniger werden in allgemeinen Alkaliiodide eingesetzt, bevorzugt Natriumiodid oder Kaliumiodid.

Die Base wird hierbei in einer Menge von 1 bis 5, bevorzugt von 1 bis 2 Mol, bezogen auf 1 Mol der Halogenverbindung eingesetzt. Die Halogenverbindung wird bevorzugt in einer bis zu 10-fachen, bevorzugt in einer bis zu 5-fachen Überschußmenge über das alkylsubstituierte 2-Aminotetralin (Ib) eingesetzt.

Verfahrensvariante B:

Im Fall der Reaktion mit primären Aminen sind die Zwischenprodukte Schiff'sche Basen, und im Fall der Reaktion mit sekundären Aminen sind die Zwischenprodukte Enamine oder Immoniumsalze.

Die Herstellung der Zwischenprodukte im ersten Schritterfolgt in inerten organischen Lösemitteln, gegebenenfalls in Anwesenheit eines Katalysators und gegebenenfalls in Anwesenheit eines wasserbindenden Mittels.

Das erfindungsgemäße Verfahren kann in 2 Schritten, d.h. mit Isolierung der Zwischenprodukte durchgeführt werden. Ebenso ist es möglich die Reduktion als Eintopfverfahren durchzuführen.

Als inerte Lösemittel eignen sich hierbei die üblichen organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Butylmethylether, Dioxan, Tetrahydrofuran, Glykoldimethylether oder Diethylenglykoldiethylether, oder Halogenkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen, oder Amide wie Dimethylformamid oder Hexamethylphosphorsäuretriamid oder Essigsäure. Außerdem ist es möglich, Gemische der genannten Lösemittel zu verwenden.

Als Katalysatoren werden im allgemeinen Protonensäuren verwendet. Hierzu gehören bevorzugt anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispeilsweise Essigsäure, Trifluoressigsäure, Trichloressigsäure oder Propionsäure, oder Sulfonsäuren mit $C_1$-$C_4$-Alkylresten oder mit Arylresten wie beispielsweise Methansulfonsäure, Ethansulfonsäure, Benzolsulfonsäure oder Toluolsulfonsäure.

Das bei der Reaktion gebildete Wasser kann gegebenenfalls im Gemisch mit dem verwendeten Lösemittel während oder nach der Reaktion z.B. durch Destillation oder durch Zugabe von wasserbindenden Mitteln, wie beispielsweise Phosphorpentoxid oder bevorzugt durch Molekularsieb entfernt werden.

Die Reaktion wird im allgemeinen in einem Temperaturbereich von 0°C bis +150°C, bevorzugt von +20°C bis +100°C durchgeführt.

Bei dem azeotropen Abdestillieren des bei der Reaktion gebildeten Wasser mit den verwendeten Lösemitteln, arbeitet man bevorzugt bei der Siedetemperatur des Azeotrops.

Die Umsetzung kann bei normalem, erhöhtem und bei erniedrigtem Druck durchgeführt werden (z.B. 0,5 - 5 bar). Im allgemeinen arbeitet man bei Normaldruck.

Bei der Durchführung wird die Verbindung (V) in einer Menge von 0,1 - 10, bevorzugt von 0,5 - 5 mol, bezogen auf 1 mol monosubstituiertes basisches 2-Aminotetralin (Ic) eingesetzt.

Die Reduktion der Zwischenprodukte erfolgt entweder durch Wasserstoff in Wasser oder in inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle oder Platin, oder mit Hydriden in inerten Lösemitteln, gegebenenfalls in Anwesenheit eines Katalysators.

Bevorzugt wird die Reaktion mit Hydriden, wie komplexen Borhydriden oder Aluminiumhydriden durchgeführt. Besonders bevorzugt werden hierbei Natriumborhydrid, Lithiumaluminiumhydrid oder Natriumcyanoborhydrid eingesetzt.

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether oder Amide wie Hexamethylphosphorsäuretriamid oder Dimethylformamid, oder Essigsäure. Ebenso ist es möglich, Gemische der genannten Lösemittel zu verwenden.

Als Katalysatoren bei der Reduktion werden im allgemeinen Protonensäuren verwendet. Hierzu gehören bevorzugt anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit $C_1-C_4$-Alkylresten oder mit Arylresten wie beispielsweise Methansulfonsäure, Ethansulfonsäure, Benzolsulfonsäure oder Toluolsulfonsäure.

Bei der Durchführung des erfindungsgemäßen Verfahrens hat es sich als günstig erwiesen, die Umsetzung der Verbindungen (V) mit den Aminen (Ic) in einem interten Lösemittel, bevorzugt in Essigsäure oder Alkoholen wie beispielsweise Methanol, Ethanol, Propanol oder Isopropanol oder deren Gemischen, in Anwesenheit von anorganischen oder organischen Säuren wie beispielsweise Chlorwasserstoffsäure oder Essigsäure, und in Anwesenheit eines Reduktionsmittels, bevorzugt von komplexen Hydriden wie beispielsweise Natriumborhydrid oder Natriumcyanoborhydrid, gegebenenfalls in Anwesenheit eines wasserentziehenden Mittel, bevorzugt Molekularsieb, als Eintopfverfahren durchzuführen.

In diesem Fall wird die Reaktion in einem Temperaturbereich von 0°C bis +150°C, bevorzugt von 0°C bis +100°C bei Normaldruck durchgeführt. Es ist ebenso möglich die Reaktion bei Unterdruck oder bei Überdruck (z.B. im Bombenrohr) durchzuführen.

Wird das erfindungsgemäße Verfahren als Eintopfreaktion durchgeführt, hat es sich als günstig erwiesen, das Aminotetralin (Ic) in einer Menge von 0,1 bis 10, bevorzugt von 0,5 bis 5 mol, bezogen auf 1 mol der Verbindung (V) einzusetzen.

Das Überführen von funktionellen Gruppen in andere funktionelle Gruppen in den oben aufgeführten Herstellungsverfahren erfolgt je nach Art der funktionellen Gruppen durch Oxidation, Reduktion, Hydrolyse oder durch Umsetzung mit elektrophilen Reagenzien und soll im folgenden erläutert werden.

1. Die Reduktion der Nitrilgruppe zur Aminogruppe erfolgt im allgemeinen mit Metallhydriden, bevorzugt mit Lithiumaluminiumhydrid, Aluminiumhydrid (hergestellt z.B. durch Umsetzung von Lithiumaluminiumhydrid mit 100%iger Schwefelsäure oder mit Aluminiumchlorid) oder deren Gemischen in inerten Lösemitteln wie Ethern oder Chlorkohlenwasserstoffen, bevorzugt in Ethern wie beispielsweise Tetrahydrofuran, Diethylether oder Dioxan in einem Temperaturbereich von -20°C bis +100°C, bevorzugt von 0°C bis +50°C bei Normaldruck.

Die Reduktion ist außerdem durch Hydrieren der Nitrile in inerten Lösemitteln wie Alkoholen z.B. Methanol, Ethanol, Propanol oder Isopropanol in Gegenwart eines Edelmetallkatalysators wie Platin, Palladium, Palladium auf Tierkohle oder Raney-Nickel, in einem Temperaturbereich von 0°C bis +150°C, bevorzugt von Raumtemperatur bis +100°C bei Normaldruck oder bei Überdruck möglich.

Die Reaktion kann durch folgendes Schema verdeutlicht werden:

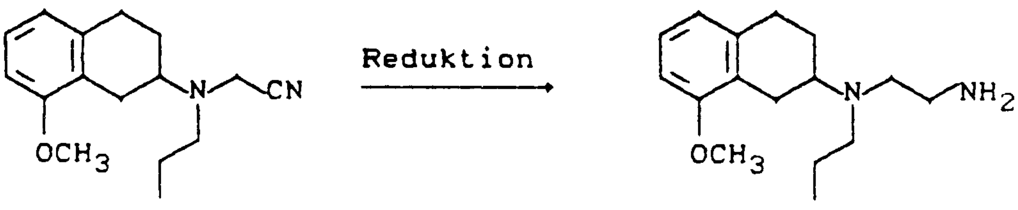


2. Die Überführung von Carbamaten in N-Methylamine erfolgt im allgemeinen durch Reduktion mit Hydriden, bevorzugt mit Lithiumaluminiumhydrid in inerten Lösemitteln wie Ethern, Kohlenwasserstoffen oder Chlorkohlenwasserstoffen, bevorzugt in Ethern wie beispielsweise Diethylether, Tetrahydrofuran

oder Dioxan, in einem Temperaturbereich von 0 ° C bis +150 ° C, bevorzugt von +20 ° C bis +100 ° C bei Normaldruck.

Die Reaktion kann durch folgendes Schema verdeutlicht werden:

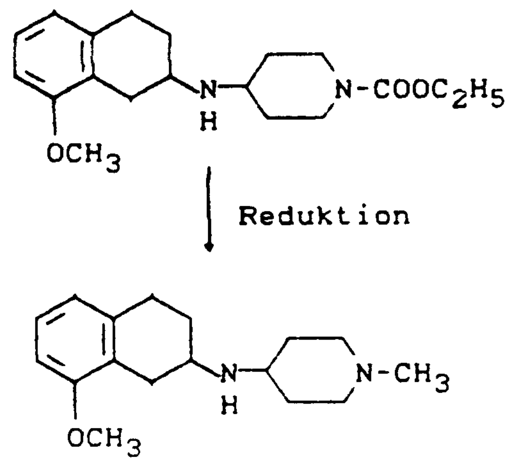


3. Die Reduktion von Alkoxycarbonylgruppen in Alkoholgruppen erfolgt im allgemeinen mit Hydriden, bevorzugt mit Lithiumaluminiumhydrid in inerten Lösemitteln wie Ethern, Kohlenwasserstoffen oder Halogenkohlenwasserstoffen oder deren Gemischen, bevorzugt in Ethern wie beispielsweise Diethylether, Tetrahydrofuran oder Dioxan in einem Temperaturbereich von 0 ° C bis +150 ° C, bevorzugt von +20 ° C bis + 100 ° C bei Normaldruck.

Die Reaktion kann durch folgendes Schema verdeutlicht werden:

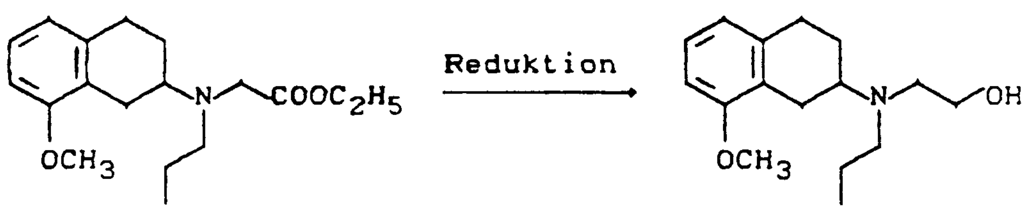


4. Die Hydrolyse der Nitrilgruppe zur Carbonamidgruppe erfolgt im allgemeinen mit Hilfe von starken Mineralsäuren, bevorzugt mit Chlorwasserstoff in inerten Lösemitteln wie Wasser und/oder Alkoholen wie beispielsweise Methanol, Ethanol, Propanol oder Isopropanol in einem Temperaturbereich von 0 ° C bis +150 ° C, bevorzugt von +20 ° C bis +100 ° C bei Normaldruck.

Die Reaktion kann durch folgendes Schema erläutert werden:

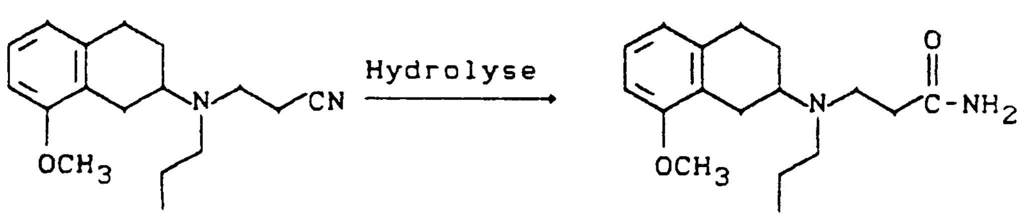


5. Durch Umsetzung von NH- oder OH-aciden Verbindung ($R^4$ = OH oder $NR^5R^6$ wobei $R^5$ = H und $R^6$ = H, Alkyl, Aryl oder Aralkyl ist) mit elekrophilen Reagenzien erhält man eine Vielzahl weiterer erfindungsgemäßer Verbindungen:

a) Die Überführung von Aminen in Carbonamide erfolgt im allgemeinen durch Umsetzung mit Carbonsäureestern in inerten Lösemitteln wie Ethern oder deren Gemischen oder Kohlenwasserstoffen, bevorzugt in Ethern wie beispielsweise Diethylether, Tetrahydrofuran oder Dioxan, gegebenenfalls

in Anwesenheit von Basen wie Alkalimetalle, Alkalihydride, Alkalialkoholate oder lithiumorganischen Verbindungen, bevorzugt in Anwesenheit von Alkalimetallen wie beispielsweise Natrium oder Alkalihydriden wie Natriumhydrid oder Kaliumhydrid in einem Temperaturbereich von +20°C bis +150°C, bevorzugt bei der Siedetemperatur des verwendeten Lösemittels bei Normaldruck.

Darüberhinaus ist es möglich, die Amide mit Carbonsäurehalogeniden oder -anhydriden, bevorzugt mit Carbonsäurenchloriden in inerten Lösemitteln wie Ethern, Kohlenwasserstoffen oder Halogenkohlenwasserstoffen oder deren Gemische, bevorzugt in Ethern wie beispielsweise Diethylether, Tetrahydrofuran, oder Halogenkohlenwasserstoffen wie Methylenchlorid oder Chloroform, gegebenenfalls in Anwesenheit von Basen wie Alkalicarbonate beispielsweise Natriumcarbonat oder Kaliumcarbonat, oder organischen Aminen wie beispielsweise Triethylamin oder Pyridin, in einem Temperaturbereich von -20°C bis + 100°C, bevorzugt von 0°C bis +60°C bei Normaldruck herzustellen.

Die Reaktion kann durch folgendes Schema verdeutlicht werden:

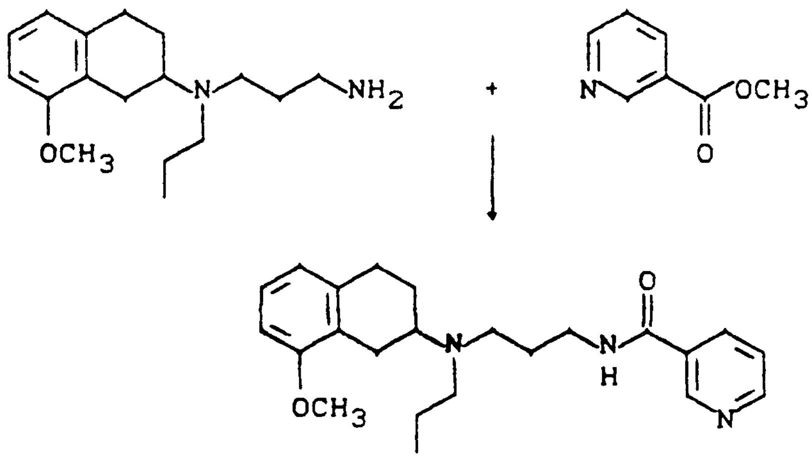

b) Die Überführung von Aminen in Carbamate erfolgt im allgemeinen mit Kohlensäurederivaten wie Kohlensäureester oder -halogeniden, bevorzugt mit unsymmetrischen Kohlensäureestern, besonders bevorzugt mit Kohlensäurestern, die einen Phenylesterrest tragen, oder mit Kohlensäurechloriden in inerten Lösemitteln wie Ethern, Kohlenwasserstoffen oder Halogenkohlenwasserstoffen oder deren Gemische. bevorzugt in Ethern wie beispielsweise Diethylether, Tetrahydrofuran oder Dioxan, in einem Temperaturbereich von +20°C bis +150°C, bevorzugt von +20°C bis +100°C bei Normaldruck.

Die Reaktion kann durch folgendes Schema erläutert werden:

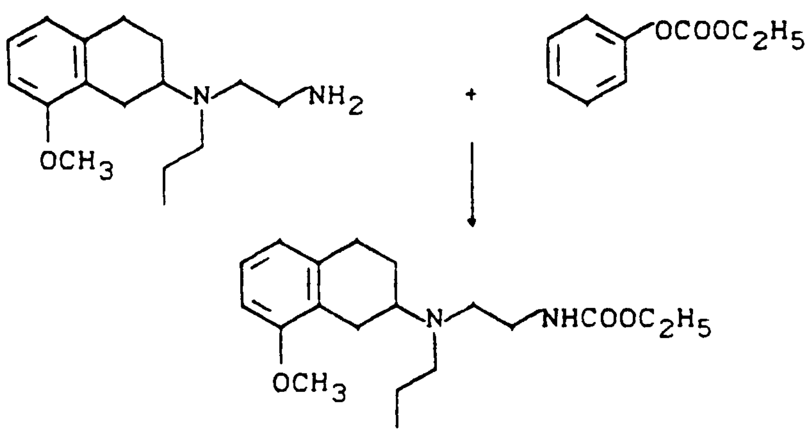

c) Die Überführung von Aminen in Harnstoffe erfolgt im allgemeinen durch Umsetzung mit Isocyanaten in inerten Lösemitteln wie Ethern, Kohlenwasserstoffen oder Halogenkohlenwasserstoffen oder deren Gemische, bevorzugt in Ethern wie beispielsweise Diethylether oder Tetrahydrofuran, oder in

Halogenkohlenwasserstoffen wie beispielsweise Methylenchlorid oder Chloroform, in einem Temperaturbereich von − 20 ° C bis + 150 ° C, bevorzugt von 0 ° C bis + 100 ° C bei Normaldruck.

Die Reaktion kann durch folgendes Schema erläutert werden:

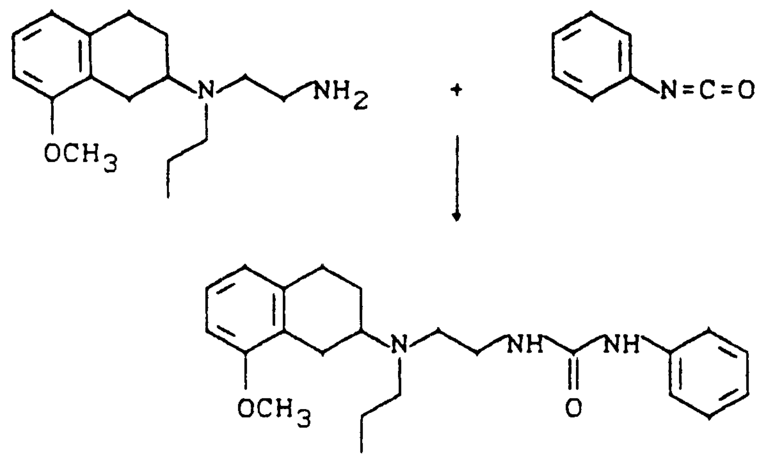

d) Die Überführung von Amiden in Sulfonamide bzw. Aminosulfamoylderivate erfolgt im allgemeinen mit Sulfonsäurehalogeniden bzw. mit Amidosulfonsäurehalogeniden, bevorzugt mit den entsprechenden Chloriden in inerten Lösemitteln wie Ethern, Kohlenwasserstoffen oder Halogenkohlenwasserstoffen oder deren Gemische, bevorzugt in Halogenkohlenwasserstoffen wie beispielsweise Methylenchlorid oder Chloroform, gegebenenfalls in Anwesenheit von Basen wie Alkalihydroxide, Alkalicarbonate, Alkalialkoholate oder organische Amine, bevorzugt mit Alkalihydroxiden wie Natriumhydroxid oder Kaliumhydroxid, Alkalicarbonaten wie beispielsweise Natriumcarbonat oder Kaliumcarbonat, oder organischen Aminen wie Triethylamin oder Pyridin, in einem Temperaturbereich von −20 ° C bis +100 ° C, bevorzugt von 0 ° C bis +50 ° C bei Normaldruck.

Die Reaktion kann durch folgendes Schema verdeutlicht werden:

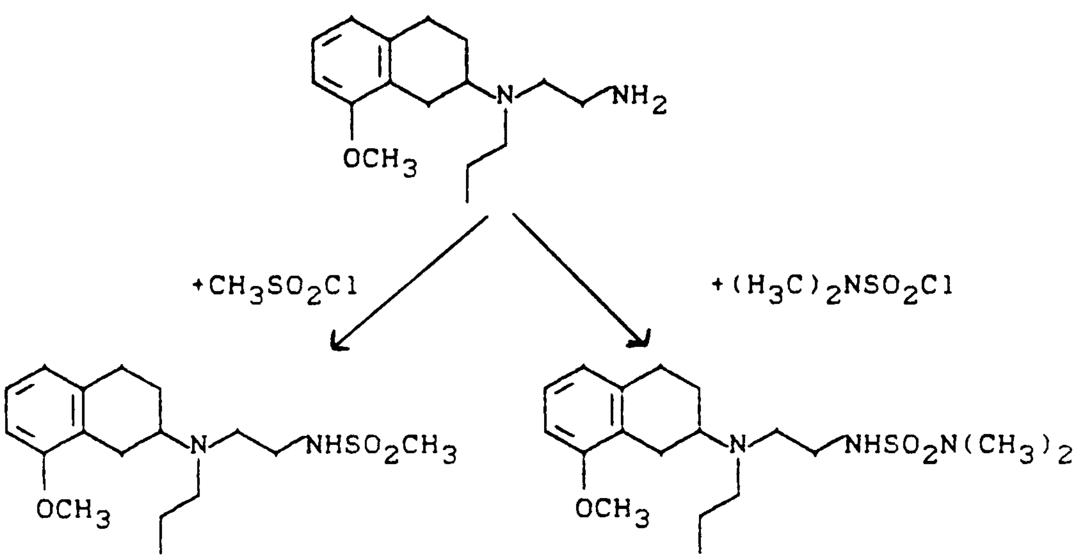


e) Die Überführung der Hydroxygruppe in Kohlensäureester erfolgt im allgemeinen durch Umsetzen mit Halogenameisensäureestern, bevorzugt mit Chlorameisensäureestern in inerten Lösemitteln wie Ethern, Kohlenwasserstoffen oder Halogenkohlenwasserstoffen, bevorzugt in Halogenkohlenwasserstoffen wie Methylenchlorid oder Chloroform, oder in Ethern wie Deithylether oder Tetrahydrofuran, gegebenenfalls in Anwesenheit von Basen wie Alkylihydroxide, Alkalicarbonate oder organischen Aminen, bevorzugt in Anwesenheit von organischen Aminen wie Triethylamin, Pyridin, Picolin oder

Dimethylaminopyridin in einem Temperaturbereich von −20 ° C bis +100 ° C, bevorzugt von 0 ° C bis +30 ° C bei Normaldruck.

Die Reaktion läßt sich durch folgendes Schema verdeutlichen:

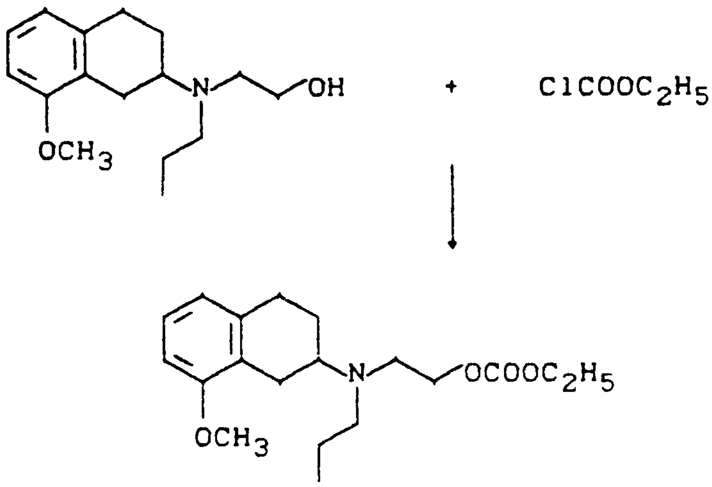

f) Cyclische Sulfonamide werden im allgemeinen durch Reaktion intramolekularer Elektrophile in inerten dipolar aprotischen Lösemitteln bevorzugt in Dimethylformamid, Hexamethylphosphorsäuretriamid oder Dimethylsulfoxid, gegebenenfalls in Anwesenheit von Basen wie Alkalimetalle, Alkalihydride, Alkaliamide, Alkalialkoholate oder lithiumorganischen Verbindungen, bevorzugt in Anwesenheit von Alkalihydriden wie Natriumhydrid oder Kaliumhydrid, oder Alkaliamiden wie Natriumamid oder Lithiumdiisopropylamid, gegebenenfalls in Gegenwart katalytischer Mengen eines Alkaliiodides beispielsweise Natriumiodid oder Kaliumiodid in einem Temperaturbereich von −20 ° C bis +100 ° C, bevorzugt von 0 ° C bis +50 ° C bei Normaldruck hergestellt.

Die Reaktion läßt sich durch folgendes Schema verdeutlichen:

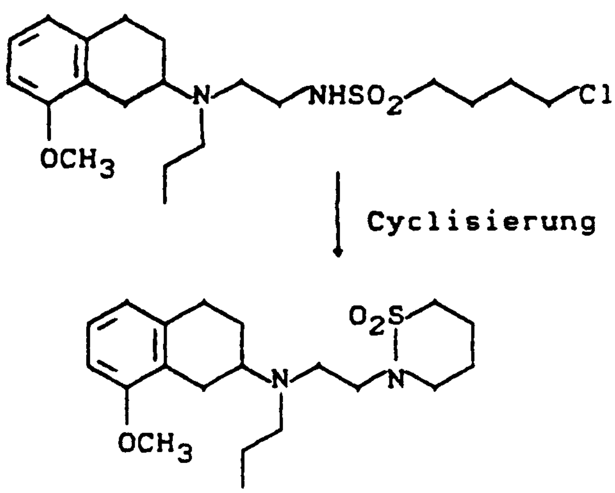


5. Die Oxidation der Thioethergruppe zu Sulfoxiden bzw. Sulfonen erfolgt im allgemeinen mit Oxidationsmitteln wie Peroxoverbindungen oder Wasserstoffperoxid selbst, bevorzugt mit Wasserstoffperoxid, in inerten Lösemitteln wie Carbonsäuren und Carbonsäureanhydriden, bevorzugt in Essigsäure, in einem Temperaturbereich von −20 ° C bis +100 ° C, bevorzugt von 0 ° C bis +50 ° C.

Die Reaktion läßt sich durch folgendes Schema verdeutlichen:

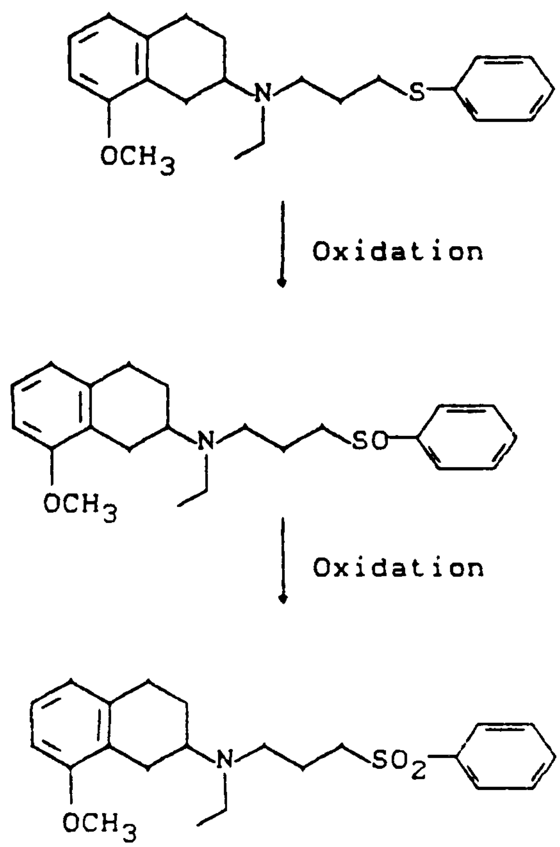


Über die Variation der funktionellen Gruppen in $R^3$ hinaus sind 8-Hydroxy-substituierte 2-Aminotetraline ($R^1$ = H) aus den entsprechenden 8-Methoxy-substituierten Verbindungen ($R^1$ = $CH_3$) durch bekannte Methoden der Dealkylierung zugänglich [T. Green, Protective Groups in Organic Chemistry, Seite 89, 1.Auflage, J. Wiley & Sons, New York, 1981]. Zweckmäßigerweise gelangen jeweils die Methoden zum Einsatz, die mit der Natur des Restes $R^4$ zu vereinbaren sind.

Außerdem ist es möglich disubstituierte 2-Aminotetraline (Ia), in welchen $R^1$ und $R^{2'}$ die oben angegebene Bedeutung haben $R^3$ füe eine 2-Cyanoethylgruppe steht, durch Umsetzung von alkylsubstituierten 2-Aminotetraline (Ib) mit Acrylnitril, gegebenenfalls in Anwesenheit eines Katalysators, insbesondere Kupferacetat, herzustellen.

Außerdem ist es möglich, den Rest $R^3$ durch Mannich-Reaktion einzuführen (durch Reaktion von alkylsubstituierten 2-Aminotetralinen (Ib) mit Formaldehyd und CH-aciden Verbindungen, insbesondere mit Acetylengruppen).

Als Tetralone können beispielsweise erfindungsgemäß verwendet werden:
8-Hydroxytetralon, 8-Methoxytetralon.

Die als Ausgangsstoffe eingesetzten Amine der allgemeinen Formel (III) sind bekannt oder können nach bekannten Methoden hergestellt werden [Houben-Weyl's "Methoden der organischen Chemie" Bd XI/1 und XI/2].

Als Amine können beispielsweise erfindungsgemäß verwendet werden:
Methylamin, Ethylamin, Propylamin, Isopropylamin, Butylamin, 4-Dimethylaminobutylamin, 4-Diethylaminobutylamin, 3-Dimethylaminopropylamin, 3-Diethylaminopropylamin, 2-Dimethylaminoethylamin, 2-Diethylaminoethylamin, 2-Amino-1-ethoxycarbonylamido-ethan, 3-Amino-1-ethoxycarbonylamido-propan, 4-Amino-1-ethoxycarbonylamido-butan, 3-Aminochinuclidin, 2-[(Phenylaminocarbonyl)-amino]ethylamin, 2-[(Phenylaminocarbonyl)amino]propylamin, 4-Aminomethylpiperidin, 4-(Ethoxycarbonyl)amino-ethyl-piperidin, N-Methylpiperazin, 4-Amino-1-carboxyethyl-piperidin, N,N-Dimethylpropyliden-diamin, N,N-Diethylpropyliden-diamin, N,N-Diethylethyliden-diamin, N,N-Dimethylethylen-diamin, N-(2-Aminoethyl)ethylcarbamat, N-(2-Aminoethyl)propylcarbamat.

Die Halogenverbindungen der allgemeinen Formel (IV) sind bekannt oder können nach bekannten Methoden hergestellt werden [Beilstein's Handbuch der organischen Chemie 2, 197, 201, 250, 278; 3, 9, 10; 21, 461, 462, 463].

Als Halogenverbindungen können beispielsweise erfindungsgemäß verwendet werden:

Chloracetonitril, 2-Chlorpropionnitril, 3-Chlorbutyronitril, 3-Brompropylphthalimid, 3-Chlorporpylphthalimid, 2-Bromethylphthalimid, 2-Bromethylphthalimid, 4-Brombutylphthalimid, 4-Chlorbutylphthalimid, Chloressigsäurediethylamid, Chloressigsäuredimethylamid, Chloressigsäuremethylester, Chloressigsäureethylester, Bromessigsäureethylester, Bromessigsäuremethylester, 2-δ-Brombutyl-1,2-benzoisothiazol-3(2H)-on-1,1-dioxid, 2-γ-Brompropyl-1,2-benzoisothiazol-3(2H)-on-1,1-dioxid.

Die als Ausgangsstoffe eingesetzten Carbonylverbindungen der allgemeinen Formel (V) sind bekannt oder können nach bekannten Methoden hergestellt werden [Beilstein's Handbuch der organischen Chemie 1, 594, 629, 662].

Als Ketonverbindungen können beispielsweise erfindungsgemäß verwendet werden:

Acetaldehyd, Propionaldehyd, Butyraldehyd;

Die erfindungsgemäßen Stoffen der allgemeinen Formel (I) haben eine hohe Affinität zu cerebralen 5-Hydroxy-tryptamin-Rezeptoren vom 5-$HT_1$-Typ. Diese Affintät der erfindungsgemäßen Verbindungen ist im Vergleich zu denen aus dem Stand der Technik bekannten erhöht (EP-A1-41 488). Hiermit verbunden sind agonistisch, partiell agonistische oder antagonistische Wirkungen am Serotonin-Rezeptor, wogegen die bekannten Stoffe rein agonistische Eigenschaften besitzen.

Die in der vorliegenden Erfindung beschriebenen hochaffinen Liganden für den Serotonin-1-Rezeptor stellen somit bessere Wirkstoffe zur Bekämpfung von Krankheiten dar, die durch Störungen des serotoninergen Systems, insbesondere bei Involvierung von Rezeptoren, die hohe Affinität zu 5-Hydroxytryptamin besitzen (5-$HT_1$-Typ), gekennzeichnet sind. Sie eignen sich daher zur Behandlung von Erkrankungen des Zentralennervensystems wie Angst-, Spannungs- und Depressionszuständen, zentralnervös bedingten Sexualdysfunktionen und Schalfstörungen. Die erfindungsgemäßen Substanzen eignen sich weiterhin zur Behandlung von Kognitiven Defiziten, wie sie beispielsweise bei der senilen Demenz und der Alzheimerschen Erkrankung auftreten, und anderer Hirnleistungsstörungen. Weiterhin eignen sich diese Wirkstoffe auch zur Modulierung des cardiovaskulären Systems. Sie greifen auch in die Regulation der cerebralen Durchblutung ein und stellen somit wirkungsvolle Mittel zur Bekämpfung von Migräne dar. Ebenso können die erfindungsgemäßen Verbindungen zur Bekämpfung von Schmerzzuständen eingesetzt werden. Auch eignen sie sich zur Bekämpfung von Erkrankungen des Intestinaltraktes, die durch Störungen des serotoninergen Systems gekennzeichnet sind.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß/Sesamöl), Alkohole (z.b: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate, Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0.01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Herstellungsbeispiele

Die jeweils aufgeführten $R_f$-Werte wurden – sofern nicht anders vermerkt – durch Dünnschichtchromatographie auf Kieselgel (Alufolie, Kieselgel 60 F254, Fa. E. Merck) ermittelt. Die Visualisierung der Substanzfleck geschah durch Betrachtung unter UV-Licht und/oder durch Besprühen mit 1%iger Kaliumpermanganat-Lösung.

Die Flash-Chromatographie wurde auf Kieselgel 60, 0,040 – 0,063 mm, Fa. E. Merck, durchgeführt (siehe Still et al., J. Org. Chem. 43, 2923, 1978; für einfachere Trennprobleme siehe Aldrichimica Acta 18, 25, 1985). Elution mit Lösemittelgradienten bedeutet: Beginnend mit der reinen, unpolaren Lösemittelgemischkomponente wird in steigendem Ausmaß die polare Laufmittelkomponente zugemischt, bis das gewünschte Produkt eluiert wird (DC-Kontrolle).

Bei allen Produkten wurde bei ca. 0,1 Torr das Lösemittel abgetrennt. Hydrochloride wurden bei diesem Druck über Nacht über Kaliumhydroxid/Phosphorpentoxid aufbewahrt.

Beispiel 1

2-[4-(Ethoxycarbonylamino-methyl)piperidin-1-yl]-8-methoxy-1,2,3,4-tetrahydronaphthalin

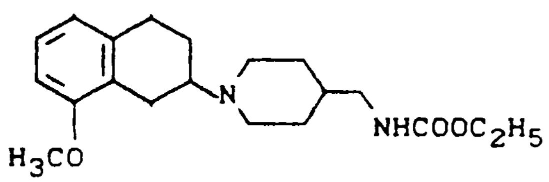

Die Lösung von 1,70 g (9,0 mmol) 4-(Ethoxycarbonylaminomethyl)-piperidin (erhalten aus 4-Aminomethyl-piperidin und Diethylcarbonat in Gegenwart von 4-Dimethylaminopyridin) in 18 ml Methanol wurde mit 9,0 ml (9,0 mmol) 1N methanolischer Salzsäure versetzt. Nach Zugabe von 0,53 g (3,0 mmol) 8-Methoxy-2-tetralon wurde noch 5 min gerührt. Danach wurden 0,20 g (3,3 mmol) Natriumcyanoborhydrid zugegeben und 15 h bei Raumtemperatur gerührt, gefolgt von 18 Tagen Stehen bei ca. +4° C.

Die Reaktionsmischung wurde am Rotationsverdampfer weitgehend eingeengt, mit tert.-Butylmethylether aufgenommen und 30 min intensiv mit verdünnter Natronlauge (pH der wäßrigen Phase wurde auf 10 eingestellt) verrührt. Die wäßrige Phase wurde sorgfältig mit tert.-Butylmethylether extrahiert. Waschen der vereinigten organischen Phasen mit Wasser und gesättigter Kochsalzlösung, Trocknen über Magnesiumsulfat und Einengen am Rotationsverdampfer ergab das Rohprodukt als Öl.
Durch Chromatographie auf Kieselgel (Toluol-Ethanol-Gradienten) ließen sich 0,45 g (43%) der Titelverbindung als gelblichen Sirup gewinnen.
$R_f$ (Toluol-Methanol 4:1): 0,38
IR (Chloroform): 3460, 3003, 2920, 1681, 1587

Beispiel 2

2 – [4 – (Ethoxycarbonylamino – methyl)piperidin – 1 – yl] – 8 – methoxy – 1,2,3,4 – tetrahydronaphthalin Hydrochlorid

Das Hydrochlorid der Verbindung aus Beispiel 1 wurde durch Behandeln mit etherischer Salzsäure erhalten. Schmelzpunkt: 85° C Sintern, 105 – 110° C unter Zersetzung

Beispiel 3

8 – Methoxy – 2 – (4 – methylpiperazin – 1 – yl) – 1,2,3,4 – tetrahydronaphthalin

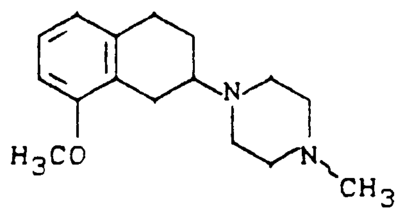

1,76 g (10 mmol) 8 – Methoxy – 2 – tetralon, 3,00 g (30 mmol) N – Methylpiperazin und 1,80 g (30 mmol) Eisessig wurden in 30 ml Methanol 4 h zum Rückfluß erhitzt. Danach wurden 1,30 g (20 mmol) Natriumcyanoborhydrid zugegeben und es wurde noch eine weitere Stunde zum Rückfluß erhitzt. Die Reaktionsmischung wurde am Rotationsverdampfer weitgehend eingeengt, mit tert. – Butylmethylether aufgenommen und 30 min intensiv in 20% Natronlauge verrührt. Die wäßrige Phase wurde sorgfältig mit tert. – Butylmethylether extrahiert. Waschen der vereinigten organischen Phasen mit Wasser und gesättigter Kochsalzlösung, Trocknen über Kaliumcarbonat und Einengen am Rotationsverdampfer ergab das Rohprodukt als Öl (2.9 g).
Dieses Rohprodukt wurde mit dem aus einem gleichgroßen Ansatz erhaltenen (Durchführung dieser Reaktion in Gegenwart von Molsieb 3Å, sonst gleich) vereinigt und auf Kieselgel chromatographiert (Toluol – Methanol 4:1). Auf diese Weise erhielt man 4,20 g der Titelverbindung als bräunliches Öl (80%).
$R_f$ (Chloroform – Methanol 2:1): 0,61

Beispiel 4

8 – Methoxy – 2 – (4 – methylpiperazin – 1 – yl) – 1,2,3,4 – tetrahydronaphthalin Dihydrochlorid

Mit etherischer Salzsäure gewann man aus der Verbindung des Beispiels 3 das Dihydrochlorid als fast farblosen Feststoff.
Schmelzpunkt: >265° C

| Analyse ($C_{16}H_{24}N_2O$ x 2HCl x 0.5 $H_2O$) | | | | |
|---|---|---|---|---|
| Ber. | C 56.1 | H 7.9 | N 8.2 | Cl 20.7 |
| Gef. | C 56.0 | H 7.9 | N 8.1 | Cl 20.6 |

Beispiel 5

2 – (1 – Ethoxycarbonylpiperidin – 4 – yl)amino – 8 – methoxy – 1,2,3,4 – tetrahydronaphthalin

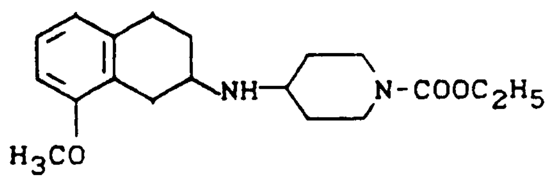

Die Lösung von 2,00 g (11,4 mmol) 8 – Methoxy – 2 – tetralon, 2,90 g (17 mmol) 4 – Amino – 1 – ethoxycarbonyl – piperidin und 3,40 g (57 mmol) Eisessig in 80 ml Methanol wurde bei 0° C 30 min gerührt. Nach Zugabe von 3,85 g (45,6 mmol) Natriumcyanoborhydrid wurde 3 h bei Raumtemperatur gerührt. Die Reaktionsmischung wurde weitgehend eingeengt, mit Toluol aufgenommen, nochmals einge – dampft, mit tert – Butylmethylether versetzt und 30 min intensiv mit verdünnter Natronlauge (pH der wäßrigen Phase auf 10 eingestellt) verrührt. Die wäßrige Phase wurde sorgfältig mit tert. – Butylmethylether extrahiert. Waschen der vereinigten organischen Phasen mit Wasser und gesättigter Kochsalzlösung, Trocknen über Kaliumcarbonat und Einengen am Rotationsverdampfer ergab das Rohprodukt als Öl.

Nach Chromatographie auf Kieselgel (Toluol – Essigester – Gradienten unter Zusatz von 0,5% Trieth – ylamin) erhielt man 2,10 g (55%) der Titelverbindung.
$R_f$ (Toluol – Methanol 4:1): 0,26
IR (Chloroform): 3005, 2931, 1679, 1587

Beispiel 6

2 – (1 – Ethoxycarbonylpiperidin – 4 – yl)amino – 8 – methoxy – 1,2,3,4 – tetrahydronaphthalin Hydrochlorid

Das durch Behandeln mit etherischer Salzsäure aus der Verbindung des Beispiels 5 zugängliche Hydrochlorid fiel als farbloser Festkörper an.
Schmelzpunkt: >270° C

| Analyse ($C_{19}H_{28}N_2O_3$ x HCl) | | | | |
|---|---|---|---|---|
| Ber.:<br>Gef.: | C 61,9<br>C 61,3 | H 7,9<br>H 8,0 | N 7,6<br>N 7,6 | Cl 9.6<br>Cl 9,4 |

Beispiel 7

2 – (3 – Dimethylaminopropyl)amino – 8 – methoxy – 1,2,3,4 – tetrahydronaphthalin

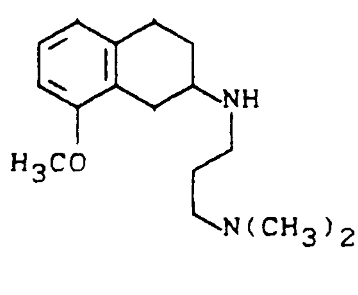

Die Lösung von 7,04 g (40 mmol) 8 – Methoxy – 2 – tetralon, 6,12 g (60 mmol) 3 – Dimethylamino – 1 – propylamin und 18 g (300 mmol) Eisessig wurde bei 0° C 30 Minuten gerührt. Nach Zugabe von 10.0 g (160 mmol) Natriumcyanoborhydrid wurde 3 h bei Raumtemperatur gerührt.

Die Reaktionsmischung wurde am Rotationsverdampfer weitgehend eingeengt, mit tert.-Butylmethylether aufgenommen und 30 min intensiv mit 20% Natronlauge verrührt. Die wäßrige Phase wurde sorgfältig mit tert.-Butylmethylether extrahiert. Waschen der vereinigten organischen Phasen mit Wasser und gesättigter Kochsalzlösung, Trocknen über Kaliumcarbonat und Einengen am Rotationsverdampfer ergab das Rohprodukt als Öl. Das Rohprodukt wurde durch Chromatographie auf Kieselgel (Chloroform-Methanol-Gradienten unter Zusatz von 1% Triethylamin) gereinigt. Auf diese Weise erhielt man 5,10 g (49%) der Titelverbindung als braunes Öl.
$R_f$ (Chloroform-Methanol-Triethylamin 20:10:0,1): 0,1 IR (Chloroform): 3666, 3006, 1587, 1470

Beispiel 8

2-(3-Dimethylaminopropyl)amino-8-methoxy-1,2,3,4-tetrahydronaphthalin Dihydrochlorid

Mit etherischer Salzsäure ließ sich das Dihydrochlorid der Verbindung des Beispiels 7 in Form hellgrauer Kristalle gewinnen.
Schmelzpunkt: 173 - 178° C

Beispiel 9

2-(2-Diethylaminoethyl)amino-8-methoxy-1,2,3,4-tetrahydronaphthalin

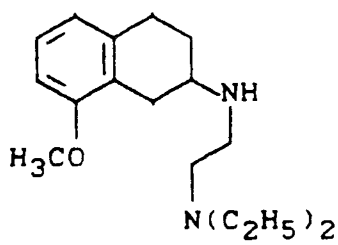

Bei 0° C wurden 3,50 g (20 mmol) 8-Methoxy-2-tetralon, 4,60 g 2-Diethylamino-1-ethylamin und 4,80 g (80 mmol) Eisessig in 150 ml Methanol 30 min gerührt. Nach Zugabe von 5,00 g Natriumcyanoborhydrid wurde 15 h bei Raumtemperatur stehengelassen.

Die Reaktionsmischung wurde am Rotationsverdampfer weitgehend eingeengt, mit tert.-Butylmethylether aufgenommen und 30 min intensiv mit 20% Natronlauge verrührt. Die wäßrige Phase wurde sorgfältig mit tert.-Butylmethylether extrahiert. Waschen der vereinigten organischen Phasen mit Wasser und gesättigter Kochsalzlösung, Trocknen über Kaliumcarbonat und Einengen am Rotationsverdampfer ergab das Rohprodukt als Öl. Chromatographie auf Kieselgel (Toluol-Ethanol-Gradienten) lieferte 3,80 g (69%) der Titelverbindung als Sirup.
$R_f$ (Chloroform-Methanol 2:1): 0,18
IR (Chloroform, $CHCl_3$): 3282, 2970, 1586, 1470

Beispiel 10

2-(2-Diethylaminoethyl)amino-8-methoxy-1,2,3,4-tetrahydronaphthalin Hydrochlorid

Das aus der Verbindung des Beispiels 9 durch Behandeln mit etherischer Salzsäure in Ether erhaltene Dihydrochlorid fiel als beiger Feststoff an.
Schmelzpunkt: 60° C Sintern, ca. 100° C Zersetzung

Beispiel 11

2 – (2 – Ethoxycarbonylamino – ethyl)amino – 8 – methoxy – 1,2,3,4 – tetrahydronaphthalin

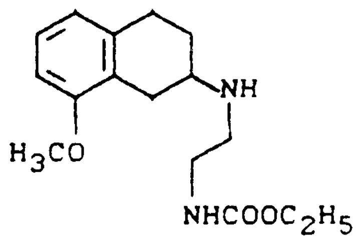

Bei 0° C wurde die Lösung von 7,00 g (40 mmol) 8 – Methoxy – 2 – tetralon, 8,00 g (61 mmol) N – (2 – Aminoethyl)ethylcarbamat und 9,60 g (160 mmol) Eisessig mit 10,0 g (159 mmol) Natriumcyanoborhydrid versetzt. Nach 15 h bei Raumtemperatur wurde eingeengt, mit tert. – Butylmethylether aufgenommen und mit Wasser versetzt. Mit Natronlauge wurde der pH der wäßrigen Phase auf 10 eingestellt. Das Gemisch wurde 30 min intensiv gerührt. Die organische Phase wurde abgetrennt, die wäßrige Phase gut mit dem zuletzt genannten Lösemittel extrahiert. Trocknen der organischen Phase und Einengen lieferten ein Öl, welches durch Chromatographie auf Kieselgel (Toluol – Ethanol – Gradienten unter Zusatz von 0,3% Trieth – ylamin) gereinigt wurde. Man erhielt 5,40 g (46%) reine Titelverbindung als bräunliches Öl.
Weitere 6,00 g des gewünschten Produktes wurden zusammen mit geringeren Mengen polarerer Verunrei – nigungen eluiert (Diese Fraktion war für weitere Umsetzungen von hinreichender Reinheit).
$R_f$ (Toluol – Methanol 4:1): 0,17

Beispiel 12

2 – (2 – Ethoxycarbonylamino – ethyl)amino – 8 – methoxy – 1,2,3,4 – tetrahydronaphtahalin Hydrochlorid

Aus dem Reinprodukt des Beispiels 11 wurde mit etherischer Salzsäure das Hydrochlorid gewonnen. Schmelzpunkt: 80° C (unter Zersetzung)

Beispiel 13

2 – [N – (3 – Dimethylaminopropyl) – N – propionyl]amino – 8 – methoxy – 1,2,3,4 – tetrahydronaphthalin

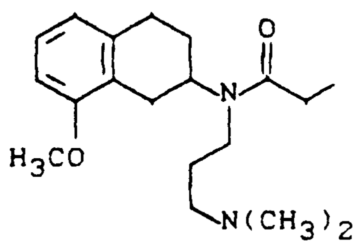

Zum Gemisch von 1,30 g (5 mmol) 2 – (3 – dimethylaminopropyl)amino – 8 – methoxy – 1,2,3,4 – tetrahydro – naphthalin in 35 ml Ether und 7 ml Wasser wurden bei 0° C 3,3 g (25 mmol) Propionsäureanhydrid und 22 ml 3N Natronlauge gegeben. Nach 1 h intensivem Rühren wurde nochmals die gleiche Menge Propions – äureanhydrid sowie 16 ml 3N Natronlauge zugegeben. Nach einer weiteren Stunde erfolgte der Zusatz von weiteren 2,5 g Propionsäureanhydrid und 12 ml 3N Natronlauge. Nach 15 h Rühren bei Raumtemperatur wurde die organische Phase abgetrennt. Die wäßrige Phase wurde zweimal mit Ether extrahiert. Die vereinigten organischen Extrakte wurden mit gesättigter Kochsalzlösung gewaschen, über Magnesiumsulfat getrocknet und im Vakuum vom Lösemittel befreit. Man erhielt so 1,20 g (75%) der freien Base als helles Öl.
$R_f$ (Toluol – Methanol 4:1): 0,22
IR (Chloroform): 2981, 1626, 1588, 1469

MS : 318, 217, 190, 160

Beispiel 14

2 – [N – (3 – Dimethylaminopropyl) – N – propionyl]amino – 8 – methoxy – 1,2,3,4 – tetrahydronaphthalin Hydrochlorid

Mit etherischer Salzsäure ließ sich das Hydrochlorid der Verbindung aus Beispiel 13 als Schaum gewinnen.

| Analyse ($C_{19}H_{30}N_2O_2$ x HCl x $H_2O$): | | | |
|---|---|---|---|
| Ber.: | C 61,2 | H 8,9 | N 7,5 |
| Gef.: | C 61,5 | H 8,9 | N 7,5 |

Beispiel 15

2 – [N – (2 – Diethylaminoethyl) – N – acetyl]amino – 8 – methoxy – 1,2,3,4 – tetrahydronaphthalin

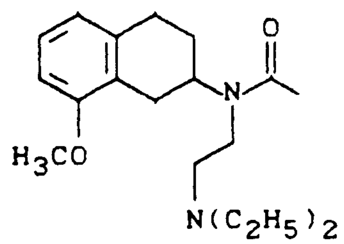

Zum Gemisch von 1,20 g (4,3 mmol) 2 – (2 – Diethylaminoethyl)amino – 8 – methoxy – 1,2,3,4 – tetrahydro – naphthalin in 35 ml Ether und 7 ml Wasser wurden bei 0 ° C 2,2 g (22 mmol) Essigsäureanhydrid und 20 ml 3 N Natronlauge gegeben.

Nach 1 h intensivem Rühren wurde nochmals die gleiche Menge Essigsäureanhydrid sowie 15 ml 3N Natronlauge zugegeben. Nach einer weiteren Stunde erfolgte der Zusatz von weiteren 1,6 g Essigsäure – anhydrid und 12 ml 3N Natronlauge. Nach 15 h Rühren bei Raumtemperatur wurde die organische Phase abgetrennt. Die wäßrige Phase wurde zweimal mit Ether extrahiert. Die vereinigten organischen Extrakte wurden mit gesättigter Kochsalzlösung gewaschen, über Magnesiumsulfat getrocknet und im Vakuum vom Lösemittel befreit. Das so erhaltene Rohprodukt wurde durch Chromatographie auf Kieselgel (Toluol – Essigester – Gradienten) gereinigt. Dies lieferte 0,50 g (37%) der Titelverbindung als gelbes Öl.
$R_f$ (Toluol – Methanol 4:1): 0,22
IR (Chloroform): 3002, 2970, 1625, 1588, 1470
MS: 318, 246, 161

Beispiel 16

2 – [N – (2 – Diethylaminoethyl) – N – acetyl]amino – 8 – methoxy – 1,2,3,4 – tetrahydronaphthalin Hydrochlorid

Mit etherischer Salzsäure konnte das Hydrochlorid der Verbindung aus Beispiel 15 als farbloser Festkörper in Ether gefällt werden.
Schmelzpunkt: ca. 100 ° C Sintern, ca. 150 ° C unter Zersetzung

| Analyse ($C_{19}H_{30}N_2O_2$ x HCl) | | | |
|---|---|---|---|
| Ber.: | C 64,3 | H 8,8 | N 7,9 |
| Gef.: | C 63,9 | H 8,8 | N 7,9 |

Beispiel 17

2-[N-(3-Dimethylaminopropyl)-N-propyl]amino-8-methoxy-1,2,3,4-tetrahydronaphthalin

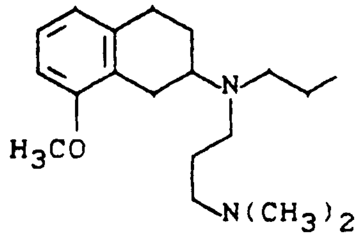

Die Lösung von 1,30g (5,0 mmol) 2-(3-Dimethylaminopropyl)amino-8-methoxy-1,2,3,4-tetrahydronaphthalin, 2,90 g (50 mmol) Propionaldehyd und 1,50 g (25 mmol) Eisessig wurde 30 min bei 0° C gerührt. Nach Zugabe von 0,70 g (10 mmol) Natriumcyanoborhydrid wurde 15 h bei Raumtemperatur gerührt. Die Reaktionsmischung wurde am Rotationsverdampfer weitgehend eingeengt, mit tert.-Butylmethylether aufgenommen und 30 min intensiv mit 20% Natronlauge verrührt. Die wäßrige Phase wurde sorgfältig mit tert.Butylmethylether extrahiert. Waschen der vereinigten organischen Phasen mit Wasser und gesättigter Kochsalzlösung, Trocknen über Kaliumcarbonat und Einengen am Rotationsverdampfer ergab die gewünschte Verbindung als helles Öl.
Ausbeute: 1,50 g (98%)
$R_f$ (Chloroform-Methanol-Triethylamin 20:10:0,1): 0,26
IR (Chloroform): 3040, 3007, 2936, 1653, 1587
MS: 304, 259, 232, 218, 204

Beispiel 18

2-[N-(3-Dimethylaminopropyl)-N-propyl]amino-8-methoxy-1,2,3,4-tetrahydronaphthalin Dihydrochlorid

Mit etherischer Salzsäure erhielt man aus der Verbindung des Beispiels 17 1,3 g (70%) Dihydrochlorid als amorphes, sehr hygroskopisches Pulver.

| Analyse ($C_{19}H_{32}N_2O_2$ x 2 HCl x $H_2O$): | | | | |
|---|---|---|---|---|
| Ber.: | C 55,2 | H 9,3 | N 6,8 | Cl 17,2 |
| Gef.: | C 55,8 | H 9,1 | N 6,3 | Cl 17,3 |

Beispiel 19

2 – [N – (2 – Diethylaminoethyl) – N – propyl]amino – 8 – methoxy – 1,2,3,4 – tetrahydronaphthalin

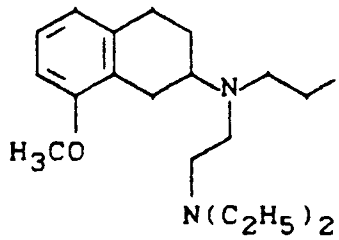

Die Lösung von 1,20 g (4,3 mmol) 2 – (2 – Diethylaminoethyl)amino – 8 – methoxy – 1,2,3,4 – tetrahydro – naphthalin, 2,50 g (43 mmol) Propionaldehyd und 0,80 g (13 mmol) Eisessig wurde 30 min bei 0° C gerührt. Anschließend wurde mit 1,10 g (17 mmol) Natriumcyanoborhydrid versetzt und über Nacht bei Raumtemperatur gerührt.

Die Reaktionsmischung wurde am Rotationsverdampfer weitgehend eingeengt, mit tert. – Butylmethy – lether aufgenommen und 30 min intensiv mit 20% Natronlauge verrührt. Die wäßrige Phase wurde sorgfältig mit tert. – Butylmethylether extrahiert. Waschen der vereinigten organischen Phasen mit Wasser und gesättigter Kochsalzlösung, Trocknen über Kaliumcarbonat und Einengen am Rotationsverdampfer ergab das Rohprodukt als Öl.
Chromatographie auf Kieselgel (Toluol – Essigester – Gradienten) ergab 0,85 g (62%) Titelverbindung als gelbes Öl.
$R_f$ (Toluol – Methanol 4:1): 0,17
MS : 318, 232, 161
IR (Chloroform): 3058, 2972, 1586

Beispiel 20

2 – [N – (2 – Diethylaminoethyl) – N – propyl]amino – 8 – methoxy – 1,2,3,4 – tetrahydronaphthalin Dihydro – chlorid

Das aus der Verbindung des Beispiels 19 durch Behandeln mit etherischer Salzsäure zugängliche Dihydrochlorid fiel amorph an und war sehr hygroskopisch.

| Analyse ($C_{20}H_{34}N_2O$ x 2 HCl x 2 $H_2O$): | | | |
|---|---|---|---|
| Ber.: | C 56,2 | H 9,4 | N 6,6 |
| Gef.: | C 56,7 | H 9,4 | N 6,5 |

Beispiel 21

2 – [N – (2 – Ethoxycarbonylamido – ethyl) – N – propyl]amino – 8 – methoxy – 1,2,3,4 – tetrahydronaphthalin

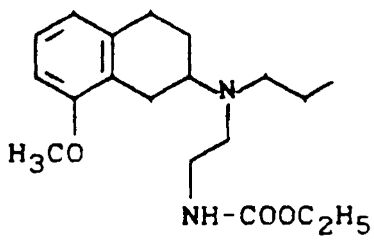

5,09g (20 mmol) 2 – (2 – Ethoxycarbonylamido – ethyl)amino – 8 – methoxy – 1,2,3,4 – tetrahydronaphthalin, 11,6 g (200 mmol) Propionaldehyd und 2,40 g (40 mmol) Eisessig wurden 30 min bei 0° C in 150 ml Methanol gerührt. Danach wurden 2,50 g (40 mmol) Natriumcyanoborhydrid zugegeben und 15 Stunden bei Raumtemperatur gerührt.

Die Reaktionsmischung wurde am Rotationsverdampfer weitgehend eingeengt, mit tert. – Butylmethylether aufgenommen und 30 min intensiv mit Natronlauge/Wasser bei pH 10 verrührt. Die wäßrige Phase wurde sorgfältig mit tert. – Butylmethylether extrahiert. Waschen der vereinigten organischen Phasen mit Wasser und gesättigter Kochsalzlösung, Trocknen über Magnesiumsulfat und Einengen am Rotationsverdampfer ergab das Rohprodukt als Öl. Das erhaltene Rohprodukt wurde auf Kieselgel (Toluol – Ethanol – Gradienten) chromatographiert. So erhielt man 5,20 g (78%) der gewünschten Verbindung als hellbraunes Öl.
$R_f$ (Toluol – Methanol 4:1): 0,25
IR (Chloroform, $CHCl_3$): 3403, 2931, 1702, 1585

Beispiel 22

2 – [N – (2 – Ethoxycarbonylamido – ethyl) – N – propyl]amino – 8 – methoxy – 1,2,3,4 – tetrahydronaphthalin Hydrochlorid

Mit etherischer Salzsäure war aus der Verbindung des Beispiels 21 das Hydrochlorid als Schaum zugänglich.
MS (C.I., reagent gas: $NH_3$): 335, 289, 232, 161

Beispiel 23

2 – (N – Chinuclidin – 3 – yl – N – propyl)amino – 8 – methoxy – 1,2,3,4 – tetrahydronaphthalin

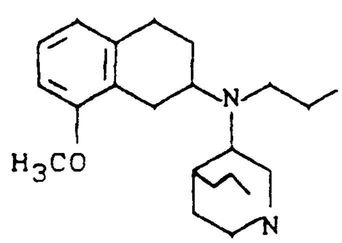

1,60 g (5,6 mmol) 2 – (Chinuclidin – 3 – yl)amino – 8 – methoxy – 1,2,3,4 – tetrahydronaphthalin (aus 8 – Methoxy – 2 – tetralon und 3 – Aminochinuclidin durch reduktive Aminierung in der üblichen Weise erhalten), 3,20 g (56 mmol) Propionaldehyd und 1,70 g (28 mmol) Eisessig wurden 30 min bei 0° C gerührt. Dann wurde 0,80 g (11 mmol) Natriumcyanoborhydrid zugegeben und es wurde 15 h bei Raumtemperatur gerührt. Die Reaktionsmischung wurde eingeengt, mit Toluol aufgenommen und nochmals eingeengt, mit tert – Butylmethylether aufgenommen und 30 min intensiv 20% Natronlauge verrührt. Die wäßrige Phase

wurde sorgfältig mit tert.-Butylmethylether extrahiert. Waschen der vereinigten organischen Phasen mit Wasser und gesättigter Kochsalzlösung, Trocknen über Kaliumcarbonat und Einengen am Rotationsverdampfer ergab das Rohprodukt als Öl (2,0 g). Chromatographie auf Kieselgel (Toluol-Ethanol 3:1) lieferte die Titelverbindung als Sirup.
Ausbeute: 0,50 g (27%).
$R_f$ (Chloroform-Methanol 2:1): 0,13
MS : 328, 216, 168, 160

Beispiel 24

2-(N-Chinuclidin-3-yl-N-propyl)amino-8-methoxy-1,2,3,4-tetrahydronaphthalin Dihydrochlorid

Mit etherischer Salzsäure wurde aus etherischer Lösung der Verbindung des Beispiels 23 das Dihydrochlorid in Form farbloser Kristalle gefällt.
Schmelzpunkt: 200 - 205° C

Beispiel 25

2-(N-Cyanomethyl-N-propyl)amino-8-methoxy-1,2,3,4-tetrahydronaphthalin

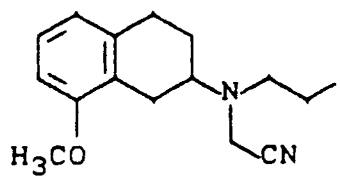

3,2 g (15 mmol) 8-Methoxy-2-propylamino-1,2,3,4-tetrahydronaphthalin, 5,6 g (72 mmol) Chloracetonitril, 9,6 g (72 mmol) Kaliumcarbonat wurden in 65 ml 2-Butanon suspendiert, mit 80 mg Natriumiodid versetzt und über Nacht bei 60° C gerührt. Nach Filtration über Celite wurde vom Solvens befreit (Rotationsverdampfer). Nach Chromatographie auf Kieselgel (Toluol-Essigester-Gradienten) erhielt man 3,65 g (98%) schwach gelbes Öl.
$R_f$ (Toluol-Methanol 4:1): 0,81
MS : 258, 229, 161

Beispiel 26

2-(N-Cyanomethyl-N-propyl)amino-8-methoxy-1,2,3,4-tetrahydronaphthalin Hydrochlorid

Mit etherischer Salzsäure gewann man aus der freien Base (Beispiel 25) das Hydrochlorid als farblose Kristalle.
Schmelzpunkt: 164 - 166° C

Beispiel 27

8-Methoxy-2-[N-propyl-N-(3-phthalimidoyl-propyl)]amino-1,2,3,4-tetrahydronaphthalin

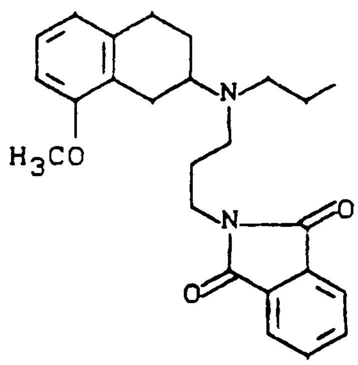

1,55 g (7,0 mmol) 8-Methoxy-2-N-propylamino-1,2,3,4-tetrahydronaphthalin,1,40 g (14 mmol) Triethylamin, 1,90 g (7,0 mmol) N-3-Brompropyl)phthalimid und eine Spatelspitze Natriumiodid wurden in 35 ml absolutem Dimethylformamid 4 h bei 60° C gerührt. Nach Abziehen des Lösemittels am Rotationsverdampfer (zuletzt bei 0,2 Torr) wurde das Hauptprodukt durch Flash-Chromatographie auf Kieselgel (Toluol-Essigester-Gradienten 0 - 33% Essigester) als gelbliches Öl gewonnen.
Ausbeute: 0,90 g
$R_f$ (Toluol-Essigester 1:1): 0,12

Beispiel 28

8-Methoxy-2-[N-propyl-N-(3-phthalimidoyl-propyl)]amino-1,2,3,4-tetrahydronaphthalin Hydrochlorid

Mit etherischer Salzsäure gewann man aus Beispiel 27 das Hydrochlorid als farblosen, hygroskopischen Feststoff.
Ausbeute: 0,58 g
Schmelzpunkt: 80 - 100° C

Beispiel 29

2-[N-(N,N-Diethylcarbamoylmethyl)-N-propyl]amino-8-methoxy-1,2,3,4-tetrahydronaphthalin

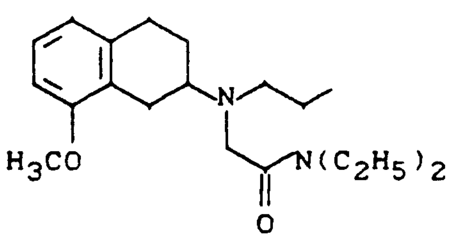

Bei 60° C wurden 0,80 g (3,7 mmol) 8-Methoxy-2-propylamino-1,2,3,4-tetrahydronaphthalin, 2,75 g (18 mmol) Chloressigsäurediethylamid, 2,50 g (18 mmol) gepulvertes Kaliumcarbonat und 30 mg Natriumiodid in 10 ml 2-Butanon zur Reaktion gebracht. Das Reaktionsgemisch wurde mit 100 ml tert.-Butylmethylether verdünnt. Vom Ungelösten wurde abfiltriert und das Lösemittel entfernt. Der hierbei verbliebene Rückstand wurde mit tert.-Butylmethylether aufgenommen. Nach Ansäueren wurde die organische Phase abgetrennt und verworfen. Die wäßrige Phase wurde basisch gestellt und mit dem letztgenannten Lösemittel extrahiert. Nach Trocknen und Einengen der organischen Phase verblieben 1,2 g Rohprodukt als braunes Öl.
$R_f$ (Toluol-Methanol 4:1): 0,5

MS (C.I. – Spektrum reagent gas $NH_3$): 333 (M + 1), 232, 161

Beispiel 30

2 – [N – (N,N – Diethylcarbamoylethyl) – N – propyl]amino – 8 – methoxy – 1,2,3,4 – tetrahydronaphthalin Hydrochlorid

Aus dem Rohprodukt des Beispiels 29 wurde mit etherischer Salzsäure 795 mg (58%) Hydrochlorid als hellbrauner Feststoff gewonnen.
MS (C.I., reagent gas: $NH_3$): 333 (M + 1), 232, 161.

Beispiel 31

2 – (N – Ethoxycarbonylmethyl – N – propyl)amino – 8 – methoxy – 1,2,3,4 – tetrahydronaphthalin

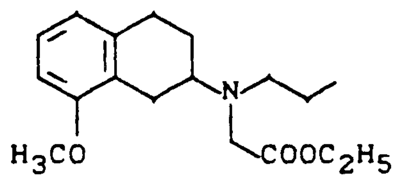

2,6 g (12 mmol) 8 – Methoxy – 2 – propylamino – 1,2,3,4 – tetrahydronaphthalin, 1,7 g (12 mmol) Kaliumcarbonat, 6,0 g Molsieb (3Å), 2,0 g (12 mmol) Bromessigsäureethylester und 100 mg Natriumiodid in 42 ml Ethanol wurden 2 h zum Rückfluß erhitzt. Nach dieser Zeit wurden nochmals 0,25 äquivalente Kaliumcarbonat und 0,25 equivalent Bromessigsäureethylester zugegeben und weitere 2 h zum Rückfluß erhitzt. Filtration über Celite und Einengen lieferten ein Rohprodukt, welches durch Chromatographie auf Kieselgel (Toluol – Essigester – Gradienten) gereinigt wurde. Man erhielt auf diese Weise 3,5 g farblosen Sirup (enthielt nach NMR noch ca. 4% Toluol).
$R_f$ (Toluol – Methanol 4:1): 0,79
IR (Chloroform): 1736, 1587, 1378, 1255

Beispiel 32

2 – (N – Ethoxycarbonylmethyl – N – propyl)amino – 8 – methoxy – 1,2,3,4 – tetrahydronaphthalin Hydrochlorid

Durch Behandeln mit etherischer Salzsäure erhielt man aus 600 mg der freien Base aus Beispiel 31 580 mg Hydrochlorid als farblosen, amorphen, hygroskopischen Feststoff.

| Analyse ($C_{18}H_{27}NO_3$ x HCl x $H_2O$): | | | |
|---|---|---|---|
| Ber.: | C 60,0 | H 8,3 | N 3,9 |
| Gef.: | C 60,0 | H 8,4 | N 3,7 |

Beispiel 33

2 – {4 – [N – (8 – Methoxy – 1,2,3,4 – tetrahydro – 2 – naphthyl) – N – propyl – amino]butyl} – 1,2 – benzisothiazol – 3(2H) – on – 1,1 – dioxid

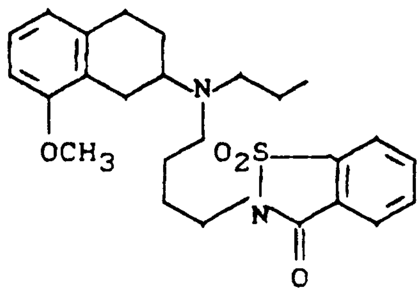

Zur Lösung von 1,3 g (6,0 mmol) 8 – Methoxy – 2 – propylamino – 1,2,3,4 – tetrahydronaphthalin und 1,3 g (12 mmol) Triethylamin in 15 ml Dimethylformamid wurden 1,9 g (6,0 mmol) 2 – (4 – Brombutyl) – 1,2 – benzisothiazol – 3(2H) – on – 1,1 – dioxid zugetropft. Nach 15 h Rühren bei Raumtemperatur wurde eine Spatelspitze Natriumiodid zugegeben und auf 60° C erhitzt. Nach 4 h bei dieser Temperatur wurden 0,4 g obigen Aminotetralins und 0,3 g Triethylamin zugegeben. Chromtographie des nach Entfernen des Lösemittels im Vakuum erhaltenen Rohproduktes auf Kieselgel (Toluol – Essigester – Gradienten) ergab 0.70 g (25%) der Titelverbindung als gelbliches Öl.
$R_f$ (Toluol – Essigester 1:1): 0,36
MS : 456, 427, 232, 219, 196, 190, 161.

Beispiel 34

2 – {4 – [N – (8 – Methoxy – 1,2,3,4 – tetrahydro – 2 – naphthyl) – N – propyl – amino]butyl} – 1,2 – benzisothiazol – 3(2H) – on – 1,1 – dioxid Hydrochlorid

Durch Behandeln der Verbindung des Beispiels 33 mit etherischer Salzsäure erhielt man das Hydrochlorid als farblosen Festkörper.
Schmelzbereich: 65 – 80° C

Beispiel 35

2 – [N – (3 – Aminopropyl) – N – propyl]amino – 8 – methoxy – 1,2,3,4 – tetrahydronaphthalin

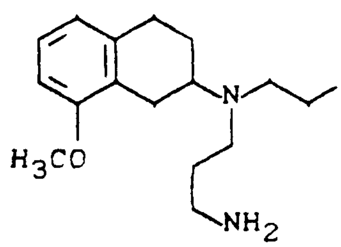

Bei Raumtemperatur wurden 135 mmol Aluminiumhydrid in 100 ml Ether (hergestellt bei 0° C aus 5,1 g Lithiumaluminiumhydrid und einer Mischung von 3,3 g 95% Schwefelsäure und 3,3 g 20% Oleum in Ether mit anschließendem einstündigem Rühren bei Raumtemperatur) vorsichtig mit einer Lösung von 12,2 g (45 mmol) 2 – [N – (2 – Cyanoethyl) – N – propyl]amino – 8 – methoxy – 1,2,3,4 – tetrahydronaphthalin in 20 ml Ether versetzt. Es wurden 2 h zum Rückfluß erhitzt, anschließend 15 h bei Raumtemperatur nachgerührt. Nach Zugabe von 30 ml Wasser und 60 ml 10%iger Natronlauge wurde die organische Phase vom voluminösen Niederschlag abgetrennt. Letzterer wurde dreimal in der Siedehitze mit Ether ausgelaugt. Trocknen der organischen Phase (Kaliumcarbonat) und Eindampfen des Lösemittels ergaben 9,30 g (75%) braunes, zähes Öl,

$R_f$ (Chloroform – Methanol – Ammoniak 30:10:1): 0,52
MS : 276, 247, 232

Beispiel 36

2 – [N – (2 – Aminoethyl) – N – propyl]amino – 8 – methoxy – 1,2,3,4 – tetrahydronaphthalin

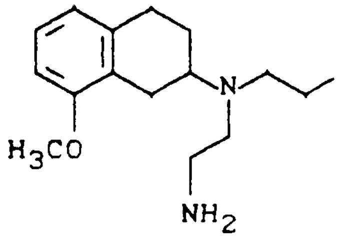

Bei 0° C wurden 1,4 g (13 mmol) Lithiumaluminiumhydrid in 25 ml Ether langsam mit einer Mischung von 0,9 g 95% Schwefelsäure und 0,9 g 20% Oleum versetzt. Nach 1 h Rühren bei Raumtemperatur wurde eine Lösung von 3,1 g (12 mmol) 2 – (N – Cyanomethyl – N – propyl)amino – 8 – methoxy – 1,2,3,4 – tetrahydronaphthalin in 5 ml Ether zugetropft. Es wurde 2 h zum Rückfluß erhitzt. Danach wurde vorsichtig mit 10 ml Wasser versetzt, gefolgt von 20 ml 10% Natronlauge. Vom Ungelösten wurde abgetrennt; dieser Feststoff wurde mehrmals mit Ether gewaschen. Nach Trocknen der organischen Phase über Kaliumcarbonat und nach Entfernen des Lösemittels im Vakuum (zuletzt Hochvakuum) erhielt man 2,3 g (73%) der Titelverbindung als Öl,
$R_f$ (Chloroform – Methanol 2:1 + 1% Triethylamin): 0,1
MS (C.I., reagent gas: $NH_3$): 263, 232, 161

Beispiel 37

2 – [N – (2 – Aminoethyl) – N – propyl]amino – 8 – methoxy – 1,2,3,4 – tetrahydronaphthalin Dihydrochlorid

Durch Behandeln von 1,0 g der freien Base aus Beispiel 3 mit etherischer Salzsäure erhielt man 1,3 g Dihydrochlorid als farblosen Feststoff.
Schmelzpunkt: ab 73° C Sintern

Beispiel 38

8 – Methoxy – 2 – [N – (2 – methylaminoethyl) – N – propyl]amino – 1,2,3,4 – tetrahydronaphthalin

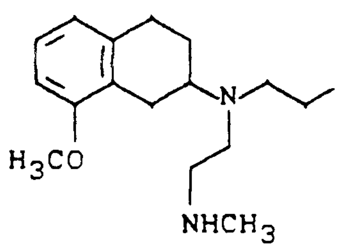

Zur Suspension von 0,75 g (20 mmol) Lithiumaluminiumhydrid in 25 ml Ether wurden bei Raumtemperatur 3,40 g (10 mmol) 2 – [N – (2 – Ethoxycarbonylamido – ethyl) – N – propyl]amino – 8 – methoxy – 1,2,3,4 – tetrahydronaphthalin in 25 ml Ether langsam zugetropft. Nach Rühren über Nacht wurde mit 15 ml Ether verdünnt. Anschließend wurden vorsichtig nacheinander 1 ml Wasser, 0,75 ml Natronlauge und 3,0 ml Wasser zugegeben. Zum Trocknen wurden 10 g Kaliumcarbonat zugesetzt und 20 min gerührt. Filtration und Einengen lieferten 2,7 g Rohprodukt. Zweimalige Chromatographie über Kieselgel (Chloroform – Methanol – Gradienten) ergab 0,60 g (33%) der Titelverbindung als farblosen Sirup.
$R_f$ (Chloroform – Methanol – Triethylamin 2:1:0,01): 0,29

Beispiel 39

8 – Methoxy – 2 – [N – (2 – methylaminoethyl) – N – Propyl]amino – 1,2,3,4 – tetrahydronaphthalin Dihydro – chlorid

Aus der Verbindung des Beispiels 38 war durch Behandeln mit etherischer Salzsäure das als sehr hygroskopischer, amorpher, farbloser Feststoff anfallende Dihydrochlorid zugänglich.

| Analyse ($C_{17}H_{28}N_2O$ x 2 HCl x $H_2O$): | | | |
|---|---|---|---|
| Ber. | C 55,6 | H 8,8 | N 7,6 |
| Gef. | C 55,4 | H 8,7 | N 7,3 |

Beispiel 40

8 – Methoxy – 2 – (1 – methylpiperidin – 4 – yl)amino – 1,2,3,4 – tetrahydronaphthalin

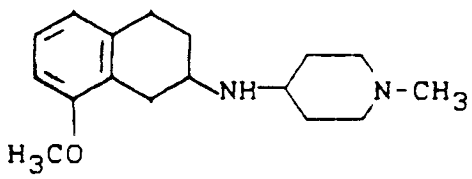

Zur Suspension von 0,27 g (7,2 mmol) Lithiumaluminiumhydrid in 10 ml Ether wurde bei Raumtemperatur eine Lösung von 1,20 g (3,6 mmol) 2 – (1 – Ethoxycarbonyl – piperidin – 4 – yl)amino – 8 – methoxy – 1,2,3,4 – tetrahydronaphthalin in 10 ml Ether zugetropft. Nach 15 h Rühren bei Raumtemperatur wurde mit 30 ml Ether verdünnt. Anschließend wurden nacheinander vorsichtig 0,3 ml Wasser, 0,25 ml 20% Natronlauge und 1 ml Wasser zugetropft. Zum Trocknen wurden 3 g Kaliumcarbonat zugesetzt und 20 min gerührt. Durch Filtration und Einengen (zuletzt Hochvakuum) erhielt man 0,90 g (91%) der Titelverbindung als Öl.
$R_f$ (Chloroform – Methanol – Triethylamin 20:10:0,1): 0,26

Beispiel 41

8 – Methoxy – 2 – (1 – methylpiperidin – 4 – yl)amino – 1,2,3,4 – tetrahydronaphthalin Dihydrochlorid

Das durch Umsetzung der Verbindung aus Beispiel 40 mit etherischer Salzsäure zugängliche Dihydro – chlorid fiel in Form hellgelber Kristalle an.
Schmelzpunkt: >260 ° C

| Analyse ($C_{17}H_{26}N_2O$ x 2 HCl x $H_2O$) | | | |
|---|---|---|---|
| Ber.: | C 56.2 | H 8.3 | N 7.7 |
| Gef.: | C 55.9 | H 8.2 | N 7.7 |

Beispiel 42

2 – [N – (2 – Hydroxyethyl) – N – propyl]amino – 8 – methoxy – 1,2,3,4 – tetrahydronaphthalin

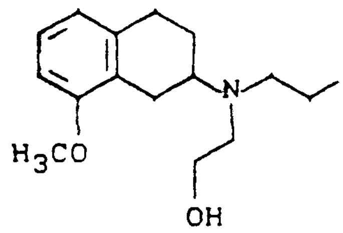

Zur Suspension von 0,22 g (5,7 mmol) Lithiumaluminiumhydrid in 10 ml Ether wurden 2,9 g (9,5 mmol) 2 – (N – Ethoxycarbonylethyl – N – propyl)amino – 8 – methoxy – 1,2,3,4 – tetrahydronaphthalin in 20 ml Ether zugetropft. Nach 1 h Rühren bei Raumtemperatur wurde nacheinander mit 0,3 ml Wasser, 0,35 20% Natronlauge und 1,0 ml Wasser versetzt. Es wurde Trockenmittel (Magnesiumsulfat) zugegeben, 20 min bei Raumtemperatur gerührt, über Celite filtriert und um Vakuum (zuletzt im Hochvakuum) vom Lösemittel befreit. Es verblieben 1,8 g (72%) klares Öl.
$R_f$ (Toluol – Ethanol 6:1): 0,25
MS : 263, 232, 161
IR (Chloroform): 3670, 3430, 1587.

Beispiel 43

2 – [N – (2 – Carbamoylethyl) – N – propyl]amino – 8 – methoxy – 1,2,3,4 – tetrahydronaphthalin

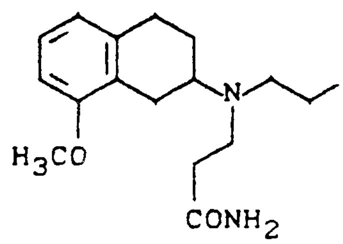

0,54 g (2,0 mmol) 2 – [N – (2 – Cyanoethyl) – N – propyl]amino – 8 – methoxy – 1,2,3,4 – tetrahydronaphthalin wurden 2 h bei Raumtemperatur mit 2 ml konzentrierter Salzsäure gerührt. Nach Verdünnen mit 20 ml Wasser wurde unter Kühlen mit 20% Natronlauge pH 7 eingestellt und die wäßrige Phase sorgfältig mit Essigester extrahiert. Das nach Trocknen und Einengen der organischen Phase erhaltene Rohprodukt wurde durch Chromatographie auf Aluminiumoxid (neutral, Aktivitätsstufe III) mit Toluol – Essigester – Gradienten gereinigt.
$R_f$ (Aluminiumoxid, Toluol – Essigester 1:1): 0,1
MS : 290, 261, 232, 161

Beispiel 44

2 – [N – (2 – Carbamoylethyl) – N – propyl]amino – 8 – methoxy – 1,2,3,4 – tetrahydronaphthalin Hydrochlorid

Durch Behandeln mit etherischer Salzsäure in Ether erhielt man aus der Verbindung des Beispiels 43 Hydrochlorid als farblosen Feststoff.
Schmelzpunkt: 80 ° C (Sintern ab 65 ° C)

Beispiel 45

8 – Methoxy – 2 – {N – [2 – (4 – toluol – sulfonamido)ethyl] – N – propyl}amino – 1,2,3,4 – tetrahydronaphthalin Hydrochlorid

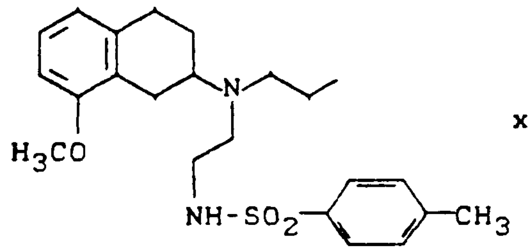

x HCl

Bei 0° C wurden zur Lösung von 1,00 g (4,0 mmol) 2 – [N – (2 – Aminoethyl) – N – propyl]amino – 8 – methoxy – 1,2,3,4 – tetrahydronaphthalin in 10 ml Dichlormethan 0,77 g (4,0 mmol) Tosylchlorid in 5 ml Dichloromethan zugetropft. Nach 15 h Rühren bei Raumtemperatur wurde im Vakuum vom Lösemittel befreit. Digerieren des Rückstandes in Ether ergab 1,6 g des Hydrochlorid der Titelverbindung als gelblichen, feinkristallinen Feststoff.
Schmelzbereich: ab 100° C under Zersetzung

Beispiel 46

8 – Methoxy – 2 – {N – [2 – (4 – toluol – sulfonamido)ethyl] – N – propyl}amino – 1,2,3,4 – tetrahydronaphthalin

Durch Behandeln der Verbindung aus Beispiel 45 mit Natriumhydrogencarbonatlösung erhielt man die freie Base.
$R_f$ (Toluol – Ethanol 3:1): 0,53

Beispiel 47

2 – {N – [3 – (4 – Fluorphenylsulfonamido)propyl] – N – propyl}amino – 8 – methoxy – 1,2,3,4 – tetrahydronaphthalin Hydrochlorid

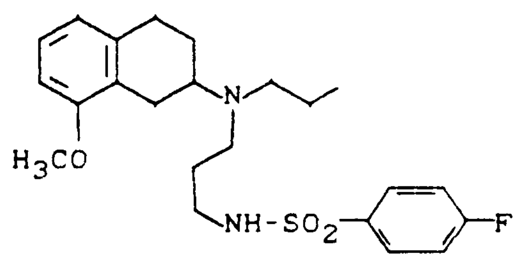

x HCl

Bei 0° C wurden zur Lösung von 0,55 g (2,0 mmol) 2 – [N – (3 – Aminopropyl) – N – propyl]amino – 8 – methoxy – 1,2,3,4 – tetrahydronaphthalin in 8 ml Methylenchlorid 0,39 g (2,0 mmol) 4 – Fluorphenylsulfo – chlorid in 5 ml Methylenchlorid zugetropft. Nach 15 h Rühren bei Raumtemperatur wurde eingeengt und aus ethanolischer Lösung durch Zusatz von Ether das Hydrochlorid in Form beiger Kristalle gefällt.
Ausbeute: 0,82 g (94%)
Schmelzbereich: 120 – 140° C (nach Sintern),
MS : 434, 405, 232, 219, 186, 161

Beispiel 48

2 – {N – [3 – (4 – Fluorphenylsulfonamido)propyl] – N – propyl}amino – 8 – methoxy – 1,2,3,4 – tetrahydronaphthalin

Durch Behandeln der Verbindung des Beispiels 47 mit Natriumhydrogencarbonatlösung erhielt man die freie Base.
$R_f$ (Toluol – Ethanol 3:1): 0,45

Beispiel 49

2 – [N – (2 – Methansulfonamidoethyl) – N – propyl]amino – 8 – methoxy – 1,2,3,4 – tetrahydronaphthalin Hydrochlorid

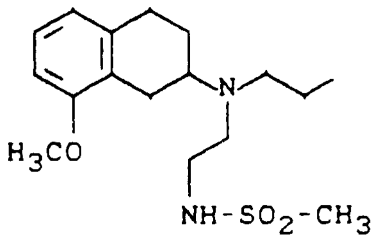

x HCl

Bei 0° C wurden zur Lösung von 520 mg (2,0 mmol) 2 – [N – (2 – Aminoethyl) – N – propyl]amino – 8 – methoxy – 1,2,3,4 – tetrahydronaphthalin in 5 ml Methylenchlorid 230 mg (2,0 mmol) Methansulfonsäurechlorid in 2 ml Methylenchlorid zugetropft. Nach 15 h Rühren bei Raumtemperatur wurde bei einer Badtemperatur von maximal +25° C am Rotationsverdampfer vom Lösemittel befreit. Digerieren des Rückstoffes mit Diethylether ergab nach Trocknen des Feststoffes im Hochvakuum 673 mg(89%) Hydrochlorid.
Schmp.: ab 100° C unter Zers.
MS (C.I., reagent gas: $NH_3$): 341 (M + 1), 232, 161

Beispiel 50

2 – [N – (2 – Methansulfonamidoethyl) – N – propyl] – 8 – methoxy – 1,2,3,4 – tetrahydronaphthalin

Durch Behandeln der Verbindung des Beispiels 49 mit Natriumhydrogencarbonatlösung erhielt man die freie Base.
$R_f$ (Chloroform – Methanol 2:1): 0,65

Beispiel 51

2 – {N – [2 – (3 – Chlorpropylsulfonamido)ethyl] – N – propyl}amino – 8 – methoxy – 1,2,3,4 – tetrahydronaphthalin

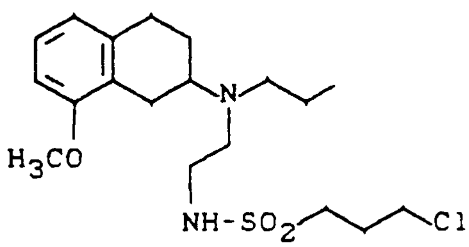

Zur Lösung von 2,70 g (10 mmol) 2 – [N – (2 – Aminoethyl) – N – propyl]amino – 8 – methoxy – 1,2,3,4 – tetrahydronaphthalin in 50 ml Dichlormethan wurden unter Feuchtigkeitsausschluß bei 0° C 1,80 g (10 mmol)

3 – Chlorpropansulfonsäurechlorid in 20 ml Dichlormethan langsam zugetropft. Nach 15 h bei Raumtempe – ratur wurde mit 150 ml Essigester und 50 ml Wasser verdünnt. Mit Natronlauge wurde pH 7 eingestellt. Extraktion mit Essigester, Waschen der organischen Phase mit gesättigter Kochsalzlösung und Trocknen über Magnesiumsulfat ergab nach Eindampfen des Lösemittels 4,3 g Rohprodukt.

Flashchromatographie auf Kieselgel (Toluol – Essigester – Gradienten) lieferte 4,10 g noch geringfügig mit Lösemittel behaftetes Reinprodukt.
$R_f$ (Toluol – Ethanol 3:1): 0,62
IR (Chloroform): 3319, 3019, 2961, 1587, 1470
MS (C.I., reagent gas: $NH_3$): 405/403 (M + 1), 367, 232, 161

Beispiel 52

2 – {N – [2 – (3 – Chlorpropylsulfonamido)ethyl] – N – propyl}amino – 8 – methoxy – 1,2,3,4 – tetrahydronaphthalin Hydrochlorid

Mit etherischer Salzsäure wurde aus der freien Base des Beispiels 51 das Hydrochlorid in einer Ausbeute von 92% als schwach gelblicher, hygroskopischer Feststoff gewonnen.
Schmelzbereich: 60 – 70° C

Beispiel 53

2 – {N – [2 – (4 – Chlorbutansulfonamido)ethyl] – N – propyl}amino – 8 – methoxy – 1,2,3,4 – tetrahydronaphthalin

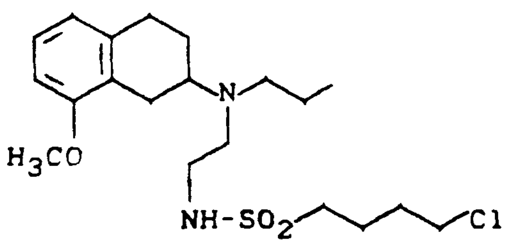

Zur Lösung von 2,70 g (10 mmol) 2 – [N – (2 – Aminoethyl) – N – propyl]amino – 8 – methoxy – 1,2,3,4 – te – trahydronaphthalin in 50 ml Dichlormethan wurden unter Feuchtigkeitsausschluß bei 0° C 2,00 g (10 mmol) 4 – Chlorbutansulfonsäurechlorid in 20 ml Dichlormethan langsam zugetropft. Nach 15 h bei Raumtempe – ratur wurde mit 150 ml Essigester und 50 ml Wasser verdünnt. Mit Natronlauge wurde pH 7 eingestellt. Extraktion mit Essigester, Waschen der organischen Phase mit gesättigter Kochsalzlösung und Trocknen über Magnesiumsulfat ergab nach Eindampfen des Lösemittels 4,5 g Rohprodukt.
Flash – Chromatographie auf Kieselgel (Toluol – Essigester – Gradienten) lieferte 3.2 g (77%) der Titelver – bindung als Sirup.
$R_f$ (Toluol – Ethanol – Triethylamin 30:10: 0,5): 0,46
IR (Chloroform): 3296, 3010, 2964, 1587, 1470
MS (C.I., reagent gas: $NH_3$): 419/417 (M + 1), 381, 232, 161

Beispiel 54

2 – {N – [2 – (4 – Chlorbutansulfonamido)ethyl] – N – propyl}amino – 8 – methoxy – 1,2,3,4 – tetrahydronaphthalin Hydrochlorid

Mit etherischer Salzsäure wurde aus der freien Base (Verbindung 53) das Hydrochlorid als schwach gelblicher, hygroskopischer Feststoff gewonnen.
Schmelzpunkt: 70° C

Beispiel 55

2-{2-{N-propyl-N-(8-methoxy-1,2,3,4-tetrahydro-2-naphthyl)]amino}ethyl-isothiazolidin-1,1-dioxid

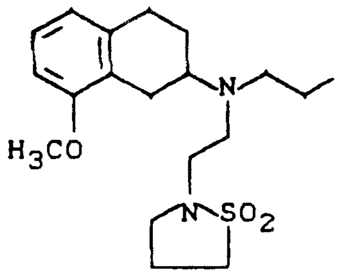

Zur Suspension von 144 mg (4,8 mmol) Natriumhydrid (80% in Paraffin) und einer Spatelspitze Natriumiodid in 20 ml Dimethylformamid wurden 1,60 g (4,0 mmol) 2-{N-[2-(3-Chlorpropylsulfonamido)ethyl]-N-propyl}amino-8-methoxy-1,2,3,4-tetrahydronaphthalin in 3 ml Dimethylformamid zugegeben. Nach einer Stunde Rühren bei Raumtemperatur wurde 0,3 ml Wasser zugegeben. Nach Einengen am Rotationsverdampfer wurde zwischen Toluol und Wasser verteilt. Durch gründliche Extraktion, Trocknen über Magnesiumsulfat und Einengen erhielt man 1,2 g (82%) der Titelverbindung als Öl.
$R_f$ (Toluol-Essigester 1:1): 0.28
MS (C.I., reagent gas: $NH_3$): 367 (M + 1), 232

Beispiel 56

2-{2-[N-propyl-N-(8-methoxy-1,2,3,4-tetrahydro-2-naphthyl)]amino}ethyl-isothiazolidin-1,1-dioxid Hydrochlorid

Durch Behandeln mit etherischer Salzsäure in Ether erhielt man das Hydrochlorid der Verbindung des Beispiels 55 als farblosen, hygroskopischen Feststoff.
Schmelzpunkt: 90 - 100° C

Beispiel 57

2-[2-N-propyl-N-(8-methoxy-1,2,3,4-tetrahydro-2-naphthyl)amino]ethyl-perhydro-1,2-thiazin-1,1-dioxid

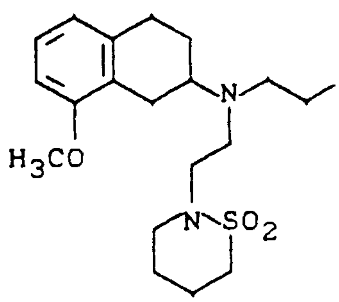

Zur Suspension von 126 mg (4,2 mmol) Natriumhydrid (80% in Paraffin) und 175 mg (1,1 mmol) Natriumiodid in 18 ml Dimethylformamid wurden 1,46 g (3,5 mmol) 2-{N-[2-(4-Chlorbutansulfonamido)ethyl]-N-propyl}amino-8-methoxy-1,2,3,4-tetrahydronaphthalin in 7 ml Dimethylformamid zugegeben. Nach einer Stunde Rühren bei Raumtemperatur wurde 0,1 ml Wasser zugegeben. Nach Einengen am Rotationsverdampfer wurde zwischen Toluol und Wasser verteilt. Durch gründliche Extraktion, Trocknen über Magnesiumsulfat und Einengen erhielt man 1,2 g (90%) der Titelverbindung als Öl.
$R_f$ (Toluol-Essigester 1:1):0,4

MS (C.I., reagent gas: $NH_3$): 381 (M + 1), 232, 161

Beispiel 58

2-[2-N-propyl-N-(8-methoxy-1,2,3,4-tetrahydro-2-naphthyl)amino]ethyl-perhydro-1,2-thiazin-1,1-dioxid Hydrochlorid

Durch Behandeln mit etherischer Salzsäure in Ether der Verbindung des Beispiels 57 erhielt man das Hydrochlorid als farblosen, hygroskopischen Feststoff.
Schmelzpunkt: 80 - 90° C

Beispiel 59

2-{-N-[2-(N,N-Dimethylaminosulfonamido)]ethyl-N-propyl}amino-8-methoxy-1,2,3,4-tetrahydronaphthalin

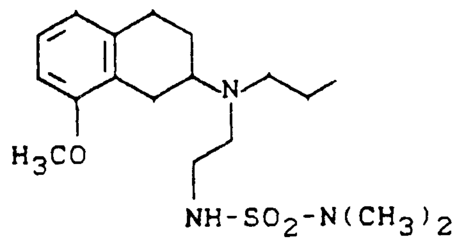

Zur Lösung von 0,53 g (2,0 mmol) 2-[N-(2-Aminoethyl)-N-propyl]amino-8-methoxy-1,2,3,4-tetrahydronaphthalin in 8 ml wasserfreiem Methylenchlorid wurde bei 0° C langsam 0,30 g (2.0 mmol) Dimethylamidosulfonsäurechlorid in 5 ml wasserfreiem Methylenchlorid zugetropft. Nach Rühren über Nacht wurde mit 50 ml Essigester und 30 ml Wasser verdünnt, mit Natronlauge auf pH 7 eingestellt und die organische Phase abgetrennt. Trocknen der organischen Phase (Magnesiumsulfat) und Einengen lieferte 0,6 g Rohprodukt.

Flash-Chromatographie auf Kieselgel (Toluol-Essigester-Gradienten) ergab 0,38 g (52%) der Titelverbindung als farblosen Sirup.
$R_f$ (Toluol-Ethanol-Triethylamin 30:10:0,5): 0.47
IR (Chloroform): 3009, 2929, 1584, 1468, 1372
MS (C.I., reagent gas: $NH_3$): 370 (M+1), 232, 153, 121

Beispiel 60

2-{N-[2-(N,N-Dimethylaminosulfonamido)]ethyl-N-propyl}amino-8-methoxy-1,2,3,4-tetrahydronaphthalin Hydrochlorid

Mit etherischer Salzsäure gewinnt man das Hydrochlorid der Verbindung 59 als hygroskopischen Feststoff.
Schmelzpunkt: 65 - 70° C

Beispiel 61

8 – Methoxy – 2 – [N – propyl – N – (2 – nicotinoylamido – propyl)]amino – 1,2,3,4 – tetrahydronaphthalin

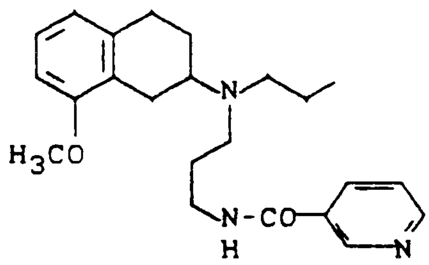

Die Lösung von 0,55 g (2,0 mmol) 2 – (N – 3 – Aminopropyl – N – propyl)amino – 8 – methoxy – 1,2,3,4 – tetrahydronaphthalin und 0,28 g (2,0 mmol) Nicotinsäuremethylester in 20 ml Tetrahydrofuran wurden mit 20 mg Natriumhydrid – Suspension (80% in Paraffin) versetzt und 5 h zum Rückfluß erhitzt. Das Solvens wurde am Rotationsverdampfer entfernt. Der verbliebene Rückstand wurde mit 30 ml Toluol und 30 ml eiskaltem pH 7 Puffer aufgenommen. Phasentrennung, Extraktion der wäßrigen Phase mit Essigester, Trocknen der vereinigten organischen Phasen über Magnesiumsulfat und Eindampfen des Lösemittels ergaben 680 mg Rohprodukt. Die Reinigung dieses Rohproduktes erfolgte durch Flash – Chromatographie auf Kieselgel (Laufmittel: Toluol, Toluol – Essigester 1:1 und Toluol – Ethanol 5:1). Man erhielt 520 mg (76%) der Titelverbindung als bräunlichen Sirup.
$R_f$ (HPTLC – Fertigplatte $NH_2$ F254s, Fa. Merck;
Toluol – Essigester 1:1): 0,3

Beispiel 62

8 – Methoxy – 2 – [N – propyl – N – (2 – nicotinoylamido – propyl)]amino – 1,2,3,4 – tetrahydronaphthalin Dihydrochlorid

Mit etherischer Salzsäure gewann man aus der Verbindung des Beispiels 61 das Dihydrochlorid als farblosen, hygroskopischen Feststoff.
Schmelzpunkt: 110 ° C

Beispiel 63

N – 6 – Chlorhexyl – N′ – {3 – [N – (8 – methoxy – 1,2,3,4 – tetrahydro – 2 – naphthyl) – N – propyl] – aminopropyl} – harnstoff

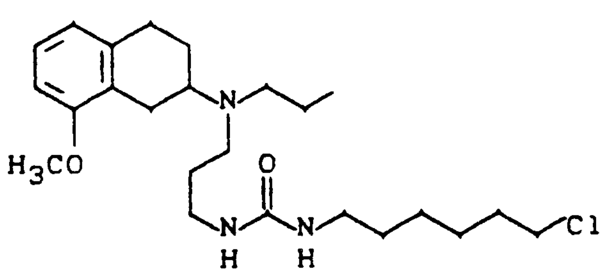

Zur Lösung von 550 mg (2,0 mmol) 2 – [N – (3 – Aminopropyl) – N – propyl]amino – 8 – methoxy – 1,2,3,4 – tetrahydronaphthalin in 10 ml wasserfreiem Tetrahydrofuran wurden bei 0 ° C 330 mg (2,0 mmol) 6 – Chlorhexylisocyanat in 3 ml Tetrahydrofuran langsam zugetropft. Nach 1 h bei 0 ° C wurde noch 1 h bei Raumtemperatur gerührt. Das nach Eindampfen des Lösemittels erhaltene Rohprodukt wurde durch Flash – Chromatographie auf Kieselgel (Toluol – Methanol – Gradienten) gereinigt.
Ausbeute: 950 mg gelblicher Sirup (enthielt noch Solvensreste)
$R_f$ (Toluol – Methanol 4:1): 0,26

MS : 439/437, 410/408, 396/394, 372, 232, 161

Beispiel 64

N – 6 – Chlorhexyl – N′ – {3 – [N – (8 – methoxy – 1,2,3,4 – tetrahydro – 2 – naphthyl) – N – propyl] – aminopropyl}harnstoff Hydrochlorid

Behandeln der Verbindung aus Beispiel 64 mit etherischer Salzsäure lieferte das Hydrochlorid als farblosen, amorphen Feststoff (795 mg).
$^1$H – NMR ($CD_3OD$): δ = 1,05 (t, 2H); 1,25 – 2,05 (m, 13H); 2,30 (m, 1H); 2,78 (m, 1H); 2,9 – 3,6 (m); 3,75 (m, 1H); 3,85 (s, 3H); 6,7 (m, 2H); 7,15 (m, 1H).

Beispiel 65

N – {2 – [N – (8 – Methoxy – 1,2,3,4 – tetrahydro – 2 – naphthyl) – N – propyl]aminoethyl} – N' – phenyl – harnstoff

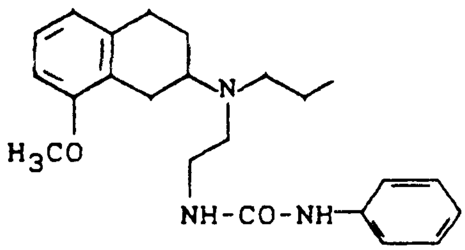

0,50 g (1,91 mmol) 2 – [N – (2 – Aminoethyl) – N – propyl]amino – 8 – methoxy – 1,2,3,4 – tetrahydronaphthalin wurde in 10 ml Tetrahydrofuran gelöst und bei 0° C langsam mit 0,23 g (1,92 mmol) Phenylisocyanat in der gleichen Menge Tetrahydrofuran versetzt. Nach 1 h bei 0° C und 1 h bei Raumtemperatur wurde eingeengt. Das Rohprodukt wurde durch Flash – Chromatographie auf Keiselgel (Toluol – Methanol – Gradienten) gereinigt. Man erhielt so 0,60 g (82%) der Titelverbindung als Sirup.
$R_f$ (Toluol – Ethanol – Triethylamin 3 : 1 : 0,05): 0,35
IR (Chloroform): 3430, 3360, 3030, 1666
MS (FAB): 382 (M + 1), 232, 161

Beispiel 66

N – {2 – [N – (8 – Methoxy – 1,2,3,4 – tetrahydro – 2 – naphthyl) – N – propyl]aminoethyl} – N′ – phenyl – harnstoff Hydrochlorid

Mit etherischer Salzsäure wurde aus der freien Base des Beispiels 65 das Hydrochlorid als amorpher, hygroskopischer Feststoff erhalten.

| Analyse ($C_{23}H_{31}N_3O_2$ x HCl x $H_2O$): | | | |
|---|---|---|---|
| Ber.: | C 63,5 | H 7,8 | N 9,6 |
| Gef.: | C 64,0 | H 7,8 | N 10,1 |

Beispiel 67

2 – [N – (2 – Ethyl – carbonyldioxy – ethyl) – N – propyl]amino – 8 – methoxy – 1,2,3,4 – tetrahydronaphthalin

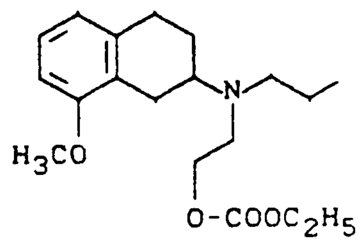

Zur Lösung von 0,70 g (8,8 mmol) Pyridin und 1,10 g (4,0 mmol) 2 – [N – (2 – Hydroxyethyl) – N – propyl] – amino – 8 – methoxy – 1,2,3,4 – tetrahydronaphthalin in 20 ml Methylenchlorid wurde bei 0 ° C eine Lösung von 0,50 g (4,4 mmol) Chlorameisensäureethylester in 15 ml Methylenchlorid zugetropft. Nach 15 h Rühren bei Raumtemperatur wurde filtiert und die organische Phase mit Wasser sowie gesättigter Kochsalzlösung gewaschen. Trocknen (Magnesiumsulfat) und Einengen lieferten 0,8 g Rohprodukt. Chromatographie auf Kieselgel (Toluol – Essigester – Gradienten) lieferte 0,25 g (19%) der Titelverbindung.
$R_f$ (Toluol – Essigester 3:1): 0,56
MS : 335, 306, 232, 176, 161

Beispiel 68

2 – [N – (2 – Ethyl – carbonyldioxy – ethyl) – N – propyl]amino – 8 – methoxy – 1,2,3,4 – tetrahydronaphthalin Hydrochlorid

Mit etherischer Salzsäure läßt sich das Hydrochlorid der Verbindung aus Beispiel 67 als farblosen, amorphen Feststoff gewinnen.
$^{1}$H – NMR ($CD_3OD$): $\delta$ = 1,05 (t, 3H); 1,38 (t, 3H); 1,82 (m, 3H); 2,35 (m, 1H); 2,7 – 3,85 (m); 4,25 (q, 2H); 4,5 (m, 2H); 6,75 (m, 2H); 7,15 (m, 1H).

Beispiel 69

2 – [N – (2 – Cyanoethyl) – N – propyl]amino – 8 – methoxy – 1,2,3,4 – tetrahydronaphthalin

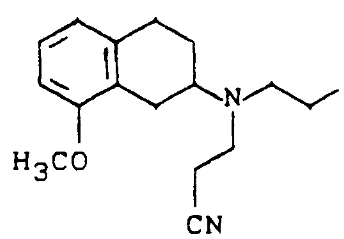

1,10 g (5.0 mmol) 8 – Methoxy – 2 – propylamino – 1,2,3,4 – tetrahydronaphthalin, 1,30 g (25 mmol) Acrylnitril und 20 mg Kupferacetat wurden 3,5 h bei 100 ° C gerührt. Flash – Chromatographie des Reaktionsansatzes auf Kieselgel (Toluol – Essigester – Gradient) ergab 1,10 g (81%) der Titelverbindung als gelbliches Öl.
$R_f$ (Toluol – Essigester 3:1): 0,62
MS : 272, 243, 232, 161
IR (Chloroform): 2249, 1587

Beispiel 70

2-[N-(2-Cyanoethyl)-N-propyl]amino-8-methoxy-1,2,3,4-tetrahydronaphthalin Hydrochlorid

Mit etherischer Salzsäure wurde aus der Verbindung des Beispiels 69 das Hydrochlorid als farbloser Feststoff gewonnen.
Schmelzbereich.: 60 - 67° C
Analog den Beispielen 1 bis 12 wurden hergestellt:

Beispiel 71

2-[3-(Indo-3-yl)propyl]amino-8-methoxy-1,2,3,4-tetrahydronaphthalin

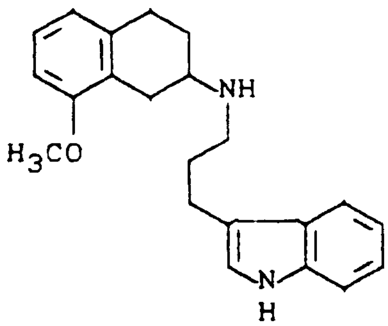

Schmelzpunkt: 154° C
Analog Beispiel 17 bis 24 wurden hergestellt:

Beispiel 72

2-{N-Propyl-N-[3-(1,2,3,4-tetrahydronaphthalin-1-yloxy)propyl]}amino-8-methoxy-1,2,3,4-tetrahydronaphthalin Hydrochlorid (Diastereomerengemisch)

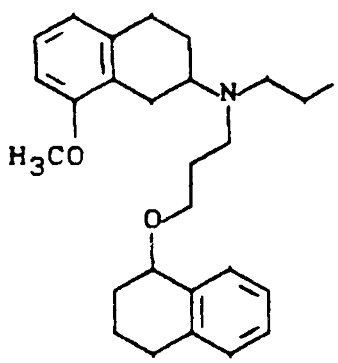

x HCl

$^1H$-NMR ($CD_3OD$): δ = 1,0 (m, 3H); 1,6 - 2,3 (m, 10 H); 2,6 - 3,9 (m); 4,5 (m, 1H); 6,7 (2d, 2H); 7,0 - 7,4 (m, 5H).

Beispiel 73

2-{N-[3-(Indol-3-yl)propyl]-N-propyl}amino-8-methoxy-1,2,3,4-tetrahydronaphthalin Dihydrochlorid

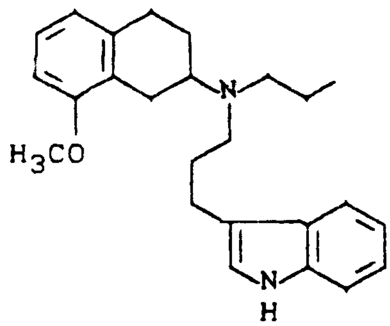

x 2 HCl

Schmelzbereich: 70 - 130° C (hygroskopisch)
$^1$H=NMR ($CD_3OD$): δ = 0,95 (t, 3H); 1,6 - 1,9 (m, 3H); 2,1 - 2,2 (m, 3H); 2,6 - 3,75 (m); 3,8 (s, 3H); 6,7 (2d, 2H); 6,9 - 7,1 (m); 7,35 (d, 1H); 7,55 (d, 1H).

Beispiel 74

2-{N-[2-(Indol-3-yl)ethyl]-N-propyl}amino-8-methoxy-1,2,3,4-tetrahydronaphthalin Dihydrochlorid

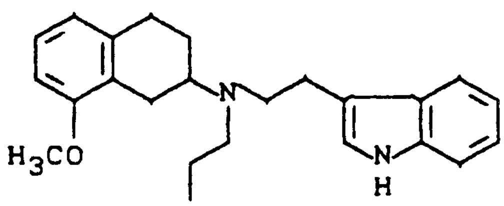

x 2 HCl

Schmelzpunkt: > 100° C
$^1$H-NMR ($CD_3OD$): δ = 1,05 (t, 3H); 1,7 - 2,0 (m, 3H); 2,3 (m, 1H); 2,7 - 3,9 (m, ); 6,6 - 6,8 (m, 2H); 7,0 (m, 3H); 7,25 (d, 1H); 7,4 (d, 1H); 7,6 (dd, 1H).

Beispiel 75

2-[N-(3-Propylthio-propyl)-N-propyl)amino-1,2,3,4-tetrahydronaphthalin Hydrochlorid

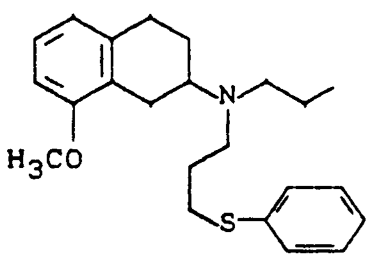

x HCl

$^1$HNMR ($CD_3OD$): δ = 1,0 (d, 3H); 1,6 - 1,9 (m, 3H); 2,1 (m, 2H); 2,25 (m, 1H); 2,1 - 3,5 (m); 3,65 (m, 1H); 3,8 (s, 3H); 6,7 (d, 1H); 6,75 (d, 1H); 7,15 (dd, 1H); 7,15 - 7,4 (m, 5H).

Analog Beispiel 25 bis 34 wurden hergestellt:

Beispiel 76

2 – [N – 4 – (4,4 – Dimethyl – 2,6 – dioxo – piperidin – 1 – yl)butyl – N – propyl]amino – 8 – methoxy – 1,2,3,4 – tetrahydronaphthalin Hydrochlorid

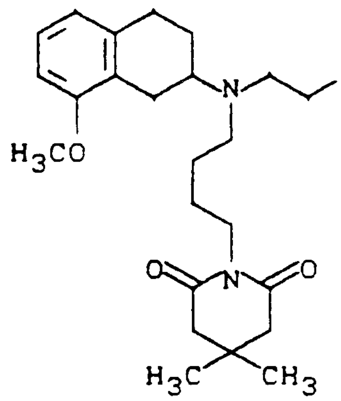

x HCl

$^1$H – NMR ($CD_3OD$): δ = 1,0 – 1,1 (m, 9H); 1,6 – 2,0 (m, 7H) 2,3 (m, 1H); 2,55 (s, 4H); 2,8 – 3,9 (m); 6,85 (2d, 2H), 7,1 (dd, 1H).

Beispiel 77

2 – {3 – [N – (8 – Methoxy – 1,2,3,4 – tetrahydronaphthalin – 2 – yl) – N – propyl – amino]propyl} – 1,2 – benzisothiazol – 3(2H) – on – 1,1 – dioxid Hydrochlorid

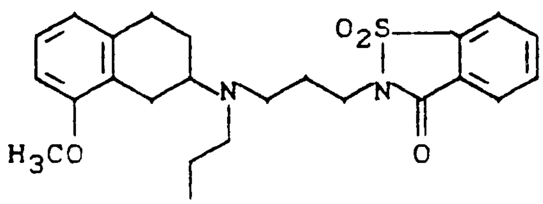

x HCl

$^1$H – NMR ($CDCl_3$): δ = 1,0 (t, 3H); 1,8 – 2,2 (m); 2,5 – 4,0 (m); 6,6 – 6,8 (m, 2H); 7,15 (dd, 1H); 7,8 – 8,2 (m, 4H) 12,3 (1H).

Beispiel 78

2 – {4 – [N – (8 – Methoxy – 1,2,3,4 – tetrahydronaphthalin – 2 – yl) – N – propyl – amino]but – 2 – enyl} – 1,2 – benzisothiazol – 3(2H) – on – 1,1 – dioxid (trans – Form)

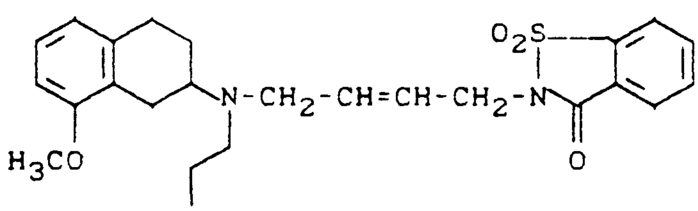

IR (Chloroform): 3008, 2935, 2840, 1732, 1587, 1470, 1440.

Beispiel 79

2-{4-[N-(8-Methoxy-1,2,3,4-tetrahydronaphthalin-2-yl)-N-propyl-amino]but-2-enyl}-1,2-benzisothiazol-3(2H)-on-1,1-dioxid Hydrochlorid (trans-Form)

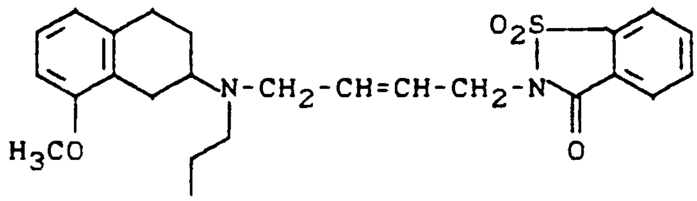

x HCl

Beispiel 80

2-{5-[N-(8-Methoxy-1,2,3,4-tetrahydronaphthalin-2-yl)-N-propyl-amino]pentyl}-1,2-benzisothiazol-3(2H)-on-1,1-dioxid Hydrochlorid

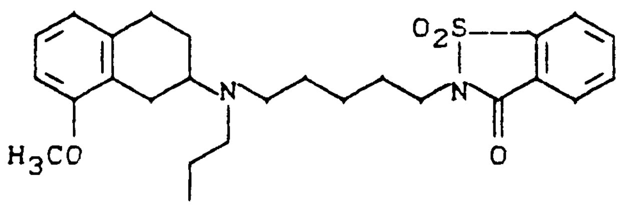

x HCl

$^1$H-NMR ($CD_3OD$): δ = 1,05 (t, 3H); 1,5 (m, 2H); 1,7 - 2,0 (m, 7H); 2,3 (m, 1H); 2,7 - 3,9 (m); 6,7 (2d, 2H); 7,15 (t, 1H); 7,9 - 8,1 (m, 4H).

Beispiel 81

2-{2-[N-(8-Methoxy-1,2,3,4-tetrahydronaphthalin-2-yl)-N-propyl-amino]propyl}-1,2-benzisothiazol-3(2H)-on-1,1-dioxid Hydrochlorid

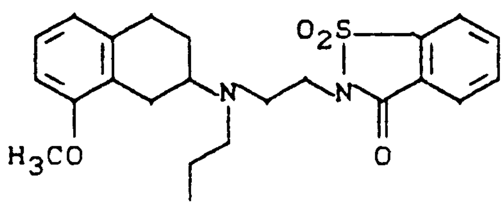

x HCl

Schmelzbereich: 90 - 110 °C (hygroskopisch)
$^1$H-NMR ($CD_3OD$): δ = 1,05 (t, 3H); 1,7 - 2,0 (m, 3H); 2,3 (m, 1H); 2,7 - 4,1 (m); 3,8 (s, 3H); 4,3 (m, 2H); 6,75 (2d, 2H); 7,15 (dd, 1H); 7,9 - 8,2 (m, 4H).

Beispiel 82

2 – [N – (2 – Cyano – benzyl) – N – propyl]amino – 1,2,3,4 – tetrahydronaphthalin

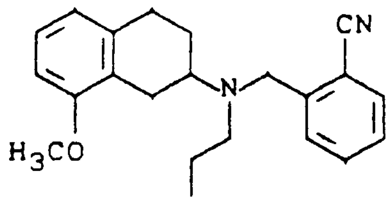

IR (Chloroform): 3010, 2965, 2938, 2227, 1603, 1588, 1471 1441.

Beispiel 83

2 – [N – (3 – Cyano – benzyl) – N – propyl]amino – 1,2,3,4 – tetrahydronaphthalin

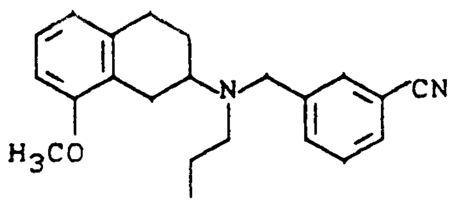

IR (Chloroform): 3008, 2964, 2938, 2842, 2234, 1604, 1588, 1471, 1442.

Beispiel 84

2 – [N – (4 – Cyano – benzyl) – N – propyl]amino – 1,2,3,4 – tetrahydronaphthalin

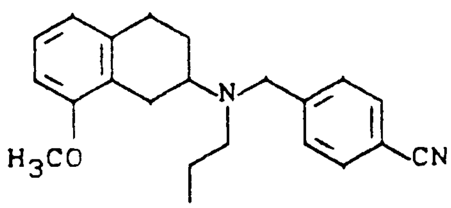

IR (Chloroform) 3010, 2934, 2876, 2230, 1610, 1588, 1471, 1441.

Analog Beispiel 45 bis 54 wurden hergestellt:

Beispiel 85

2-{N-[3-(4-Methylphenylsulfonamido)propyl]-N-propyl}amino-8-methoxy-1,2,3,4-tetrahydronaphthalin Hydrochlorid

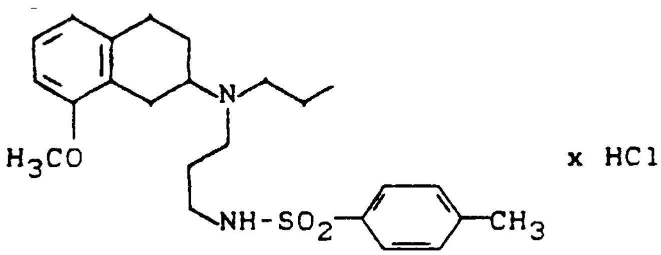

Schmelzbereich: 80 - 90 °C
$^1$H-NMR ($CD_3OD$): δ = 1,05 (t, 3H); 1,7 - 2,0 (m, 5H); 2,25 (m, 1H); 2,4 (s, 3H); 2,6 -3,8 (m); 3,85 (s, 3H); 6,75 (2d, 2H); 7,15 (dd, 1H); 7,45 (d, 2H); 7,75 (d, 2H).

Beispiel 86

2-{N-[3-(3-Ethoxycarbonyl-propylsulfonamido)propyl]-N-propyl}amino-8-methoxyl-1,2,3,4-tetrahydronaphthalin Hydrochlorid

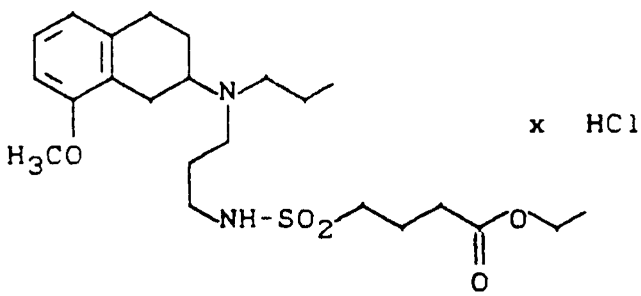

Schmelzpunkt: > 50 °C
$^1$H-NMR ($CD_3OD$): δ = 1,0 (2t, 3H); 1,25 (t, 3H); 1,8 - 2,2 (m, 7H); 2,35 (m, 1H), 2,5 (m, 2H); 2,7 -3,9 (m); 3,85 (s, 3H); 4,1 (q, 2H); 6,75 (2d, 2H); 7,15 (dd, 1H).

Beispiel 87

2-{N-[2-[N',N'-Bis(4-methylphenylsulfonyl)amino]ethyl]-N-propyl}amino-1,2,3,4-tetrahydronaphthalin Hydrochlorid

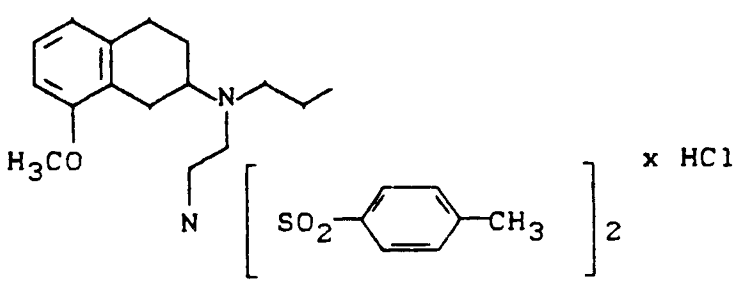

Schmelzbereich: 105 - 110 °C

Beispiel 88

2 – [N – 3 – Benzyloxycarbonylamido)propyl – N – Propyl]amino – 1,2,3,4 – tetrahydronaphthalin Hydrochlorid

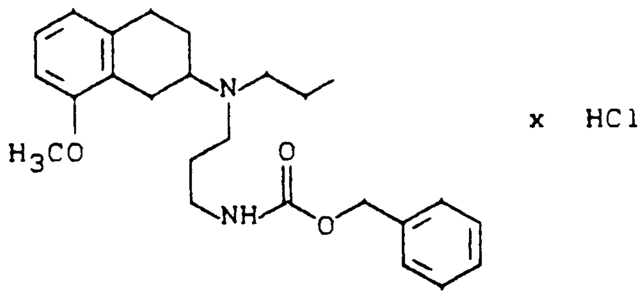

$^1$H – NMR ($CD_3OD$): δ = 1,05 (t, 3H); 1,7 – 2,1 (m, 5H); 2,25 (m, 1H), 2,6 – 3,8 (m); 3,85 (s, 3H); 5,1 (ws, 2H); 6,75 (2d, 2H); 7,15 (t, 1H); 7,2 – 7,4 (m, 5H).

Beispiel 89

2 – {4 – [N – (8 – Methoxy – 1,2,3,4 – tetrahydro – 2 – naphthyl) – N – propyl – amino]but – 2 – inyl} – 1,2 – benzisothiazol – 3(2H) – on – 1,1 – dioxid

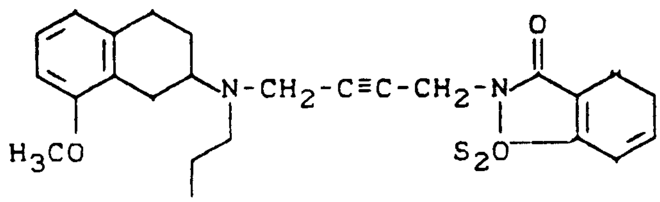

Bei 50 ° C wurden zu 2,19 g (10 mmol) 8 – Methoxy – 2 – propylamino – 1,2,3,4 – tetrahydronaphthalin, 0,36 g (12 mmol) Paraformaldehyd und 0,1 g Kupfer(II)acetat – monohydrat in 10 ml Dioxan 2,21 g Propargylsac – charin in 20 ml Dioxan zugetropft. Nach 5 h bei 80 ° C wurde vom Lösungsmittel befreit. Das verbliebene Rohprodukt wurde durch Flash – Chromatographie auf Kieselgel mit Toluol – Essigester – Gradienten gerei – nigt. Man erhielt 3,4 g (75%) der Titelverbindung als erstarrendes Öl.
Schmelzpunkt: 80 – 84 ° C (aus Ethanol)
$R_f$ (Toluol – Ethanol 1:1): 0,51
IR (Chloroform) 3008, 2967, 2949, 2839, 1740, 1589, 1470, 1440

Beispiel 90

2 – {4 – [N – (8 – Methoxy – 1,2,3,4 – tetrahydro – 2 – naphthyl) – N – propyl – amino]but – 2 – inyl} – 1,2 – benzisothiazol – 3(2H) – on – 1,1 – dioxid Hydrochlorid

Durch Behandeln der Verbindung des Beispiels 89 mit etherischer Salzsäure erhielt man 2,0 g des Hydrochlorids als farblose Kristalle .
Schmelzpunkt: 195 – 200 ° C

Beispiel 91

8 – Methoxy – 2 – {N – propyl – N – [3 – (2 – isoindolinyl}propyl]}amino – 1,2,3,4 – tetrahydronaphthalin

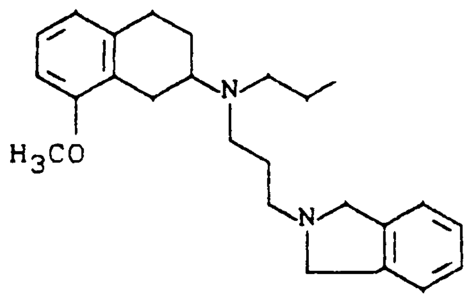

Bei 0 ° C wurden zur Suspension von 0,88 g (23 mmol) Lithiumaluminiumhydrid in 35 ml Tetrahydrofuran 2,35 g 8 – Methoxy – 2 – [N – propyl – N – (3 – phthalimidoyl – propyl)]amino – 1,2,3,4 – tetrahydronaphthalin in 15 ml Tetrahydrofuran zugetropft. Nach 3 h bei Rückfluß wurde mit 10 ml Tetrahydrofuran – Wasser – Gemisch 2:1 vorsichtig hydrolysiert. Anschließend wurden 2 ml verdünnte Natronlauge zugegeben. Nach Trocknen über Kaliumcarbonat und Filtration wurde eingeengt. Das derart erhaltene Rohprodukt wird durch Flash – Chromatographie auf Kieselgel mit Toluol – Essigester – Gradienten in Gegenwart von 2% Triethylamin gereinigt. Man erhielt 1,0 g (46%) der Titelverbindung als Öl.
$R_f$(Toluol – Essigester 1:1): ~ 0,1
IR (Chloroform) 3010, 2938, 2840, 1585, 1470, 1439

Beispiel 92

8 – Methoxy – 2 – {N – propyl – N – [3 – (2 – isoindolinyl) – propyl]}amino – 1,2,3,4 – tetrahydronaphthalin Dihydrochlorid

Aus der Verbindung des Beispiels 91 ließ sich mit etherischer Salzsäure das Dihydrochlorid in Form hellgrauer Kristalle gewinnen.
Schmelzpunkt: > 75 ° C (Sintern, Zers.)

Beispiel 93

2 – [N – (2 – Methoxyethyl} – N – propyl]amino – 8 – methoxy – 1,2,3,4 – tetrahydronaphthalin

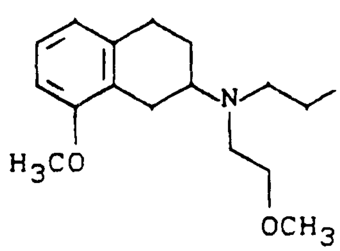

Die Lösung von 2,0 g (6,9 mmol) 2 – (N – Methoxyacetyl – N – propyl)amino – 8 – methoxy – 1,2,3,4 – tetrahydronaphthalin in 10 ml Tetrahydrofuran wurde zur siedenden Suspension von 0,53 g (8,3 mmol) Lithiumaluminiumhydrid in 20 ml Tetrahydrofuran zugetropft. Nach 2 h bei Siedetemperatur wurde die Reaktionsmischung nacheinander mit 0,5 ml Wasser, 0,3 ml 20%iger Natronlauge und 1,8 ml Wasser versetzt. Die organische Phase wurde mit Kaliumcarbonat getrocknet und eingeengt. Man erhielt auf diese Weise 1,5 g (79%) der Titelverbindung als Öl.
$R_f$ (Toluol – Essigester 1:1): 0,36
MS: 277, 248, 232, 205, 161
IR (Chloroform): 3009, 2963, 2934, 1588, 1471, 1441

Beispiel 94

2 – [N – (2 – Methoxyethyl) – N – propyl]amino – 8 – methoxy – 1,2,3,4 – tetrahydronaphthalin Hydrochlorid

Mit etherischer Salzsäure gewann man aus 1,5 g der Verbindung aus Beispiel 93 das Hydrochlorid als amorphen hygroskopischen Feststoff (1,6 g) gewinnen.
$^1H$ – NMR ($CD_3OD$): $\delta$ = 1,05 (t, 3H); 1,8 (m, 3H); 2,3 (m, 1H); 2,75 (m, 1H); 2,95 (m, 2H); 3,2 – 3,9 (m, ); darunter 3,42 (s, 3H); 3,85 (s, 3H); 6,75 (dd, 2H); 7,15 (dd, 1H).

Beispiel 95

8 – Methoxy – 2 – [N – (3 – phenylsulfinyl – propyl) – N – propyl]amino – 1,2,3,4 – tetrahydronaphthalin

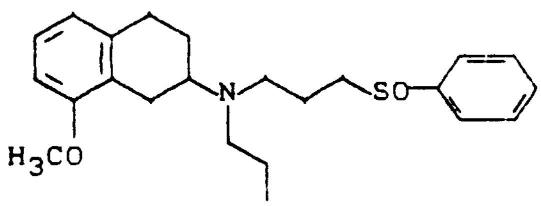

Die Lösung von 3,7 g (10 mmol) 8 – Methoxy – 2 – [N – (3 – phenylthio – propyl) – N – propyl]amino – 1,2,3,4 – tetrahydronaphthalin in 50 ml Eisessig wurde bei 0 ° C mit 1,2 g 30%igem Wasserstoffperoxid versetzt. Innerhalb 1 h wurde die Reaktionsmischung auf Raumtemperatur erwärmt. Dann wurde mit 0,3 g 30%igem Wasserstoffperoxid versetzt und weitere 2 h bei Raumtemperatur gerührt. Das Reaktionsgemisch wurde auf 10%ige Kaliumcarbonatlösung und Essigester gegossen. Die Essigesterphase wurde abgetrennt und die wäßrige Phase mehrmals mit Essigester extrahiert. Trocknen der organischen Phase und Einengen lieferten ein Rohprodukt, das durch Flash – Chromatographie auf Kieselgel mit Toluol – Essigester – Gradienten gereinigt wurde. Man erhielt 2,35 g (61%) der Titelverbindung als 1:1 Diastereomerengemisch (farbloses Öl).
MS: 385, 368, 340, 232, 161
$^{13}C$ – NMR ($CDCl_3$): $\delta$ = 11,8 (q), 21,1 (t), 21,9 (t), 22,0 (t), 24,9 (t), 25,0 (t), 25,8 (t), 25,9 (t), 30,0 (t), 48,2 (t), 48,6 (t), 52,1 (t), 52,1 (t), 54,8 (t), 54,9 (t), 55,1 (q), 56,1 (d), 106,7 (d), 120,7 (d), 123,9 (d), 124,9 (s), 125,9 (d), 129,0 (d), 130,7 (d), 137,6 (s), 143,9 (s), 157,4 (s).

Beispiel 96

8 – Methoxy – 2 – [N – (3 – phenylsulfinyl – propyl) – N – propyl]amino – 1,2,3,4 – tetrahydronaphthalin Hydro – chlorid

Aus der Verbindung des Beispiels 95 ließ sich mit etherischer Salzsäure das Hydrochlorid in Form eines farblosen hygroskopischen Feststoffs gewinnen.
$^1H$ – NMR ($CD_3OD$): $\delta$ = 1,05 (m, 3H); 1,8 (m, 3H); 2,2 (m, 3H); 2,6 – 3,9 (m) darunter 3,85 (2s), insgesammt 3H; 6,85 (2d, 2H); 7,15 (dd, 1H); 7,5 – 7,8 (m, 5H).

Beispiel 97

8 – Methoxy – 2 – [N – (3 – phenylsulfonylpropyl) – N – propyl]amino – 1,2,3,4 – tetrahydronaphthalin

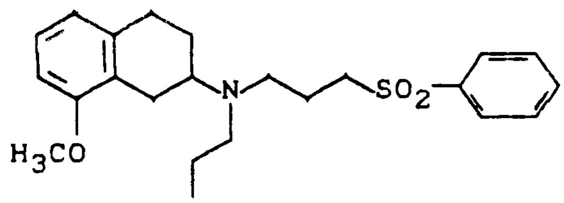

Die Lösung von 3,7 g (10 mmol) 2 – [N – (3 – Phenylthiopropyl) – N – propyl]amino – 8 – methoxy – 1,2,3,4 – tetrahydronaphthalin und 2,6 g 30%iges Wasserstoffperoxid in 50 ml Eisessig wurde 1 h bei 90 ° C erhitzt. Dann wurden 0,4 g 30%iges Wasserstoffperoxid zugegeben. Nach eine weiteren Stunde bei 90 ° C wurden 0,4 g 30%iges Wasserstoffperoxid zugegeben. Nach weiteren 2 h bei 90 ° C wurde abgekühlt. Das Reaktionsgemisch wurde auf ca. 10%ige Kaliumcarbonatlösung und Essigester gegossen. Die Essigesterphase wurde abgetrennt, die wäßrige Phase extrahiert. Trocknen und Einengen ergaben ein Rohprodukt, welches durch Flash – Chromatographie auf Kieselgel mit Toluol – Essigester – Gradienten gereinigt wurde. Auf diese Weise erhielt man 1,65 g (41%) der Titelverbindung als zähen Sirup.
$R_f$ (Toluol – Essigester 3:1): 0,23
MS: 401, 372, 232, 161

Beispiel 98

8 – Methoxy – 2 – [N – (3 – phenylsulfonylpropyl) – N – propyl]amino – 1,2,3,4 – tetrahydronaphthalin Hydrochlorid

Aus der Verbindung des Beispiels 97 ließ sich mit etherischer Salzsäure das Hydrochlorid in Form eines farblosen sehr hygroskopischen Feststoffs gewinnen.
$^1$H – NMR ($CD_3OD$): $\delta$ = 1,05 (t, 3H); 1,8 – 2,0 (m, 3H); 2,1 – 2,4 (m, 3H); 2,7 – 3,9 (m), darunter 3,85 (s, 3H); 6,75 (2d, 2H); 7,15 (t, 1H); 7,6 – 7,8 (m, 3H); 8,0 (d, 2H).

Beispiel 99

2 – [N – 3 – (4,4 – Dimethyl – 2,6 – dioxo – piperidin – 1 – yl)propyl – N – propyl]amino – 8 – methoxy – 1,2,3,4 – tetrahydronaphthalin

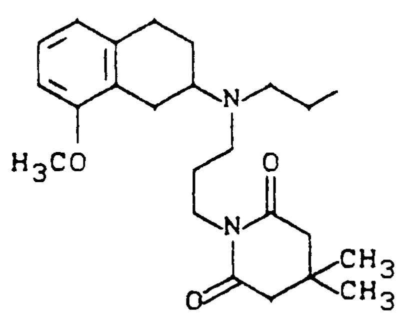

2,2 g (8 mmol) 2 – [N – (3 – Aminopropyl) – N – propyl]amino – 8 – methoxy – 1,2,3,4 – tetrahydronaphthalin, 1,1 g (8 mmol) 3,3 – Dimethylglutarsäureanhydrid, 1 Tropfen Tributylamin, 0,5 g Molsieb 3 Å wurden in 5 ml Toluol 2 h zum Rückfluß erhitzt. Das nach Filtration und Einengen erhaltene Rohprodukt wurde durch Flash – Chromatographie auf Kieselgel mit Toluol – Essigester – dann Toluol – Ethanol – Gradienten gereinigt. Man erhielt so 1,6 g (50%) der Titelverbindung als zähen Sirup.
$R_f$ (Toluol – Methanol 4:1): 0,3
MS 400, 371, 232, 182, 161

IR (Chloroform) 3007, 2965, 2875, 2841, 1727, 1670, 1602, 1588, 1471, 1441

Beispiel 100

2-[N-3-(4,4-Dimethyl-2,6-dioxo-piperidin-1-yl)propyl-N-propyl]amino-8-methoxy-1,2,3,4-tetrahydronaphthalin Hydrochlorid

Mit etherischer Salzsäure ließ sich das Hydrochlorid aus Beispiel 99 gewinnen.
Analog den Beispielen 98 und 99 wurden hergestellt:

Beispiel 101

2-[N-2-(4,4-Dimethyl-2,6-dioxo-piperidin-1-yl)ethyl-N-propyl]amino-8-methoxy-1,2,3,4-tetrahydronaphthalin

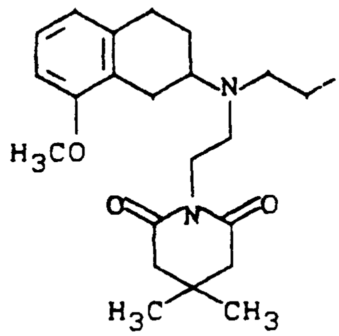

Ausbeute: 2,3 g (75%) der Titelverbindung als zähen Sirup $R_f$ (Toluol-Methanol 4:1): 0,49
IR (Chloroform) 3009, 2966, 2933, 2877, 1726, 1675, 1603, 1587, 1470, 1441

Beispiel 102

2-[N-2-(4,4-Dimethyl-2,6-dioxo-piperidin-1-yl)ethyl-N-propyl]amino-8-methoxy-1,2,3,4-tetrahydronaphthalin Hydrochlorid

Mit etherischer Salzsäure ließ sich aus der Verbindung des Beispiels 101 das Hydrochlorid gewinnen.
Analog zu den Beispielen 1 - 12 wurden hergestellt:

Beispiel 103

8-(8-Methoxy-1,2,3,4-tetrahydro-2-naphthalinyl)-1-phenyl-1,3,8-triazabicyclo[4,5]decan-4-on

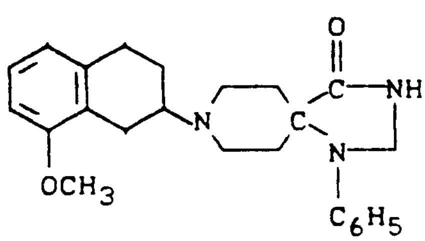

Schmp. (aus Toluol/Petrolether): 210 - 220 °C
IR (KBr): 3424, 2926, 2839, 1703, 1600, 1502, 1470

Beispiel 104

2 – {N – [3 – (1,2,3,4 – Tetrahydronaphthalin – 1 – yloxy) – propyl]}amino – 8 – methoxy – 1,2,3,4 – tetrahydronaphthalin (Diastereomerengemisch)

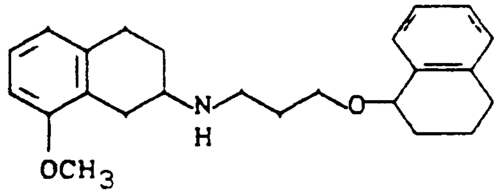

Aus 8 – Methoxy – 2 – tetralon und 1 – (3 – Aminopropyloxy) – 1,2,3,4 – tetrahydronaphthalin (zugänglich aus 1 – Hydroxy – 1,2,3,4 – tetrahydronaphthalin und Acrylnitril mit anschließender Reduktion).
$R_f$ (Acetonitril: Triethylamin 30:1): 0.44

Beispiel 105

8 – Methoxy – 2 – {3 – [3 – (N – 4 – methylphenylsulfonyl)indolyl]propyl}amino – 1,2,3,4 – tetrahydronaphthalin

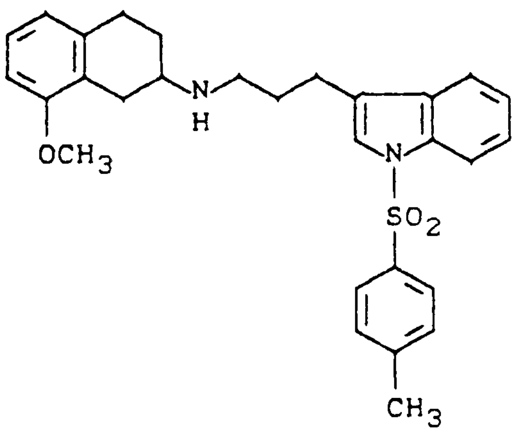

Aus 8 – Methoxy – 2 – tetralon und 3 – (3 – Aminopropyl) – 1 – tosylindol
$R_f$ (Chloroform:Methanol 2:1): 0,5
Analog zu den Beispielen 17 – 24 wurden hergestellt:

Beispiel 106

8 – Methoxy – 2 – {N – propyl – N – {3 – [3 – (N – 4 – methylphenylsulfonyl)indolyl]propyl}}amino – 1,2,3,4 – tetrahydronaphthalin

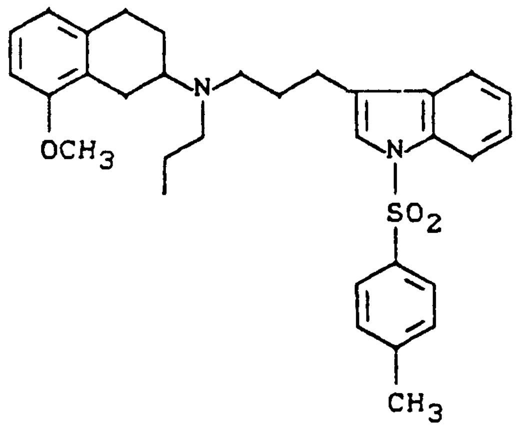

$R_f$ (Toluol:Essigester 1:1): 0,34
IR (Chloroform): 3006, 2936, 2839, 1599, 1586, 1495

Beispiel 107

8 – Methoxy – 2 – {N – propyl – N – {3 – [3 – (N – 4 – methylphenylsulfonyl)indolyl]propyl}}amino – 1,2,3,4 – tetrahydronaphthalin Hydrochlorid

Schmp.: 55 – 90 ° C (unter Zersetzung)
$^1$H – NMR ($CD_3OD$): δ = 0,9 – 1,0 (m, 3H); 1,6 – 1,9 (m, 4H); 2,1 – 2,2 (m, 3H); 2,3 (s, 3H); 2,6 – 3,3 (m); 3,6 – 3,8 (m, 1H); 3,8 (s, 3H); 6,7 (d, 1H); 6,75 (d, 1H); 7,15 (dd, 1H); 7,25 (m, 3H); 7,35 (dd, 1H); 7,55 (m, 2H); 7,8 (m, 2H); 8,0 (d, 1H).
Analog zu den Beispielen 25 – 34 wurden hergestellt:

Beispiel 108

2 – {3 – [6 – (1,2,3 – Benzothiadiazolyl)oxy]ethyl – N – propyl}amino – 8 – methoxy – 1,2,3,4 – tetrahydronaphthalin

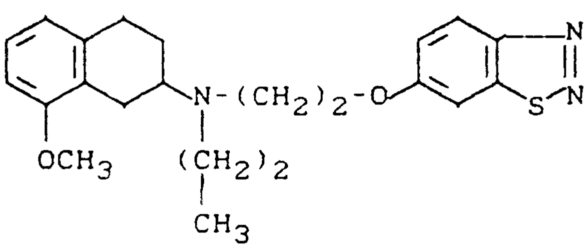

Aus 2 – Propylamino – 8 – methoxy – 1,2,3,4 – tetrahydronaphthalin und 6 – (2 – Chlorethyloxy) – 1,2,3 – benzothiadiazol.
$R_f$ (Toluol:Essigester 3:1): 0,46
MS: 397, 368, 232, 161
Analog zu den Beispielen 45 – 54 wurden hergestellt:

Beispiel 109

2 – {N – [3 – (4 – Fluorphenylsulfonamido)ethyl] – N – propyl}amino – 8 – methoxy – 1,2,3,4 – tetrahydronaphthalin Hydrochlorid

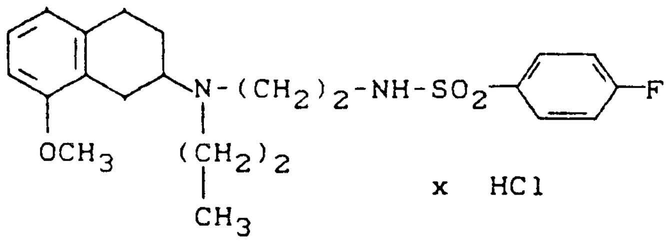

Schmelzbereich: 95 – 110 ° C
$^1$H – NMR ($CD_3OD$): $\delta$ = 1,05 (t, 3H); 1,7 – 2,0 (m, 2H); 2,35 (m, 1H); 2,75 (dd, 1H); 2,95 (m, 2H); 3,2 – 3,6 (m); 3,85 (s, 3H); 6,75 (d, 1H); 6,8 (d, 1H); 7,15 (dd, 1H); 7,35 (m, 2H); 7,95 (m, 2H) ppm.

Beispiel 110

2 – {N – [3 – (4 – Methylphenylsulfonylamido)methyl]phenylmethyl – N – propyl} – 8 – methoxy – 1,2,3,4 – tetrahydronaphthalin

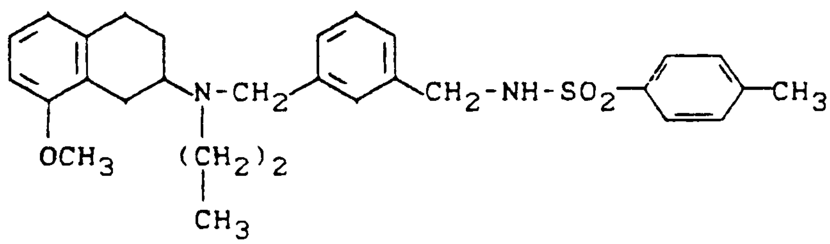

$R_f$ (Toluol:Essigester 3:1): 0,42

Beispiel 111

2 – {N – [3 – (4 – Methylphenylsulfonylamido)methyl]phenylmethyl – N – propyl} – 8 – methoxy – 1,2,3,4 – tetrahydronaphthalin Hydrochlorid

Schmp.: 115 ° C

Beispiel 112

2 – {N – [2 – (4 – Methylphenylsulfonylamido)methyl]phenylmethyl – N – propyl} – 8 – methoxy – 1,2,3,4 – tetrahydronaphthalin

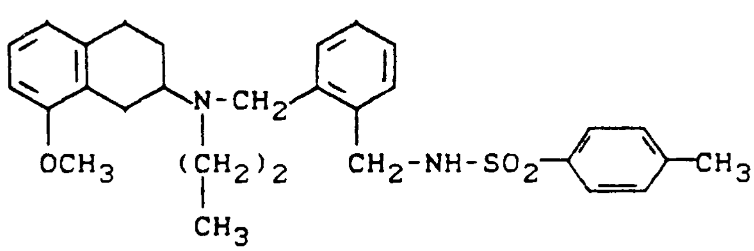

$R_f$ (Toluol:Essigester 3:1): 0,73

Beispiel 113

2 – {N – [2 – (4 – Methylphenylsulfonylamido)methyl]phenylmethyl – N – propyl} – 8 – methoxy – 1,2,3,4 – tetrahydronaphthalin Hydrochlorid

Schmp.: 120 ° C

Beispiel 114

2 – {N – [4 – (4 – Methylphenylsulfonylamido)methyl]phenylmethyl – N – propyl} – 8 – methoxy – 1,2,3,4 – tetrahydronaphthalin

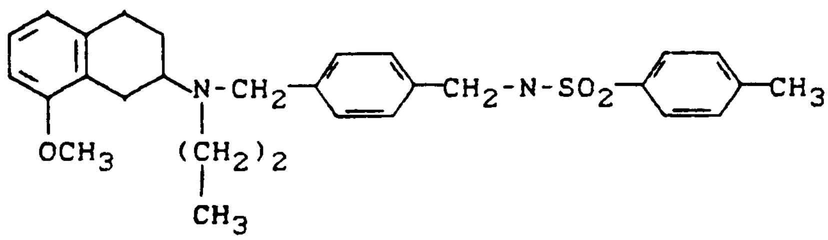

$R_f$ (Toluol:Essigester 3:1): 0,37

Beispiel 115

2 – {N – [4 – (4 – Methylphenylsulfonylamido)methyl]phenylmethyl – N – propyl} – 8 – methoxy – 1,2,3,4 – tetrahydronaphthalin Hydrochlorid

Schmp.: 185 ° C
Analog zu den Beispielen 99 – 102 wurden hergestellt:

Beispiel 116

2 – [N – 2 – (3 – Phenyl – 2,6 – dioxo – piperidin – 1 – yl)ethyl – N – propyl]amino – 8 – methoxy – 1,2,3,4 – tetrahydronaphthalin

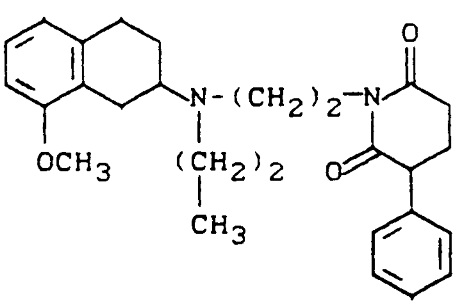

Aus Verbindung von Beispiel 36 und 2 – Phenylglutarsäureanhydrid.
Ausbeute: 77% zäher Sirup
$R_f$ (Methylenchlorid:Methanol 10:1): 0,9
IR (Chloroform): 3010, 2963, 2841, 1727, 1676, 1602, 1587

Beispiel 117

2 – [N – 2 – (3 – Phenyl – 2,6 – dioxo – piperidin – 1 – yl)ethyl – N – propyl]amino – 8 – methoxy – 1,2,3,4 – tetrahydronaphthalin Hydrochlorid

Schmelzbereich: 110 – 115 ° C

Beispiel 118

2 – [N – (2 – Ethoxycarbonylamido – propyl) – N – propyl]amino – 8 – methoxy – 1,2,3,4 – tetrahydronaphthalin

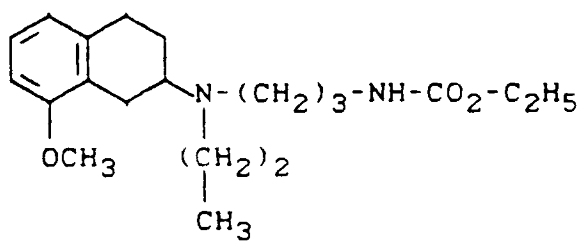

Bei 0 ° C wurde zu einer Lösung von 550 mg (2,0 mmol) Verbindung aus Beispiel 35 und 1,40 g (20 mmol) gepulvertem Kaliumcarbonat in 10 ml Diisopropylether eine Lösung von 220 mg (2,0 mmol) Chlorameisensäureethylester in 2 ml Diisopropylether getropft. Nach 2 Stunden wurde auf Raumtemperatur gebracht und 3 Tage gerührt. Das Reaktionsgemisch wurde auf 50 ml Essigester/Eiswasser – Gemisch gegossen. Die wäßrige Phase wurde mehrmals mit Essigester extrahiert. Trocknen (Magnesiumsulfat) und Einengen der vereinigten organischen Extrakte lieferte ein Rohprodukt, welches durch Chromatographie auf Kieselgel (Toluol/Essigester bzw. Toluol/Ethanol – Gradienten) gereinigt wurde. Dies ergab 380 mg (55%) der Titelverbindung als zähes Öl.
MS: 348, 319, 232, 161
IR (Chloroform): 3008, 2937, 2855, 1704, 1588, 1512, 1471

Beispiel 119

2 – [N – (2 – Ethoxycarbonylamido – propyl) – N – propyl]amino – 8 – methoxy – 1,2,3,4 – tetrahydronaphthalin Hydrochlorid

In etherischer Lösung läßt sich aus der Verbindung von Beispiel 118 mit etherischer Salzsäure das Hydrochlorid als hygroskopischer Schaum gewinnen.
$^1$H – NMR ($CD_3OD$): $\delta$ = 1,05 (t, 3H); 1,25 (t, 3H); 1,7 – 2,1 (m, 5H); 2,3 (m, 1H); 2,6 – 3,5 (m); 3,7 – 3,9 (m, 4H), darunter 3,85 (s, 3H); 4,10 (q, 2H); 6,75 (m, 2H); 7,15 (dd, 1H) ppm.

Beispiel 120

2 – [N – (1 – Adamantylcarbonamido – ethyl) – N – propyl]amino – 8 – methoxy – 1,2,3,4 – tetrahydronaphthalin

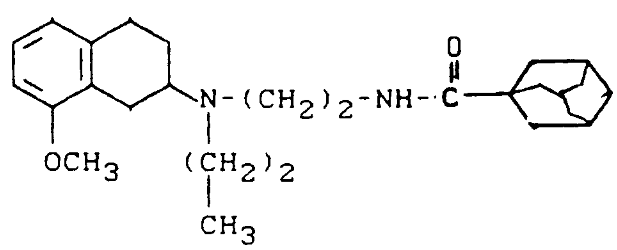

Bei 0 ° C wurden zur Lösung von 1,05 g (4,0 mmol) Verbindung von Beispiel 36 in 10 ml Toluol die Lösung von 0,80 g (4,0 mmol) 1 – Adamantancarbonsäurechlorid in 5 ml Toluol zugegeben. Es wurde auf Raum –

temperatur kommen gelassen und 1 Tag gerührt. Die Lösung wurde mit Natriumhydrogencarbonat versetzt und extrahiert. Trocknen der organischen Phase (Magnesiumsulfat) und Einengen ergaben 1,80 g Rohprodukt. Hieraus wurde durch Flash-Chromatographie auf Kieselgel (Toluol/Essigester bzw. Toluol/Ethanol-Gradienten) 1,50 g der Titelverbindung (88%) als Sirup gewonnen.
IR (Chloroform): 3407, 3005, 2908, 2856, 1638, 1588, 1509, 1471
$R_f$ (Toluol:Ethanol 3:1): 0,65

Beispiel 121

2-[N-(1-Adamantylcarbonamido-ethyl)-N-propyl]amino-8-methoxy-1,2,3,4-tetrahydronaphthalin Hydrochlorid

In etherischer Lösung wurde hieraus mit etherischer Salzsäure das Hydrochlorid als amorpher Feststoff gewonnen.
$^1$H-NMR ($CD_3OD$): $\delta$ = 1,1 (t, 3H); 1,6 - 2,1 (m), 2,3 (m, 1H); 2,7 (m, 1H); 2,95 (m, 2H); 3,1 - 3,9 (m); 6,7 (d, 1H); 6,75 (d, 1H); 7,15 (dd, 1H) ppm.

Beispiel 122

2-[N-(1-Adamantylcarbonamido-propyl)-N-propyl]amino-8-methoxy-1,2,3,4-tetrahydronaphthalin

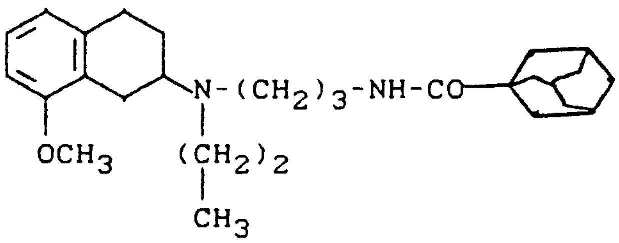

Analog Beispiel 120 mit der Verbindung aus Beispiel 35.
$R_f$ (Toluol:Ethanol 3:1): 0,53
IR (Methylenchlorid): 3467, 3318, 3004, 2910, 2856, 1630, 1588, 1510, 1470.

Beispiel 123

2-[N-(1-Adamantylcarbonamido-propyl)-N-propyl]amino-8-methoxy-1,2,3,4-tetrahydronaphthalin Hydrochlorid
$^1$H-NMR ($CD_3OD$): $\delta$ = 1,05 (t, 3H); 1,55 - 2,1 (m); 2,35 (m, 1H); 2,30 (m, 1H); 2,95 (m, 2H); 3,1 - 3,4 (m); 3,75 (m, 1H); 3,85 (s, 3H); 6,70 (d, 1H); 6,75 (d, 1H); 7,15 (t, 1H) ppm.

Beispiel 124

8 – Methoxy – 2 – [N – propyl – N – (2 – triphenylmethylamino – ethyl)]amino – 1,2,3,4 – tetrahydronaphthalin

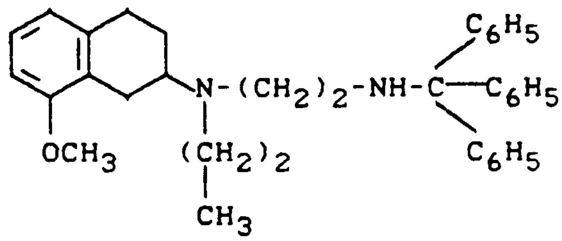

Bei 0 ° C wurde die Lösung von 1,30 g (5,0 mol) Verbindung aus Beispiel 36 und 0,75 g (7,5 mmol) Triethylamin in 30 ml Methylenchlorid mit 1,70 g (6,0 mmol) Tritylchlorid versetzt. Anschließend wurde 15 Stunden bei Raumtemperatur zur Reaktion gebracht. Das Reaktionsgemisch wurde mit Wasser versetzt und gut extrahiert. Die organische Phase wurde getocknet (Magnesiumsulfat) und eingeengt. Das derart erhaltene Rohprodukt konnte durch Chromatographie auf Kieselgel (Toluol/Essigester – Gradienten) gereinigt werden. Auf diese Weise erhielt man 2,05 g (81%) der Titelverbindung als Sirup.
$R_f$ (Toluol/Essigester 3:1): 0,67
IR (Chloroform): 3586, 3306, 3062, 3006, 2961, 2839, 1586, 1489, 1470

Beispiel 125

8 – Methoxy – 2 – [N – propyl – N – (2 – triphenylmethylamino – ethyl)]amino – 1,2,3,4 – tetrahydronaphthalin Dihydrochlorid

Aus der etherischen Lösung mit etherischer Salzsäure gewonnen.
Schmp.: 140 – 150 ° C

| Analyse ($C_{35}H_{40}N_2O$ x 2HCl x $H_2O$): | | | | | |
|---|---|---|---|---|---|
| Ber.:<br>Gef.: | C 70,6<br>C 70,8 | H 7,4<br>H 7,7 | N 4,7<br>N 4,7 | O 5,4<br>O 5,0 | Cl 11,9<br>Cl 12,2 |

Beispiel 126

8 – Methoxy – 2 – [N – propyl – N – (2 – triphenylmethyl)amino – propyl)]amino – 1,2,3,4 – tetrahydronaphthalin

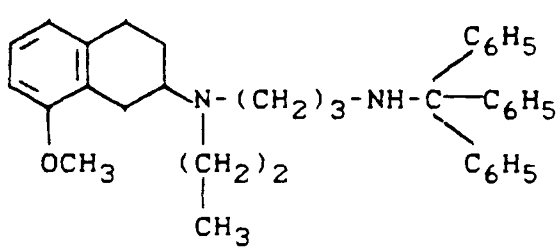

Wie in Beispiel 124 mit der Verbindung aus Beispiel 35.
$R_f$ (Toluol:Essigester 3:1) : 0,40
IR (Chloroform): 3584, 3061, 3008, 2961, 2839, 1587, 1489, 1470.

Beispiel 127

8 – Methoxy – 2 – [N – propyl – N – (2 – triphenylmethyl)amino – propyl)]amino – 1,2,3,4 – tetrahydronaphthalin Dihydrochlorid

Aus der Verbindung des Beispiels 126 wurde mit etherischer Salzsäure das Produkt in Form farbloser Kristalle gefällt.
Schmelzbereich: 130 ° C – 150 ° C

## Anwendungsbeispiele

Beispiel 128

A) Affinität zum 5 – $HT_1$ – Rezeptor
In Tabelle 1 wird beispielhaft die hohe Affinität der erfindungsgemäßen Verbindungen zu 5 – Hydroxytryptamin – Rezeptoren vom Subtyp 1 dargestellt. Bei den angegebenen Werten handelt es sich um Daten, die aus Rezeptorbindungsstudien mit Kalbs – Hippocampus – Membranpräparationen ermittelt wurden. Als radioaktiv markierter Ligand wurde hierzu $^3H$ – Serotonin verwendet.

Tabelle 1

| Verbindung des Beispiels – Nr. | Ki (nmol/l) |
|---|---|
| 20 | 11 |
| 22 | 3 |
| 26 | 2 |
| 34 | 1 |
| 42 | 4 |
| 45 | 1 |
| 58 | 4 |
| 62 | 4 |

B.) Untersuchungen auf Serotonin – agonistische / – antagonistische Wirkung.
Hierzu wird die Wirkung auf die Serotonin – vermittelte Kontraktion der arteria basilaris des Hundes untersucht [vgl. Peroutka et al., Brain Research 259, 327 (1983)].

Tabelle 2

| Verbindung des Beispiels-Nr. | Effekt agonist. | antagonist. |
|---|---|---|
| 2 | + | + |
| 22 | ++ | 0 |
| 34 | 0 | ++ |
| 45 | 0 | +++ |
| 66 | + | ++ |
| zum Vergleich aus EP-A1 41 488 | | |
| $R^1 = H$, $R^2 = nC_3H_7$, $R^3 = nC_3H_7$ | ++ | 0 |

In diesem Testmodell kann eine agonistische Wirkung durch die serotonin-mimetische Wirkung (Kontraktion) erkannt werden. Die agonistische Wirkkomponente zeigt sich durch die dosisabhängige Unterdrückung der Kontraktion bei der Gabe von Serotonin zu mit Testsubstanz vorbehandelten Präparationen.

Beispiel 129

Aufhebung des Abwehrverhaltens der Maus

In diesem Test (Tedeschi et al., J. Pharm. Esep. Ther. 125, 28 bis 34 (1959)) wird die tranquilisierende und anxciolytische Wirkung von Wirkstoffen untersucht. Dabei wird die Kampfaktivität von Mäusen, die mindestens 8 Tage isoliert gehalten wurden, nach Reizung mit elektrischen Fußschocks mit und ohne Verabreichung der erfindungsgemäßen substituierten basischen 2-Aminotetraline gemessen. Die 2-Aminotetraline hemmen die Kampfaktivität der Mäuse.

**Patentansprüche**
**Patentansprüche für folgende Vertragsstaaten : AT, BE, CH, DE, FR, GB, GR, IT, LI, NL, SE**

**1.** Substituierte basische 2-Aminotetraline der allgemeinen Formel

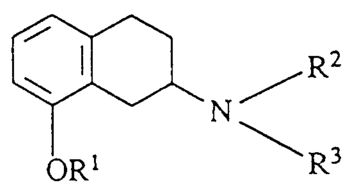

(I)

worin

$R^1$ – für Wasserstoff oder Methyl steht,

$R^2$ – für Wasserstoff, Methyl, Ethyl, Propyl, Isopropyl, Acetyl oder Propionyl steht,

und

$R^3$ – für Chinuclidin oder eine Gruppe der Formel $-(CH_2)_a-R^4$ , $-CH_2-CH=CH-(CH_2)_b-R^4$,

$-CH_2-C \equiv C-(CH_2)_b-R^4$,

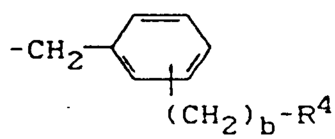 oder 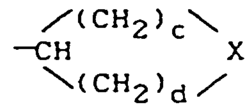

steht,
worin

a – eine Zahl 1 bis 6 bedeutet,
b – eine Zahl 0, 1 oder 2 bedeutet,
c – eine Zahl 1 oder 2 bedeutet,
d – die Zahl 2 bedeutet,
X – die Gruppe $NR^5$ bedeutet, wobei
$R^5$ – für Wasserstoff, Methyl, Ethyl, Propyl, Isopropyl, Butyl, Isobutyl, Phenyl, Benzyl, Methoxycarbonyl, Ethoxycarbonyl, Propoxycarbonyl, Isopropoxycarbonyl, Methylsulfonyl, Ethylsulfonyl oder Carbamoyl steht,
und
$R^4$ – Cyano oder
– eine Gruppe der Formel
$-OR^6$, $-COOR^7$, $-CONR^8R^9$, $-SO_mR^{10}$,
$-NR^{11}R^{12}$,

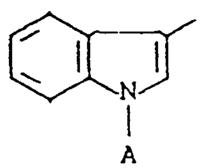 oder 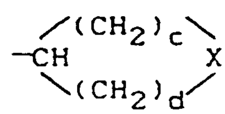

bedeutet,
wobei
c,d und X die oben angegebene Bedeutung haben,
A – für Wasserstoff, Methylsulfonyl, Phenylsulfonyl, Tolylsulfonyl Methoxycarbonyl oder Ethoxycarbonyl steht,
$R^6$ – für Wasserstoff, Methyl, Ethyl, Propyl, Isopropyl, Butyl, Isobutyl, Methoxycarbonyl, Ethoxycarbonyl, Propoxycarbonyl, Isopropoxycarbonyl, Butoxycarbonyl, Isobutoxycarbonyl, Tetrahydronaphthalin-1-yl oder 1,2,3-Benzothiadiazol-6-yl steht,
$R^7$ – für Wasserstoff, Methyl, Ethyl, Propyl, Isopropyl, Butyl oder Isobutyl steht,
$R^8$ und $R^9$ gleich oder verschieden sind
und
– für Wasserstoff, Methyl, Ethyl, Propyl, Isopropyl, Butyl oder Isobutyl stehen,
$R^{10}$ – für Methyl, Ethyl, Propyl, Isopropyl, Butyl, Isobutyl, oder
– für gegebenenfalls durch Fluor, Chlor, Methyl, Ethyl, Propyl oder Isopropyl substituiertes Phenyl steht,
m – für eine Zahl 0, 1 oder 2 steht,
$R^{11}$ und $R^{12}$ gleich oder verschieden sind,
und
– für Wasserstoff, Methyl, Ethyl, Propyl, Isopropyl, Butyl, Isobutyl, oder
– für gegebenenfalls durch Fluor, Chlor, Methyl oder Methoxy subsituiertes Phenyl, oder
– für eine Gruppe $-COR^{13}$ oder $-SO_2R^{14}$ stehen,
worin
$R^{13}$ – eine Gruppe $NHR^{15}$ bedeutet,
oder

- Methyl, Ethyl, Propyl, Isopropyl, Methoxy, Ethoxy, Propoxy, Isopropoxy, oder
- gegebenenfalls durch Methyl, Methoxy, Fluor oder Chlor substituiertes Phenyl, Benzyl, Benzyloxy, Thienyl, Furyl, Pyridyl, Pyrimidyl, Chinolyl oder Isochinolyl bedeutet,

$R^{14}$ – gegebenenfalls durch Fluor, Chlor, Methoxycarbonyl, Ethoxycarbonyl, Propoxycarbonyl, Isopropoxycarbonyl, Butoxycarbonyl oder Isobutoxycarbonyl substituiertes Methyl, Ethyl, Propyl, Isopropyl, Butyl oder Isobutyl bedeutet, oder
- gegebenenfalls durch Methyl, Ethyl, Propyl, Isopropyl, Methoxy, Fluor oder Chlor substituiertes Phenyl, Thienyl, Furyl, Pyridyl, Pyrimidyl, Chinolyl oder Isochinolyl bedeutet, oder
- eine Gruppe $NR^8R^9$ bedeutet,

wobei

$R^8$ und $R^9$ die oben angegebene Bedeutung haben,

und

$R^{15}$ – gegebenenfalls durch Fluor oder Chlor substituiertes Methyl, Ethyl, Propyl, Isopropyl, Butyl, Isobutyl, Pentyl, Isopentyl, Hexyl oder Isohexyl bedeutet, oder
- Phenyl bedeutet, das durch Fluor, Chlor, Methyl oder Methoxy substituiert sein kann,

oder

$R^{11}$ und $R^{12}$ gemeinsam mit dem Stickstoffatom einen Ring der Reihe

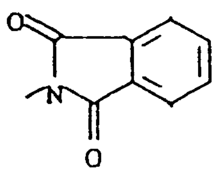, 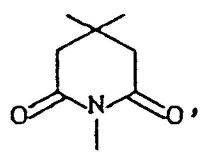, 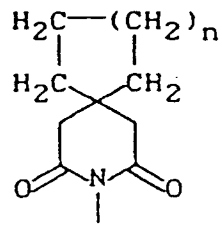,

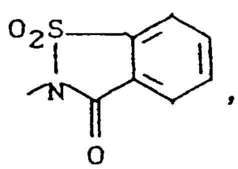, 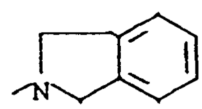, 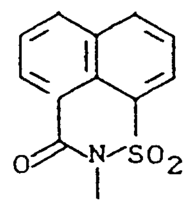

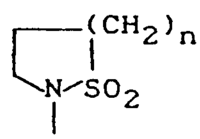, 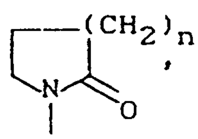, 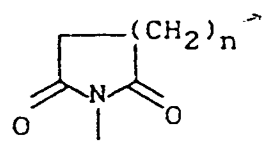

oder 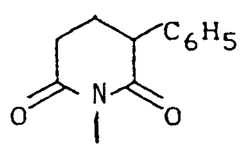

bilden, worin

n – eine Zahl 1 oder 2 bedeutet

oder

in welcher

$R^2$ und $R^3$ gemeinsam mit dem Stickstoffatom eine Gruppe der Formel

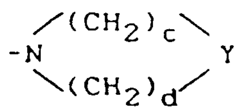

bilden,
worin
c und d die oben angegebene Bedeutung haben,
und
Y – eine Gruppe der Formel $NR^5$ oder $CH(CH_2)_e-NHR^5$ bedeutet,
wobei
$R^5$ die oben angegebene Bedeutung hat
und
e – für eine Zahl 1 oder 2 steht
wobei jedoch
$R^3$ nicht 3 – Hydroxypropyl bedeutet, wenn
$R^1$ – für Methyl und $R^2$ für Propyl steht,
$R^3$ nicht 2 – Methylthioethyl bedeutet, wenn
$R^1$ – für Wasserstoff oder Methyl und $R^2$ für Wasserstoff, Propyl oder Propionyl steht,
und
$R^2$ – nicht für Wasserstoff oder Methyl steht,
wenn
$R^1$ – für Wasserstoff oder Methyl steht und
$R^3$ – für einen Rest der Formel

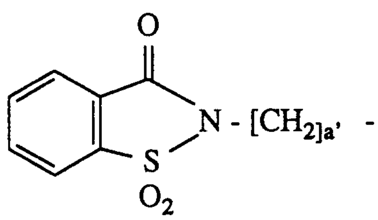

steht
worin
a' eine Zahl 2 bis 5 bedeutet,
und deren Salze.

**2.** Substituierte basische 2 – Aminotetraline nach Anspruch 1
worin
$R^1$ – für Wasserstoff oder Methyl steht,
$R^2$ – für Wasserstoff, Methyl, Propyl, Acetyl oder Propionyl steht,
und
$R^3$ – für Chinuclidin oder eine Gruppe der Formel
$-(CH_2)_a-R^4$ , $-CH_2-CH=CH-(CH_2)_b-R^4$,
$-CH_2-C\equiv C-(CH_2)_b-R^4$,

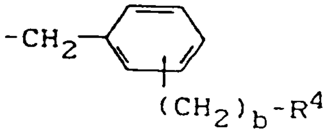

oder

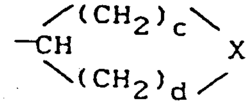

steht,
worin
a – eine Zahl 1 bis 6 bedeutet,

| | |
|---|---|
| b | – eine Zahl 0 oder 1 bedeutet, |
| c | – die Zahl 2 bedeutet, |
| d | – die Zahl 2 bedeutet, |
| x | – die Gruppe $NR^5$ bedeutet, |
| | wobei |
| $R^5$ | – für Wasserstoff, Methyl oder Methoxycarbonyl steht, |
| $R^4$ | – Cyano oder |
| | – eine Gruppe der Formel |
| | $-OR^6$, $-COOR^7$, $-CONR^8R^9$, $SO_mR^{10}$ oder $NR^{11}R^{12}$, |
| | 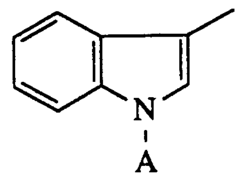 |
| | bedeutet, |
| | wobei |
| A | – für Wasserstoff oder Tolylsulfonyl steht, |
| $R^6$ | – für Wasserstoff, Methyl, Methoxycarbonyl, Ethoxycarbonyl, Tetrahydronaphthalin-1-yl oder 1,2,3-1,2,3-Benzothiadiazol-6-yl steht, |
| $R^7$ | – für Methyl oder Ethyl steht, |
| $R^8$ und $R^9$ | gleich oder verschieden sind und |
| | – für Wasserstoff, Methyl oder Ethyl stehen, |
| $R^{10}$ | – für Phenyl steht, |
| m | – für eine Zahl 0, 1 oder 2 steht, |
| $R^{11}$ und $R^{12}$ | gleich oder verschieden sind, |
| | und |
| | – für Wasserstoff, Methyl oder Ethyl, oder |
| | – für eine Gruppe $-COR^{13}$ oder $SO_2R^{14}$ stehen, |
| | worin |
| $R^{13}$ | – eine Gruppe $NHR^{15}$ bedeutet, |
| | oder |
| | Pyridyl oder Adamantyl bedeutet, |
| $R^{14}$ | – gegebenenfalls durch Chlor, Methoxycarbonyl oder Ethoxycarbonyl, substituiertes Methyl, Ethyl, Propyl, Isopropyl oder Butyl bedeutet, oder |
| | – gegebenenfalls durch Methyl, oder Fluor substituiertes Phenyl bedeutet, oder |
| | – eine Gruppe $NR^8R^9$ bedeutet, |
| | wobei |
| $R^8$ und $R^9$ | die oben angegebene Bedeutung haben, |
| | und |
| $R^{15}$ | – gegebenenfalls durch Chlor substituiertes Butyl oder Hexyl bedeutet, |
| | oder |
| $R^{11}$ und $R^{12}$ | gemeinsam mit dem Stickstoffatom einen Ring der Reihe |

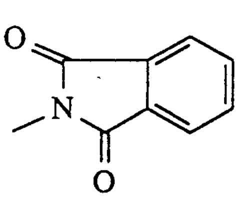 , 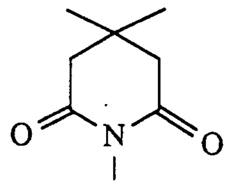 , 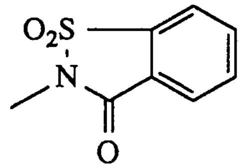 ,

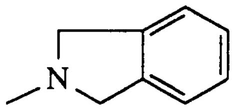 , 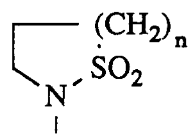 oder 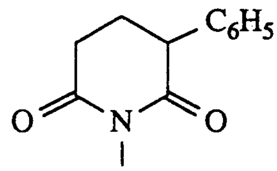

bilden,
worin

n – eine Zahl 1 oder 2 bedeutet, oder
in welcher

$R^2$ und $R^3$ gemeinsam mit dem Stickstoffatom eine Gruppe der Formel

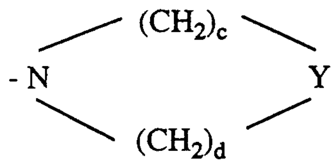

bilden,
worin

c und d die oben angegebene Bedeutung haben,
und

Y – eine Gruppe der Formel $NR^5$ oder $CH(CH_2)_e-NHR^5$ bedeutet,
wobei

$R^5$ die oben angegebene Bedeutung hat
und

e – für eine Zahl 1 oder 2 steht
wobei jedoch

$R^3$ nicht 3-Hydroxypropyl bedeutet, wenn

$R^1$ – für Methyl und $R^2$ für Propyl steht,

$R^3$ nicht 2-Methylthioethyl bedeutet, wenn

$R^1$ – für Wasserstoff oder Methyl und $R^2$ für Wasserstoff, Propyl oder Propionyl steht,
und

$R^2$ – nicht für Wasserstoff oder Methyl steht und
wenn

$R^1$ – für Wasserstoff oder Methyl steht und

$R^3$ – für einen Rest der Formel

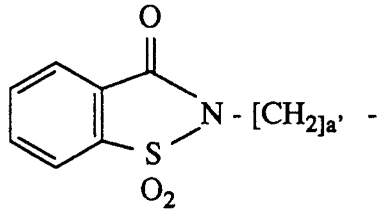

steht
worin
a' eine Zahl 2 bis 5 bedeutet,
und deren Salze.

**3.** 2 – {4 – [N – (8 – Methoxy – 1,2,3,4 – tetrahydro – 2 – naphthyl) – N – propyl – amino]butyl} – 1,2 – benzisothiazol – 3(2H) – on – 1,1 – dioxid und sein Salz.

**4.** 8 – Methoxy – 2 – {N – [2 – (4 – toluol – sulfonamido)ethyl] – N – propyl}amino – 1,2,3,4 – tetrahydronaphthalin Hydrochlorid und sein Salz.

**5.** 2 – [2 – N – propyl – N – (8 – methoxy – 1,2,3,4 – tetrahydro – 2 – naphthyl)amino]ethyl – perhydro – 1,2 – thiazin – 1,1 – dioxid Hydrochlorid und sein Salz.

**6.** 2 – {3 – [N – (8 – Methoxy – 1,2,3,4 – tetrahydronaphthalin – 2 – yl) – N – propyl – amino]propyl} – 1,2 – benzisothiazol – 3(2H) – on – 1,1 – dioxid Hydrochlorid und sein Salz.

**7.** 2 – {5 – [N – (8 – Methoxy – 1,2,3,4 – tetrahydronaphthalin – 2 – yl) – N – propyl – amino]pentyl} – 1,2 – benzisothiazol – 3(2H) – on – 1,1 – dioxid Hydrochlorid und sein Salz.

**8.** 2 – {2 – [N – (8 – Methoxy – 1,2,3,4 – tetrahydronaphthalin – 2 – yl) – N – propyl – amino]propyl} – 1,2 – benzisothiazol – 3(2H) – on – 1,1 – dioxid Hydrochlorid und sein Salz.

**9.** Verfahren zur Herstellung von substituierten basische 2 – Aminotetralinen nach Anspruch 1 und deren Salze, dadurch gekennzeichnet, daß man Tetralone der allgemeinen Formel (II)

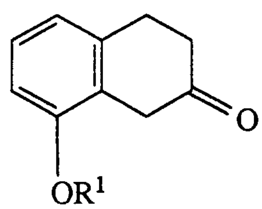

(II)

in welcher
$R^1$ die angegebene Bedeutung hat,
mit Aminen der allgemeinen Formel (III)

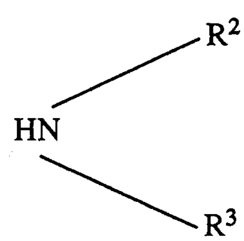

(III),

in welchen
$R^2$ und $R^3$ die angegebene Bedeutung haben,
$R^2$ jedoch nicht für Acyl steht,
in inerten Lösemitteln, gegebenenfalls in Anwesenheit von Hilfsstoffen umsetzt,
dann die erhaltenen Zwischenprodukte in inerten Lösemitteln reduziert,
dann im Fall der Herstellung der Acylverbindungen ($R^2$ = Acyl) mit einem Acylierungsmittel umsetzt,
dann gegebenenfalls fünktionelle Gruppen durch Reduktion, Hydrolyse, Oxidation oder Umsetzung mit elektrophilen Reagenzien in andere funktionelle Gruppen überführt,
und dann im Fall der Herstellung der Salze, mit der entsprechenden Säure umsetzt.

**10.** Verfahren nach Anspruch 4, dadurch gekennzeichnet, daß es als Eintopfverfahren durchgeführt wird.

**11.** Verfahren zur Herstellung von Verbindungen der Formel (Ia) nach Anspruch 1

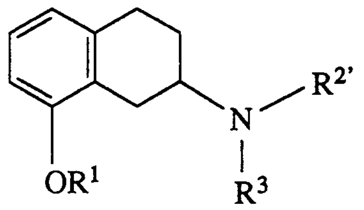

(Ia)

in welcher
$R^1$ und $R^3$ die in Anspruch 1 angegebene Bedeutung haben
und
$R^{2'}$ für Alkyl steht,
und deren Salze, dadurch gekennzeichnet, daß man
[A] alkylsubstituierte 2-Aminotetraline der allgemeinen Formel (Ib)

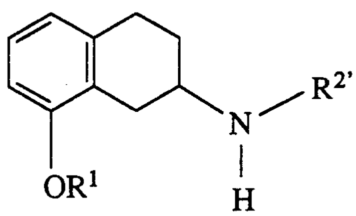

(Ib)

in welcher
$R^1$ und $R^{2'}$ die oben angegebene Bedeutung haben,
mit Halogenverbindungen der allgemeinen Formel (IV)

Hal - $R^3$ (IV)

in welcher
$R^3$ die oben angegebene Bedeutung hat
und
Hal - für Halogen, bevorzugt für Chlor, Brom oder Iod steht,
in inerten Lösemitteln, in Anwesenheit von Basen, gegebenenfalls in Gegenwart von Reaktionsbeschleunigern umsetzt,
dann gegebenenfalls funktionelle Gruppen durch Reduktion, Hydrolyse, Oxidation oder Umsetzung mit elektrophilen Reagenzien in andere funktionalle Gruppen überführt,
und dann im Fall der Herstellung der Salze mit entsprechenden Säuren umsetzt,
oder daß man
[B] monosubstituierte basische 2-Aminotetraline der allgemeinen Formel (Ic)

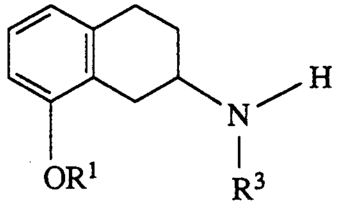

(Ic)

in welcher
$R^1$ und $R^3$ die oben angegebene Bedeutung haben,

mit Verbindungen der allgemeinen Formel (V)

$D - R^{2'}$ (V)

in welcher
$R^{2'}$ die oben angegebene Bedeutung hat,
und
D für einen Carbonylsauerstoff steht,
in inerten Lösemitteln, gegebenenfalls in Gegenwart eines Katalysators umsetzt,
dann die erhaltenen Zwischenprodukte in inerten Lösemitteln reduziert,
dann gegebenenfalls funktionelle Gruppen durch Reduktion, Hydrolyse, Oxidation oder Umsetzung mit elektrophilen Reagenzien in andere funktionelle Gruppen überführt und dann im Fall der Herstellung der Salze mit entsprechenden Säuren umsetzt.

**12.** Arzneimittel enthaltend substituierte basische 2-Aminotetraline nach Anspruch 1.

**13.** Arzneimittel nach Anspruch 12 zur Behandling von Erkrankungen des Zentralnervensystems.

**14.** Verwendung von substituierten basischen 2-Aminotetralinen nach Anspruch 1 zur Herstellung von Arzneimitteln.

**15.** Verwendung von substituierten basischen 2-Aminotetralinen nach Anspruch 1 zur Herstellung von Arzneimitteln zur Behandlung von Erkrankungen des Zentralnervensystems.

**Patentansprüche für folgenden Vertragsstaat : ES**

**1.** Verfahren zur Herstellung von substituierten basischen 2-Aminotetralinen der Formel

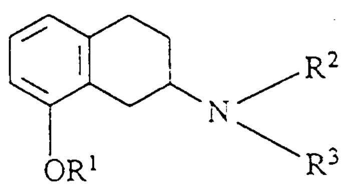

(I)

worin
$R^1$ - für Wasserstoff oder Methyl steht,
$R^2$ - für Wasserstoff, Methyl, Ethyl, Propyl, Isopropyl, Acetyl oder Propionyl steht,
und
$R^3$ - für Chinuclidin oder eine Gruppe der Formel
$-(CH_2)_a-R^4$ , $-CH_2-CH=CH-(CH_2)_b-R^4$,
$-CH_2-C\equiv C-(CH_2)_b-R^4$,

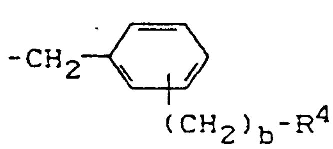

oder

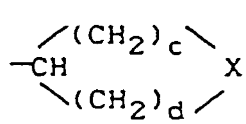

steht,
worin
a - eine Zahl 1 bis 6 bedeutet,
b - eine Zahl 0, 1 oder 2 bedeutet,
c - eine Zahl 1 oder 2 bedeutet,
d - die Zahl 2 bedeutet,
X - die Gruppe $NR^5$ bedeutet, wobei

$R^5$ – für Wasserstoff, Methyl, Ethyl, Propyl, Isopropyl, Butyl, Isobutyl, Phenyl, Benzyl, Methoxycarbonyl, Ethoxycarbonyl, Propoxycarbonyl, Isopropoxycarbonyl, Methylsulfonyl, Ethylsulfonyl oder Carbamoyl steht,

und

$R^4$ – Cyano oder

– eine Gruppe der Formel

$-OR^6$, $-COOR^7$, $-CONR^8R^9$, $-SO_mR^{10}$,

$-NR^{11}R^{12}$,

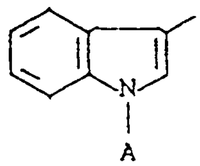

oder

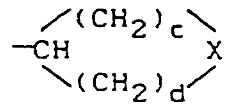

bedeutet,

wobei

c,d und X die oben angegebene Bedeutung haben,

A – für Wasserstoff, Methylsulfonyl, Phenylsulfonyl, Tolylsulfonyl Methoxycarbonyl oder Ethoxycarbonyl steht,

$R^6$ – für Wasserstoff, Methyl, Ethyl, Propyl, Isopropyl, Butyl, Isobutyl, Methoxycarbonyl, Ethoxycarbonyl, Propoxycarbonyl, Isopropoxycarbonyl, Butoxycarbonyl, Isobutoxycarbonyl, Tetrahydronaphthalin-1-yl oder 1,2,3-Benzothiadiazol-6-yl steht,

$R^7$ – für Wasserstoff, Methyl, Ethyl, Propyl, Isopropyl, Butyl oder Isobutyl steht,

$R^8$ und $R^9$ gleich oder verschieden sind

und

– für Wasserstoff, Methyl, Ethyl, Propyl, Isopropyl, Butyl oder Isobutyl stehen,

$R^{10}$ – für Methyl, Ethyl, Propyl, Isopropyl, Butyl, Isobutyl, oder

– für gegebenenfalls durch Fluor, Chlor, Methyl, Ethyl, Propyl oder Isopropyl substituiertes Phenyl steht,

m – für eine Zahl 0, 1 oder 2 steht,

$R^{11}$ und $R^{12}$ gleich oder verschieden sind,

und

– für Wasserstoff, Methyl, Ethyl, Propyl, Isopropyl, Butyl, Isobutyl, oder

– für gegebenenfalls durch Fluor, Chlor, Methyl oder Methoxy subsituiertes Phenyl, oder

– für eine Gruppe $-COR^{13}$ oder $-SO_2R^{14}$ stehen,

worin

$R^{13}$ – eine Gruppe $NHR^{15}$ bedeutet, oder

– Methyl, Ethyl, Propyl, Isopropyl, Methoxy, Ethoxy, Propoxy, Isopropoxy, oder

– gegebenenfalls durch Methyl, Methoxy, Fluor oder Chlor substituiertes Phenyl, Benzyl, Benzyloxy, Thienyl, Furyl, Pyridyl, Pyrimidyl, Chinolyl oder Isochinolyl bedeutet,

$R^{14}$ – gegebenenfalls durch Fluor, Chlor, Methoxycarbonyl, Ethoxycarbonyl, Propoxycarbonyl, Isopropoxycarbonyl, Butoxycarbonyl oder Isobutoxycarbonyl substituiertes Methyl, Ethyl, Propyl, Isopropyl, Butyl oder Isobutyl bedeutet, oder

– gegebenenfalls durch Methyl, Ethyl, Propyl, Isopropyl, Methoxy, Fluor oder Chlor substituiertes Phenyl, Thienyl, Furyl, Pyridyl, Pyrimidyl, Chinolyl oder Isochinolyl bedeutet, oder

– eine Gruppe $NR^8R^9$ bedeutet,

wobei

$R^8$ und $R^9$ die oben angegebene Bedeutung haben,

und

$R^{15}$ – gegebenenfalls durch Fluor oder Chlor substituiertes Methyl, Ethyl, Propyl, Isopropyl, Butyl, Isobutyl, Pentyl, Isopentyl, Hexyl oder Isohexyl bedeutet, oder

– Phenyl bedeutet, das durch Fluor, Chlor, Methyl oder Methoxy substituiert sein kann,

oder

$R^{11}$ und $R^{12}$ gemeinsam mit dem Stickstoffatom einen Ring der Reihe

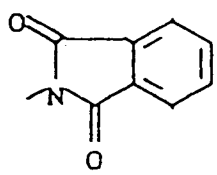, 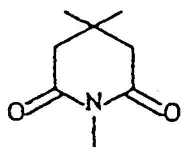, 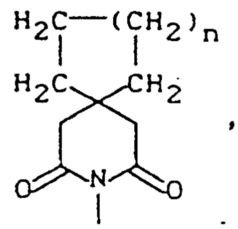,

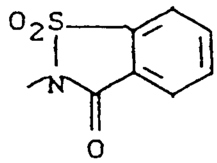, 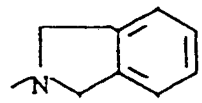, 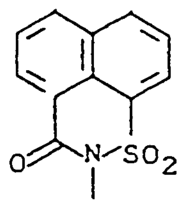

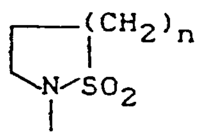, 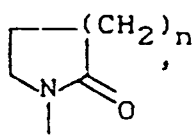, 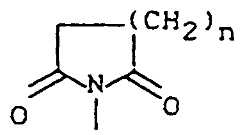

oder 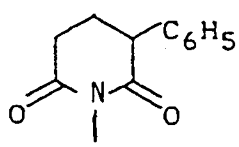

bilden, worin

n – eine Zahl 1 oder 2 bedeutet

oder

in welcher

$R^2$ und $R^3$ gemeinsam mit dem Stickstoffatom eine Gruppe der Formel

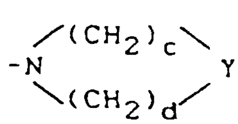

bilden, worin

c und d die oben angegebene Bedeutung haben,

und

Y – eine Gruppe der Formel $NR^5$ oder $CH(CH_2)_e-NHR^5$ bedeutet,

wobei

$R^5$ die oben angegebene Bedeutung hat

und

e – für eine Zahl 1 oder 2 steht

wobei jedoch

$R^3$ nicht 3-Hydroxypropyl bedeutet, wenn

$R^1$ – für Methyl und $R^2$ für Propyl steht,

$R^3$ nicht 2-Methylthioethyl bedeutet, wenn

$R^1$ – für Wasserstoff oder Methyl und $R^2$ für Wasserstoff, Propyl oder Propionyl steht,

und

$R^2$ – nicht für Wasserstoff oder Methyl steht,

wenn

$R^1$ – für Wasserstoff oder Methyl steht und
$R^3$ – für einen Rest der Formel

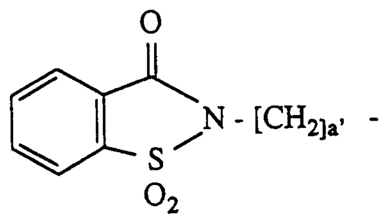

steht
worin
a' eine Zahl 2 bis 5 bedeutet,
und deren Salze, dadurch gekennzeichnet, daß man Tetralone der allgemeinen Formel

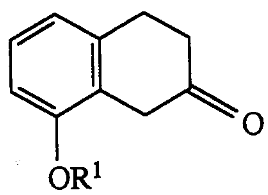

(II)

in welcher
$R^1$ – die angegebene Bedeutung hat,
mit Aminen der allgemeinen Formel (III)

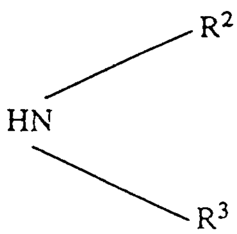

(III),

in welchen
$R^2$ und $R^3$ die angegebene Bedeutung haben,
$R^2$ jedoch nicht für Acyl steht,
in inerten Lösemitteln, gegebenenfalls in Anwesenheit von Hilfsstoffen umsetzt,
dann die erhaltenen Zwischenprodukte in inerten Lösemitteln reduziert,
dann im Fall der Herstellung der Acylverbindungen ($R^2$ = Acyl) mit einem Acylierungsmittel umsetzt,
dann gegebenenfalls funktionelle Gruppen durch Reduktion, Hydrolyse, Oxidation oder Umsetzung mit elektrophilen Reagenzien in andere funktionelle Gruppen überführt,
und dann im Fall der Herstellung der Salze, mit der entsprechenden Säure umsetzt.

**2.** Verfahren nach Anspruch 1, dadurch gekennzeichnet, daß es als Eintopfverfahren durchgeführt wird.

3. Verfahren zur Herstellung von Verbindungen der Formel (Ia) nach Anspruch 1

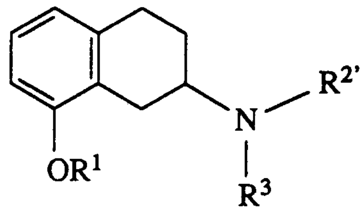 (Ia)

in welcher

$R^1$ und $R^3$ die in Anspruch 1 angegebene Bedeutung haben

und

$R^{2'}$ für Alkyl steht,

und deren Salze, dadurch gekennzeichnet, daß man

[A] alkylsubstituierte 2-Aminotetraline der allgemeinen Formel (Ib)

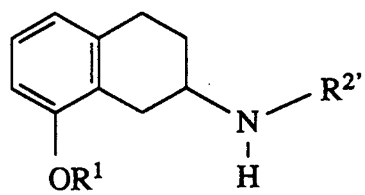 (Ib)

in welcher

$R^1$ und $R^{2'}$ die oben angegebene Bedeutung haben,

mit Halogenverbindungen der allgemeinen Formel (IV)

Hal - $R^3$ (IV)

in welcher

$R^3$ die oben angegebene Bedeutung hat

und

Hal - für Halogen, bevorzugt für Chlor, Brom oder Iod steht,

in inerten Lösemitteln, in Anwesenheit von Basen, gegebenenfalls in Gegenwart von Reaktionsbeschleunigern umsetzt,

dann gegebenenfalls funktionelle Gruppen durch Reduktion, Hydrolyse, Oxidation oder Umsetzung mit elektrophilen Reagenzien in andere funktionelle Gruppen überführt,

und dann im Fall der Herstellung der Salze mit entsprechenden Säuren umsetzt,

oder daß man

[B] monosubstituierte basische 2-Aminotetraline der allgemeinen Formel (Ic)

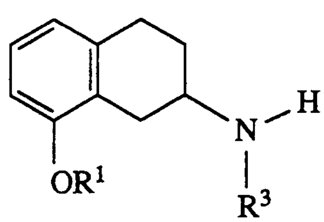 (Ic)

in welcher

$R^1$ und $R^3$ die oben angegebene Bedeutung haben,

mit Verbindungen der allgemeinen Formel (V)

D - $R^{2'}$ (V)

in welcher

$R^{2'}$ die oben angegebene Bedeutung hat,

und

D für einen Carbonylsauerstoff steht,

in inerten Lösemitteln, gegebenenfalls in Gegenwart eines Katalysators umsetzt, dann die erhaltenen Zwischenprodukte in inerten Lösemitteln reduziert, dann gegebenenfalls funktionelle Gruppen durch Reduktion, Hydrolyse, Oxidation oder Umsetzung mit elektrophilen Reagenzien in andere funktionelle Gruppen überführt und dann im Fall der Herstellung der Salze mit entsprechenden Säuren umsetzt.

**Claims**

**Claims for the following Contracting States : AT, BE, CH, DE, FR, GB, GR, IT, LI, NL, SE**

**1.** Substituted basic 2 – aminotetralins of the general formula

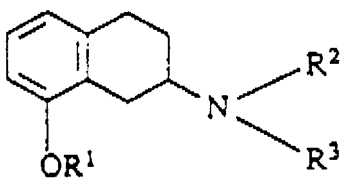

(I)

in which

$R^1$ represents hydrogen or methyl,

$R^2$ represents hydrogen, methyl, ethyl, propyl, isopropyl, acetyl or propionyl,

and

$R^3$ represents quinuclidine or a group of the formula

$-(CH_2)_a-R^4$, $-CH_2-CH=CH-(CH_2)_b-R^4$,

$-CH_2-C=C-(CH_2)_b-R^4$,

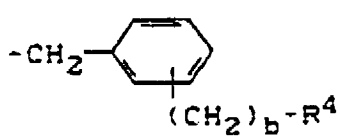

or

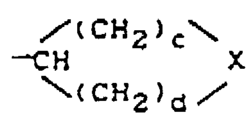

wherein

a – denotes a number from 1 to 6

b – denotes a number 0, 1 or 2,

c – denotes a number 1 or 2,

d – denotes the number 2,

x – denotes the group $NR^5$,

where

$R^5$ represents hydrogen, methyl, ethyl, propyl, isopropyl, butyl, isobutyl, phenyl, benzyl, methoxycarbonyl, ethoxycarbonyl, propoxycarbonyl, isopropoxycarbonyl, methylsulphonyl, ethylsulphonyl or carbamoyl,

and

$R^4$ denotes cyano or a group of the formula

$-OR^6$, $-COOR^7$, $-CONR^8R^9$, $-SO_mR^{10}$, $-NR^{11}R^{12}$,

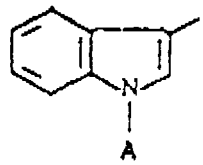 or 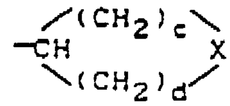

where
c, d and X have the abovementioned meaning,

A represents hydrogen, methylsulphonyl, phenylsulphonyl, tolylsulphonyl, methoxycarbonyl or ethoxycarbonyl,

$R^6$ represents hydrogen, methyl, ethyl, propyl, isopropyl, butyl, isobutyl, methoxycarbonyl, ethoxycarbonyl, propoxycarbonyl, isopropoxycarbonyl, butoxycarbonyl, isobutoxycarbonyl, tetrahydronaphthalen-1-yl or 1,2,3-benzothiadiazol-6-yl,

$R^7$ represents hydrogen, methyl, ethyl, propyl, isopropyl, butyl or isobutyl,

$R^8$ and $R^9$ are identical or different
and
represent hydrogen, methyl, ethyl, propyl, isopropyl, butyl, or isobutyl,

$R^{10}$ represents methyl, ethyl, propyl, isopropyl, butyl, isobutyl or represents phenyl which is optionally substituted by fluorine, chlorine, methyl, ethyl, propyl or isopropyl,

m represents a number 0, 1 or 2,

$R^{11}$ and $R^{12}$ are identical or different
and
represent hydrogen, methyl, ethyl, propyl, isopropyl, butyl, isobutyl,
or
represent phenyl which is optionally substituted by fluorine, chlorine, methyl or methoxy,
or
represent a group $-COR^{13}$ or $-SO_2R^{14}$,
wherein

$R^{13}$ denotes a group $NHR^{15}$,
or
denotes methyl, ethyl, propyl, isopropyl, methoxy, ethoxy, propoxy, isopropoxy or denotes phenyl, benzyl, benzyloxy, thienyl, furyl, pyridyl, pyrimidyl, quinolyl or isoquinolyl, each of which is optionally substituted by methyl, methoxy, fluorine or chlorine,

$R^{14}$ denotes methyl, ethyl, propyl, isopropyl, butyl or isobutyl, each of which is optionally substituted by fluorine, chlorine, methoxycarbonyl, ethoxycarbonyl, propoxycarbonyl, isopropoxycarbonyl, butoxycarbonyl or isobutoxycarbonyl, or denotes phenyl, thienyl, furyl, pyridyl, pyrimidyl, quinolyl or isoquinolyl, each of which is optionally substituted by methyl, ethyl, propyl, isopropyl, methoxy, fluorine or chlorine, or
denotes a group $NR^8R^9$,
where

$R^8$ and $R^9$ have the abovementioned meaning
and

$R^{15}$ denotes methyl, ethyl, propyl, isopropyl, butyl, isobutyl, pentyl, isopentyl, hexyl or isohexyl, each of which is optionally substituted by fluorine or chlorine,
or
denotes phenyl which may be substituted by fluorine, chlorine, methyl or methoxy,
or

$R^{11}$ and $R^{12}$, together with the nitrogen atom, form a ring from the series comprising

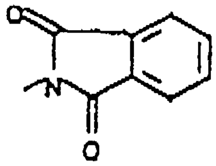,
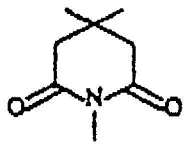,
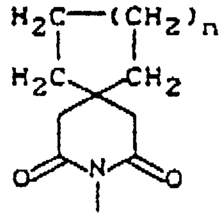,

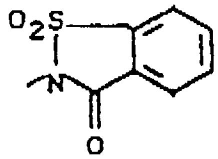,
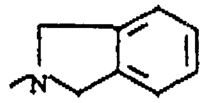,
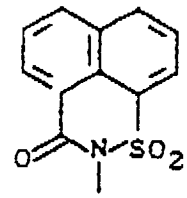

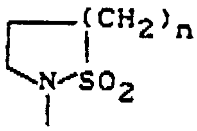,
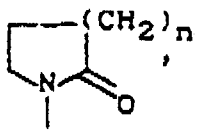,
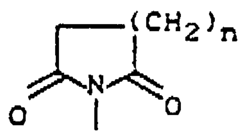
or
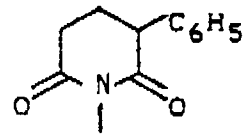

wherein
n denotes a number 1 or 2,

or
in which
$R^2$ and $R^3$, together with the nitrogen atom, form a group of the formula

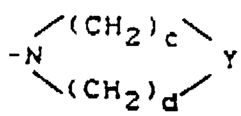

wherein
c and d have the abovementioned meaning,
and
Y denotes a group of the formula $NR^5$ or $CH(CH_2)_e-NHR^5$,
where
$R^5$ has the abovementioned meaning,
and
e represents a number 1 or 2
but where
$R^3$ does not denote 3-hydroxypropyl when
$R^1$ represents methyl and $R^2$ represents propyl,
$R^3$ does not denote 2-methylthioethyl when
$R^1$ represents hydrogen or methyl and $R^2$ represents hydrogen, propyl or propionyl,
and
$R^2$ does not represent hydrogen or methyl
when
$R^1$ represents hydrogen or methyl and
$R^3$ represents a radical of the formula

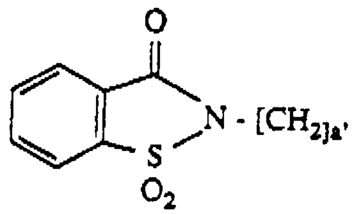

wherein

a' denotes a number from 2 to 5

and their salts.

**2.** Substituted basic 2-aminotetralins according to Claim 1, wherein

$R^1$ represents hydrogen or methyl.

$R^2$ represents hydrogen, methyl, propyl, acetyl or propionyl

and

$R^3$ represents quinuclidine or a group of the formula
$-(CH_2)_a-R^4$, $-CH_2-CH=CH-(CH_2)_b-R^4$,
$-CH_2-C=C-(CH_2)_b-R^4$,

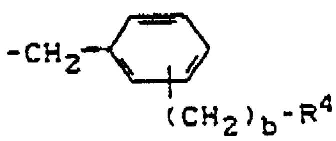

or

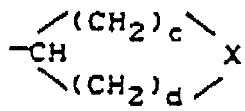

wherein

| | |
|---|---|
| a | denotes a number 1 to 6, |
| b | denotes a number 0 or 1 |
| c | denotes the number 2, |
| d | denotes the number 2, |
| x | denotes the group $NR^5$, where |
| $R^5$ | represents hydrogen, methyl or methoxycarbonyl, |
| $R^4$ | denotes cyano or a group of the formula $-OR^6$, $-COOR^7$, $-CONR^8R^9$, $-SO_mR^{10}$, $NR^{11}R^{12}$, or |

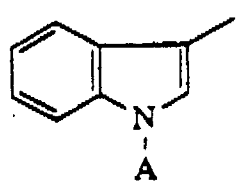

where

| | |
|---|---|
| A | represents hydrogen or tolylsulphonyl, |
| $R^6$ | represents hydrogen, methyl, methoxycarbonyl, ethoxycarbonyl, tetrahydronaphthalen-1-yl or 1,2,3-benzothiadiazol-6-yl, |
| $R^7$ | represents methyl or ethyl, |
| $R^8$ and $R^9$ | are identical or different and represent hydrogen, methyl or ethyl, |
| $R^{10}$ | represents phenyl, |
| m | represents a number 0, 1 or 2, |
| $R^{11}$ and $R^{12}$ | are identical or different and represent hydrogen, methyl or ethyl, or represent a group $-COR^{13}$ or $-SO_2R^{14}$, wherein |

$R^{13}$ denotes a group $NHR^{15}$,
or
pyridyl or adamantyl,
$R^{14}$ denotes methyl, ethyl, propyl, isopropyl or butyl which are optionally substituted by chlorine, methoxycarbonyl or ethoxycarbonyl, or denotes phenyl which is optionally substituted by methyl or fluorine, or denotes a group $NR^8R^9$,
where
$R^8$ and $R^9$ have the abovementioned meaning,
and
$R^{15}$ denotes butyl or hexyl which are optionally substituted by chlorine,
or
$R^{11}$ and $R^{12}$, together with nitrogen atom, form a ring from the series comprising

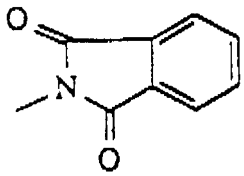 , 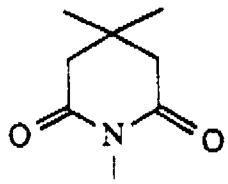 , 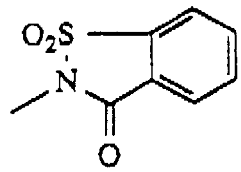 ,

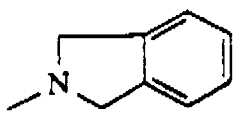 , 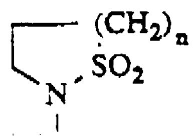 or 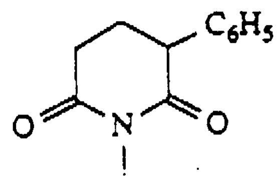 .

wherein
n denotes a number 1 or 2,
or in which
$R^2$ and $R^3$, together with the nitrogen atom, form a group of the formula

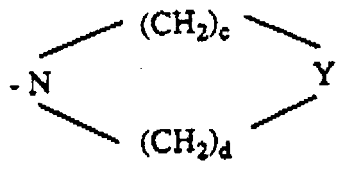

wherein
c and d have the abovementioned meaning,
and
Y denotes a group of the formula $NR^5$ or $CH(CH_2)_e-NHR^5$
where
$R^5$ has the abovmentioned meaning
and
e represents a number 1 or 2,
but where
$R^3$ does not denote 3-hydroxypropyl when
$R^1$ represents methyl and $R^2$ represents propyl,
$R^3$ does not denote 2-methylthioethyl when
$R^1$ represents hydrogen or methyl and $R^2$ represents hydrogen, propyl or propionyl,
and
$R^2$ does not represent hydrogen or methyl
when

$R^1$ represents hydrogen or methyl and
$R^3$ represents a radical of the formula

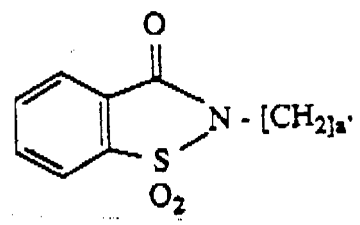

wherein
a' denotes a number from 2 to 5
and their salts.

3. 2-{4-[N-(8-Methoxy-1,2,3,4-tetrahydro-2-naphthyl)-N-propyl-amino]butyl}-1,2-benzisothiazol-3(2H)-one 1,1-dioxide and its salt.

4. 8-Methoxy-2-{N-[2-(4-toluenesulphonamido)ethyl]-N-propyl}amino-1,2,3,4-tetrahydronaphthalene hydrochloride and its salt.

5. 2-[2-N-propyl-N-(8-methoxy-1,2,3,4,-tetrahydro-2-naphthyl)amino]ethyl-perhydro-1,2-thiazine 1,1-dioxide hydrochloride and its salt.

6. 2-{3-[N-(8-Methoxy-1,2,3,4-tetrahydronaphthalene-2-yl)-N-propyl-amino]propyl}-1,2-benzisothiazol-3(2H)-one 1,1-dioxide hydrodichloride and its salt.

7. 2-{5-[N-(8-Methoxy-1,2,3,4-tetrahydronaphthalene-2-yl)-N-propyl-amino]pentyl}-1,2-benzisothiazol-3(2H)-one 1,1-dioxide hydrodichloride and its salt.

8. 2-{2-[N-(8-Methoxy-1,2,3,4-tetrahydronaphthalene-2-yl)-N-propyl-amino]propyl}-1,2-benzisothiazol-3(2H)-one 1,1-dioxide hydrochloride and its salt.

9. Process for the preparation of substituted basic 2-aminotetralins according to Claim 1 and their salts, characterised in that tetralones of the general formula (II)

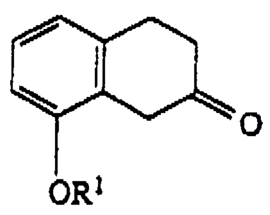

(II)

in which
$R^1$ has the meaning mentioned,
are reacted with amines of the general formula (III)

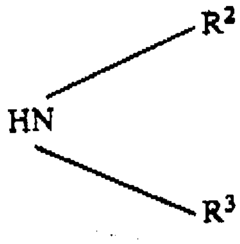

(III)

in which

$R^2$ and $R^3$ have the meaning mentioned, but

$R^2$ does not represent acyl,

in inert solvents, if appropriate in the presence of auxiliaries,
then the intermediates obtained are reduced in inert solvents,
then, in the case of the preparation of the acyl compounds ($R^2$ = acyl), are reacted with an acylating agent,
then, if appropriate, functional groups are converted into other functional groups by reduction, hydrolysis, oxidation or reaction with electrophilic reagents
and then, in the case of the preparation of the salts, the product is reacted with the appropriate acid.

**10.** Process according to Claim 4, characterised in that the reaction is carried out as a one-pot process.

**11.** Process for the preparation of compounds of the formula (Ia) according to Claim 1

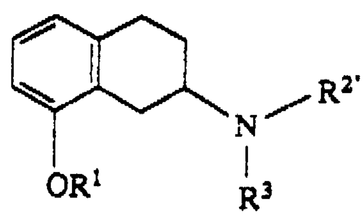

(Ia)

in which

$R^1$ and $R^3$ have the meaning given in Claim 1

and

$R^{2'}$ represents alkyl,

and their salts, characterised in that
[A] alkyl-substituted 2-aminotetralins of the general formula (Ib)

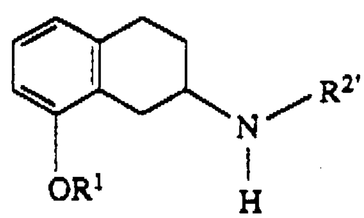

(Ib)

in which

$R^1$ and $R^{2'}$ have the abovementioned meaning,

are reacted with halogen compounds of the general formula (IV)

Hal - $R^3$ (IV)

in which

$R^3$ has the abovementioned meaning

and

Hal represents halogen, preferably chlorine, bromine or iodine

in inert solvents, in the presence of bases, if appropriate in the presence of reaction accelerators, if appropriate, functional groups are then converted into other functional groups by reduction, hydrolysis, oxidation or reaction with electrophilic reagents,

and then, in the case of the preparation of the salts, the product is reacted with appropriate acids,

or in that

[B] monosubstituted basic 2-aminotetralins of the general formula (Ic)

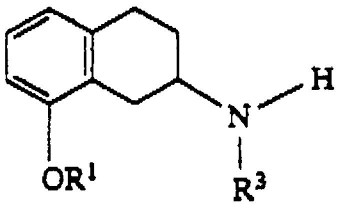

(Ic)

in which

$R^1$ and $R^3$ have the abovementioned meaning,

are reacted with compounds of the general formula (V)

D-$R^{2'}$ (V)

in which

$R^{2'}$ has the abovementioned meaning

and

D represents a carbonyl oxygen,

in inert solvents, if appropriate in the presence of a catalyst,

then the intermediates obtained are reduced in inert solvents,

then, if appropriate, functional groups are converted into other functional groups by reduction, hydrolysis, oxidation or reaction with electrophilic reagents and then, in the case of the preparation of the salts, the product is reacted with appropriate acids.

**12.** Medicaments containing substituted basic 2-aminotetralins according to Claim 1.

**13.** Medicaments according to Claim 12, for the treatment of diseases of the central nervous system.

**14.** Use of substituted basic 2-aminotetralins according to Claim 1 for the preparation of medicaments.

**15.** Use of substituted basic 2-aminotetralins according to Claim 1, for the preparation of medicaments for the treatment of diseases of the central nervous system.

**Claims for the following Contracting State : ES**

**1.** Process for the preparation of substituted basic 2-aminotetralins of the formula

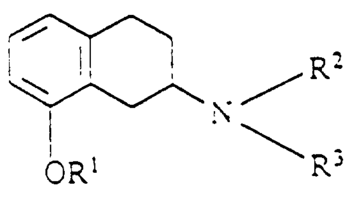

(I)

in which

$R^1$ represents hydrogen or methyl,

$R^2$ represents hydrogen, methyl, ethyl, propyl, isopropyl, acetyl or propionyl,

and

$R^3$ represents quinuclidine or a group of the formula
$-(CH_2)_a-R^4$, $-CH_2-CH=CH-(CH_2)_b-R^4$,
$-CH_2-C\equiv C-(CH_2)_b-R^4$,

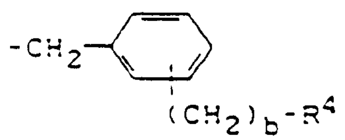

or

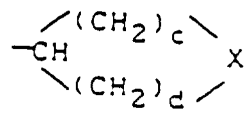

wherein

a – denotes a number from 1 to 6
b – denotes a number 0, 1 or 2,
c – denotes a number 1 or 2,
d – denotes the number 2,
x – denotes the group $NR^5$,

where

$R^5$ represents hydrogen, methyl, ethyl, propyl, isopropyl, butyl, isobutyl, phenyl, benzyl, methoxycarbonyl, ethoxycarbonyl, propoxycarbonyl, isopropoxycarbonyl, methylsulphonyl, ethylsulphonyl or carbamoyl,

and

$R^4$ denotes cyano or
a group of the formula
$-OR^6$, $-COOR^7$, $-CONR^8R^9$, $-SO_mR^{10}$, $-NR^{11}R^{12}$,

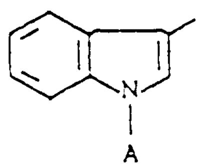

or

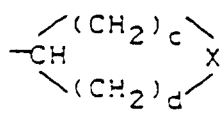

where
c, d and X have the abovementioned meaning,

A represents hydrogen, methylsulphonyl, phenylsulphonyl, tolylsulphonyl, methoxycarbonyl or ethoxycarbonyl,

$R^6$ represents hydrogen, methyl, ethyl, propyl, isopropyl, butyl, isobutyl, methoxycarbonyl, ethoxycarbonyl, propoxycarbonyl, isopropoxycarbonyl, butoxycarbonyl, isobutoxycarbonyl, tetrahydronaphthalen-1-yl or 1,2,3-benzothiadiazol-6-yl,

$R^7$ represents hydrogen, methyl, ethyl, propyl, isopropyl, butyl or isobutyl,

$R^8$ and $R^9$ are identical or different
and
represent hydrogen, methyl, ethyl, propyl, isopropyl, butyl, or isobutyl,

$R^{10}$ represents methyl, ethyl, propyl, isopropyl, butyl, isobutyl or represents phenyl which is optionally substituted by fluorine, chlorine, methyl, ethyl, propyl or isopropyl,

m represents a number 0, 1 or 2,

$R^{11}$ and $R^{12}$ are identical or different
and
represent hydrogen, methyl, ethyl, propyl, isopropyl, butyl, isobutyl,
or
represent phenyl which is optionally substituted by fluorine, chlorine, methyl or methoxy,
or
represent a group $-COR^{13}$ or $-SO_2R^{14}$,

wherein

$R^{13}$ denotes a group $NHR^{15}$,
or denotes methyl, ethyl, propyl, isopropyl, methoxy, ethoxy, propoxy, isopropoxy or denotes phenyl, benzyl, benzyloxy, thienyl, furyl, pyridyl, pyrimidyl, quinolyl or isoquinolyl, each of which is optionally substituted by methyl, methoxy, fluorine or chlorine,

$R^{14}$ denotes methyl, ethyl, propyl, isopropyl, butyl or isobutyl, each of which is optionally substituted by fluorine, chlorine, methoxycarbonyl, ethoxycarbonyl, propoxycarbonyl, isopropoxycarbonyl, butoxycarbonyl or isobutoxycarbonyl, or denotes phenyl, thienyl, furyl, pyridyl, pyrimidyl, quinolyl or isoquinolyl, each of which is optionally substituted by methyl, ethyl, propyl, isopropyl, methoxy, fluorine or chlorine, or
denotes a group $NR^8R^9$,
where

$R^8$ and $R^9$ have the abovementioned meaning
and

$R^{15}$ denotes methyl, ethyl, propyl, isopropyl, butyl, isobutyl, pentyl, isopentyl, hexyl or isohexyl, each of which is optionally substituted by fluorine or chlorine,
or
denotes phenyl which may be substituted by fluorine, chlorine, methyl or methoxy,
or

$R^{11}$ and $R^{12}$, together with the nitrogen atom, form a ring from the series comprising

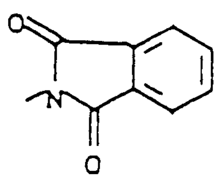 , 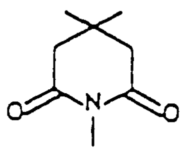 , 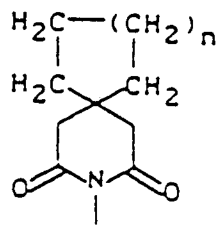 ,

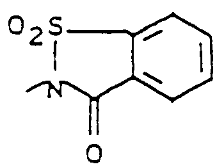 , 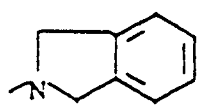 , 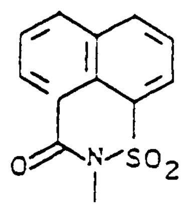

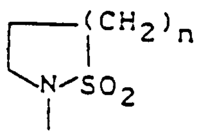 , 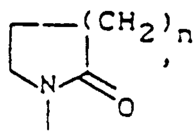 , 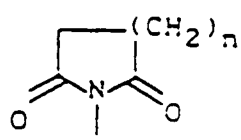 or 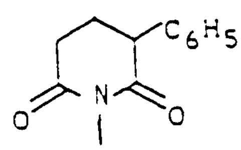

wherein

n denotes a number 1 or 2,

or

in which

$R^2$ and $R^3$, together with the nitrogen atom, form a group of the formula

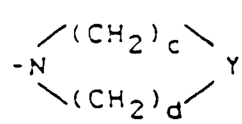

wherein

c and d have the abovementioned meaning,

and

Y denotes a group of the formula $NR^5$ or $CH(CH_2)_e-NHR^5$, where

$R^5$ has the abovementioned meaning, and

e represents a number 1 or 2

but where

$R^3$ does not denote 3-hydroxypropyl when

$R^1$ represents methyl and $R^2$ represents propyl,

$R^3$ does not denote 2-methylthioethyl when

$R^1$ represents hydrogen or methyl and $R^2$ represents hydrogen, propyl or propionyl,

and

$R^2$ does not represent hydrogen or methyl

when

$R^1$ represents hydrogen or methyl and

$R^3$ represents a radical of the formula

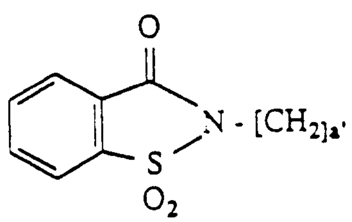

wherein

a' denotes a number from 2 to 5

and their salts, which is characterised in that tetralones of the general formula (II)

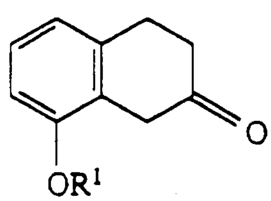

(II)

in which

$R^1$ has the meaning mentioned, are reacted with amines of the general formula (III)

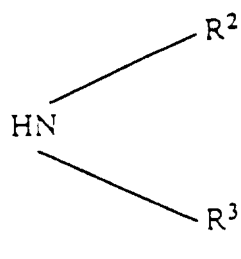

(III),

in which

$R^2$ and $R^3$ have the meaning mentioned, but

$R^2$ does not represent acyl,

in inert solvents, if appropriate in the presence of auxiliaries,

then the intermediates obtained are reduced in inert solvents,

then, in the case of the preparation of the acyl compounds ($R^2$ = acyl), are reacted with an acylating agent,
then, if appropriate, functional groups are converted into other functional groups by reduction, hydrolysis, oxidation or reaction with electrophilic reagents
and then, in the case of the preparation of the salts, the product is reacted with the appropriate acid.

**2.** Process according to Claim 1, characterised in that the reaction is carried out as a one-pot process.

**3.** Process for the preparation of compounds of the formula (Ia) according to Claim 1.

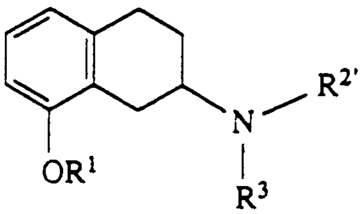

(Ia)

in which
$R^1$ and $R^3$ have the meaning given in Claim 1
and
$R^{2'}$ represents alkyl,
and their salts, characterised in that
[A] alkyl-substituted 2-aminotetralins of the general formula (Ib)

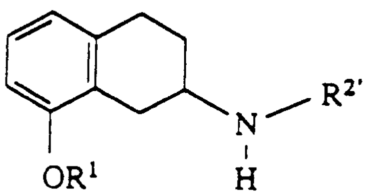

(Ib)

in which
$R^1$ and $R^{2'}$ have the abovementioned meaning,
are reacted with halogen compounds of the general formula (IV)

Hal-$R^3$ (IV)

in which
$R^3$ has the abovementioned meaning
and
Hal represents halogen, preferably chlorine, bromine or iodine
in inert solvents, in the presence of bases, if appropriate in the presence of reaction accelerators,
if appropriate, functional groups are then converted into other functional groups by reduction, hydrolysis, oxidation or reaction with electrophilic reagents,
and then, in the case of the preparation of the salts, the product is reacted with appropriate acids,
or
[B] monosubstituted basic 2-aminotetralins of the general formula (Ic)

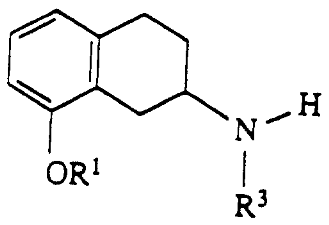

(Ic)

in which
R[1] and R[3] have the abovementioned meaning,
are reacted with compounds of the general formula (V)

$D-R^{2'}$ (V)

in which
$R^{2'}$ has the abovementioned meaning
and
D represents a carbonyl oxygen,
in inert solvents, if appropriate in the presence of a catalyst,
then the intermediates obtained are reduced in inert solvents,
then, if appropriate, functional groups are converted into other functional groups by reduction, hydrolysis, oxidation or reaction with electrophilic reagents and then, in the case of the preparation of the salts, the product is reacted with appropriate acids.

**Revendications**
**Revendications pour les Etats contractants suivants : AT, BE, CH, DE, FR, GB, GR, IT, LI, NL, SE**

**1.** 2-aminotétralines basiques substituées répondant à la formule générale

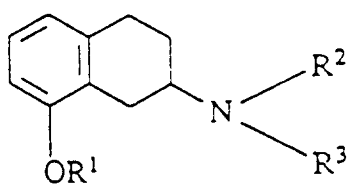

(I)

dans laquelle
$R^1$ représente un atome d'hydrogène ou un groupe méthyle,
$R^2$ représente un atome d'hydrogène, un groupe méthyle, un groupe éthyle, un groupe propyle, un groupe isopropyle, un groupe acétyle ou un groupe propionyle
et
$R^3$ représente la quinuclidine ou un groupe répondant aux formules
$-(CH_2)_a-R^4$, $-CH_2-CH=CH-(CH_2)_b-R^4$,
$-CH_2-C\equiv C-(CH_2)_b-R^4$,

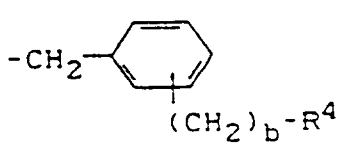

ou

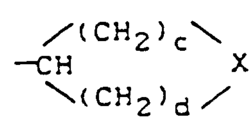

dans lesquelles
a - représente un nombre de 1 à 6,
b - représente le nombre 0, 1 ou 2,
c - représente le nombre 1 ou 2,

d – représente le nombre 2,

x – représente le groupe $NR^5$

dans lequel

$R^5$ représente un atome d'hydrogène, un groupe méthyle, un groupe éthyle, un groupe propyle, un groupe isopropyle, un groupe butyle, un groupe isobutyle, un groupe phényle, un groupe benzyle, un groupe méthoxycarbonyle, un groupe éthoxycarbonyle, un groupe propoxycarbonyle, un groupe isopropoxycarbonyle, un groupe méthylsulfonyle, un groupe éthylsulfonyle ou un groupe carbamoyle

et

$R^4$ représente un groupe cyano ou un groupe répondant aux formules
$-OR^6$, $-COOR^7$, $-CONR^8R^9$, $-SO_mR^{10}$, $-NR^{11}R^{12}$,

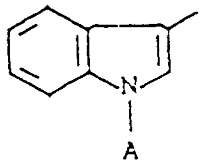

ou

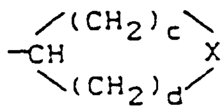

dans lesquelles
c, d et X ont la signification indiquée ci-dessus,

A représente un atome d'hydrogène, un groupe méthylsulfonyle, un groupe phénylsulfonyle, un groupe tolylsulfonyle, un groupe méthoxycarbonyle ou un groupe éthoxycarbonyle,

$R^6$ représente un atome d'hydrogène, un groupe méthyle, un groupe éthyle, un groupe propyle, un groupe isopropyle, un groupe butyle, un groupe isobutyle, un groupe méthoxycarbonyle, un groupe éthoxycarbonyle, un groupe propoxycarbonyle, un groupe isopropoxycarbonyle, un groupe butoxycarbonyle, un groupe isobutoxycarbonyle, un groupe tétrahydronaphtalén-1-yle, ou un groupe 1,2,3-benzothiadiazol-6-yle,

$R^7$ représente un atome d'hydrogène, un groupe méthyle, un groupe éthyle, un groupe propyle, un groupe isopropyle, un groupe butyle ou un groupe isobutyle,

$R^8$ et $R^9$ sont identiques ou différents et représentent un atome d'hydrogène, un groupe méthyle, un groupe éthyle, un groupe propyle, un groupe isopropyle, un groupe butyle ou un groupe isobutyle,

$R^{10}$ représente un groupe méthyle, un groupe éthyle, un groupe propyle, un groupe isopropyle, un groupe butyle, un groupe isobutyle ou
un groupe phényle éventuellement substitué par un atome de fluor, un atome de chlore, un groupe méthyle, un groupe éthyle, un groupe propyle ou un groupe isopropyle,

m représente le nombre 0, 1 ou 2,

$R^{11}$ et $R^{12}$ sont identiques ou différents et représentent un atome d'hydrogène, un groupe méthyle, un groupe éthyle, un groupe propyle, un groupe isopropyle, un groupe butyle, un groupe isobutyle ou un groupe phényle éventuellement substitué par un atome de fluor, un atome de chlore, un groupe méthyle ou un groupe méthoxy, ou encore
un groupe $-COR^{13}$ ou $-SO_2R^{14}$,
dans lesquels

$R^{13}$ représente un groupe $NHR^{15}$ ou un groupe méthyle, un groupe éthyle, un groupe propyle, un groupe isopropyle, un groupe méthoxy, un groupe éthoxy, un groupe propoxy, un groupe isopropoxy ou encore
un groupe phényle, un groupe benzyle, un groupe benzyloxy, un groupe thiényle, un groupe furyle, un groupe pyridyle, un groupe pyrimidyle, un groupe quinolyle ou un groupe isoquinolyle éventuellement substitués par un groupe méthyle, par un groupe méthoxy, par un atome de fluor ou par un atome de chlore.

$R^{14}$ représente un groupe méthyle, un groupe éthyle, un groupe propyle, un groupe isopropyle, un groupe butyle ou un groupe isobutyle éventuellement substitués par un

atome de fluor, un atome de chlore, un groupe méthoxycarbonyle, un groupe éthoxycarbonyle, un groupe propoxycarbonyle, un groupe isopropoxycarbonyle, un groupe butoxycarbonyle ou un groupe isobutoxycarbonyle, ou encore
un groupe phényle, un groupe thiényle, un groupe furyle, un groupe pyridyle, un groupe pyrimidyle, un groupe quinolyle ou un groupe isoquinolyle éventuellement substitués par un groupe méthyle, un groupe éthyle, un groupe propyle, un groupe isopropyle, un groupe méthoxy, par un atome de fluor ou par un atome de chlore ou encore
représente un groupe $NR^8R^9$
où
$R^8$ et $R^9$ ont la signification indiquée ci-dessus
et
$R^{15}$ représente un groupe méthyle, un groupe éthyle, un groupe propyle, un groupe isopropyle, un groupe butyle, un groupe isobutyle, un groupe pentyle, un groupe isopentyle, un groupe hexyle ou un groupe isohexyle éventuellement substitués par un atome de fluor ou par un atome de chlore ou encore
représente un groupe phényle qui peut être substitué par un atome de fluor, par un atome de chlore, par un groupe méthyle ou par un groupe méthoxy,
ou bien
$R^{11}$ et $R^{12}$, conjointement avec l'atome d'azote, forment un noyau de la série

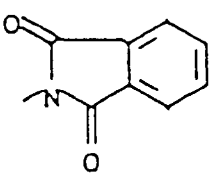 , 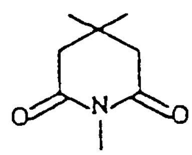 , 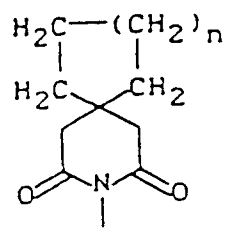 ,

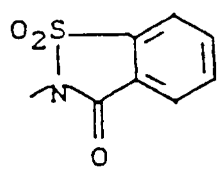 , 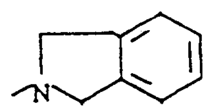 , 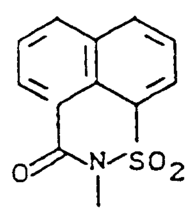

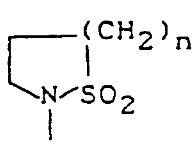 , 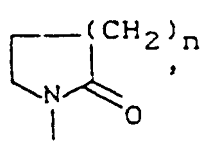 , 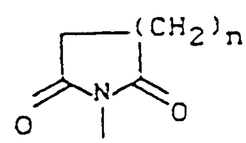

ou 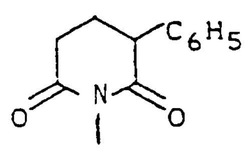

où
n représente le nombre 1 ou 2
ou bien
dans laquelle
$R^2$ et $R^3$, conjointement avec l'atome d'azote, forment un groupe de formule

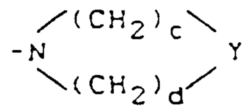

dans laquelle

c et d ont la signification indiquée ci-dessus et

Y représente un groupe de formule $NR^5$ ou $CH(CH_2)_e-NHR^5$ où

$R^5$ a la signification indiquée ci-dessus et

e représente le nombre 1 ou 2

dans laquelle, toutefois,

$R^3$ ne représente pas un groupe 3-hydroxypropyle lorsque

$R^1$ représente un groupe méthyle et $R^2$ représente un groupe propyle,

$R^3$ ne représente pas un groupe 2-méthylthioéthyle lorsque

$R^1$ représente un atome d'hydrogène ou un groupe méthyle et $R^2$ représente un atome d'hydrogène, un groupe propyle ou un groupe propionyle,

et

$R^2$ ne représente pas un atome d'hydrogène ou un groupe méthyle

lorsque

$R^1$ représente un atome d'hydrogène ou un groupe méthyle et

$R^3$ représente un radical de formule

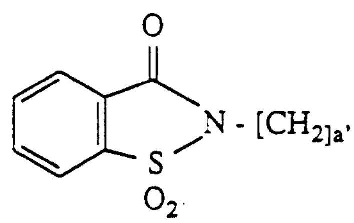

dans laquelle

a' représente un nombre de 2 à 5,

ainsi que leurs sels.

**2.** 2-aminotétralines basiques substituées selon la revendication 1 dans lesquelles

$R^1$ représente un atome d'hydrogène ou un groupe méthyle

$R^2$ représente un atome d'hydrogène, un groupe méthyle, un groupe propyle, un groupe acétyle ou un groupe propionyle

et

$R^3$ représente la quinuclidine ou un groupe répondant aux formules $-(CH_2)_aR^4$, $-CH_2-CH=CH-(CH_2)_b-R^4$, $-CH_2-C{\equiv}C-(CH_2)_b-R^4$,

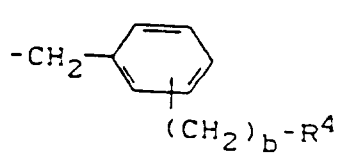

ou

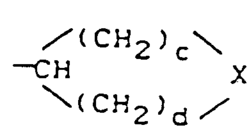

dans lesquelles

a - représente un nombre de 1 à 6,

b – représente le nombre 0 ou 1,

c – représente le nombre 2,

d – représente le nombre 2,

x – représente le groupe $NR^5$

où

$R^5$ représente un atome d'hydrogène, un groupe méthyle ou un groupe méthoxycarbonyle

$R^4$ représente un groupe cyano ou un groupe répondant aux formules $-OR^6$, $-COOR^7$, $-CONR^8R^9$, $-SO_mR^{10}$, $-NR^{11}R^{12}$,

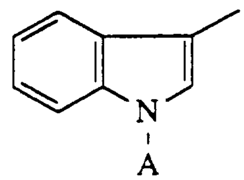

dans lesquelles

A représente un atome d'hydrogène ou un groupe tolylsulfonyle,

$R^6$ représente un atome d'hydrogène, un groupe méthyle, un groupe méthoxycarbonyle, un groupe éthoxycarbonyle, un groupe tétrahydronaphtalén-1-yle ou un groupe 1,2,3-benzothiadiazol-6-yle,

$R^7$ représente un groupe méthyle ou un groupe éthyle,

$R^8$ et $R^9$ sont identiques ou différents et représentent un atome d'hydrogène, un groupe méthyle ou un groupe éthyle,

$R^{10}$ représente un groupe phényle,

m représente le nombre 0, 1 ou 2,

$R^{11}$ et $R^{12}$ sont identiques ou différents et représentent un atome d'hydrogène, un groupe méthyle ou un groupe éthyle ou représentent un groupe $-COR^{13}$ ou $SO_2R^{14}$,

où

$R^{13}$ représente un groupe $NHR^{15}$

ou

un groupe pyridyle ou un groupe adamantyle,

$R^{14}$ représente un groupe méthyle, un groupe éthyle, un groupe propyle, un groupe isopropyle ou un groupe butyle éventuellement substitués par un atome de chlore, par un groupe méthoxycarbonyle ou par un groupe éthoxycarbonyle, ou bien

représente un groupe phényle éventuellement substitué par un groupe méthyle ou par un atome de fluor, ou bien

représente un groupe $NR^8R^9$,

où

$R^8$ et $R^9$ ont la signification indiquée ci-dessus,

et

$R^{15}$ représente un groupe butyle ou un groupe hexyle éventuellement substitué par un atome de chlore

ou bien

$R^{11}$ et $R^{12}$, conjointement avec l'atome d'azote, forment un noyau de la série

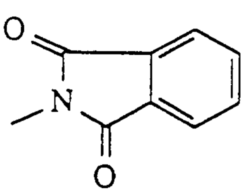 , 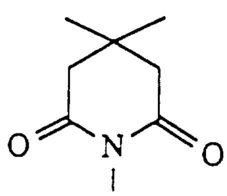 , 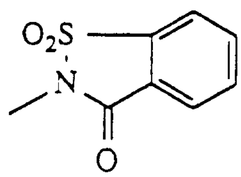 ,

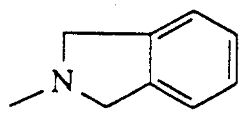 , 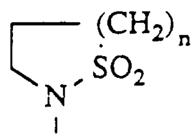 ou 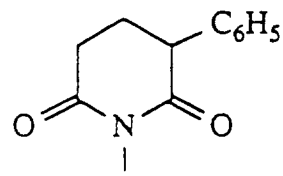

où

n représente le nombre 1 ou 2

ou bien

dans laquelle

$R^2$ et $R^3$, conjointement avec l'atome d'azote, forment un groupe de formule

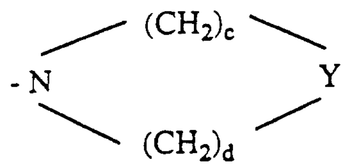

dans laquelle

c et d ont la signification indiquée ci-dessus

et

Y représente un groupe de formule $NR^5$ ou $CH(CH_2)_e-NHR^5$

où

$R^5$ a la signification indiquée ci-dessus

et

e représente le nombre 1 ou 2,

dans laquelle, toutefois,

$R^3$ ne représente pas un groupe 3-hydroxypropyle lorsque

$R^1$ représente un groupe méthyle et $R^2$ représente un groupe propyle,

$R^3$ ne représente pas un groupe 2-méthylthioéthyle lorsque

$R^1$ représente un atome d'hydrogène ou un groupe méthyle et

$R^2$ représente un atome d'hydrogène, un groupe propyle ou un groupe propionyle,

et

$R^2$ ne représente pas un atome d'hydrogène ou un groupe méthyle lorsque

$R^1$ représente un atome d'hydrogène ou un groupe méthyle et

$R^3$ représente un radical de formule

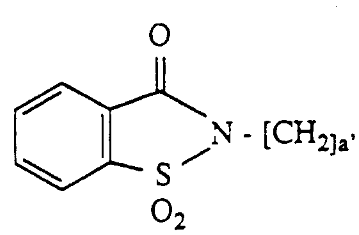

dans laquelle

a' représente un nombre de 2 à 5,

ainsi que leurs sels.

3. 1,1 – dioxyde de la 2 – {4[N – (8 – méthoxy – 1,2,3,4 – tétrahydro – 2 – naphtyl) – N – propyl – amino] – butyl} – 1,2 – benzisothiazol – 3(2H) – one et son sel.

4. Chlorhydrate de la 8 – méthoxy – 2 – {N – [2 – (4 – toluènesulfonamido)éthyl] – N – propyl}amino – 1,2,3,4 – tétrahydronaphtalène et son sel.

5. Chlorhydrate du 1,1 – dioxyde de la 2 – [2 – N – propyl – N – (8 – méthoxy – 1,2,3,4 – tétrahydro – 2 – naphtyl)amino]éthyl – perhydro – 1,2 – thiazine et son sel.

6. Chlorhydrate du 1,1 – dioxyde de la 2 – {3 – [N – (8 – méthoxy – 1,2,3,4 – tétrahydronaphtalén – 2 – yl) – N – propyl – amino]propyl} – 1,2 – benzisothiazol – 3(2H) – one et son sel.

7. Chlorhydrate du 1,1 – dioxyde de la 2 – {5 – [N – (8 – méthoxy – 1,2,3,4 – tétrahydronaphtalén – 2 – yl) – N – propyl – amino]pentyl} – 1,2 – benzisothiazol – 3(2H) – one et son sel.

8. Chlorhydrate du 1,1 – dioxyde de la 2 – {2 – [N – (8 – méthoxy – 1,2,3,4 – tétrahydronaphtalén – 2 – yl) – N – propyl – amino]propyl} – 1,2 – benzisothiazol – 3(2H) – one et son sel.

9. Procédé pour la préparation de 2 – aminotétralines basiques substituées selon la revendication 1, ainsi que de leurs sels, caractérisé en ce qu'on fait réagir des tétralones répondant à la formule générale (II)

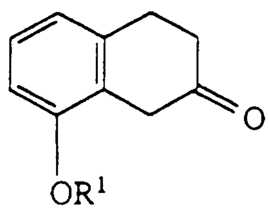

(II)

dans laquelle

$R^1$ a la signification indiquée ci – dessus,

avec des amines répondant à la formule générale (III)

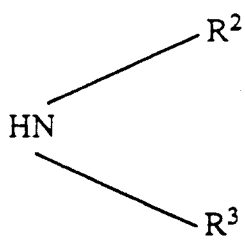

(III),

dans laquelle

$R^2$ et $R^3$ ont la signification indiquée ci – dessus,

$R^2$ ne représentant toutefois pas un groupe acyle,

dans des solvants inertes, éventuellement en présence de substances auxiliaires;

ensuite, on réduit dans des solvants inertes les produits intermédiaires obtenus

puis, dans le cas de la préparation des composés acylés ($R^2$ = acyle), on les fait réagir avec un agent d'acylation,

ensuite, on transforme éventuellement des groupes fonctionnels en d'autres groupes fonctionnels par réduction, hydrolyse, oxydation ou mise en réaction avec des réactifs électrophiles,

et ensuite, dans le cas de la préparation des sels, on les fait réagir avec les acides correspondants.

10. Procédé selon la revendication 4, caractérisé en ce qu'on l'effectue sous forme d'un procédé en un seul pot.

**11.** Procédé pour la préparation de composés de formule (Ia) selon la revendication 1

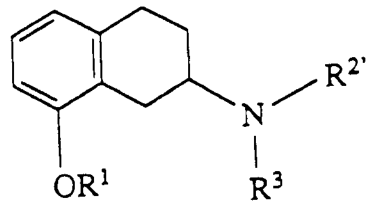

(Ia)

dans laquelle
$R^1$ et $R^3$ ont la signification indiquée à la revendication 1
et
$R^{2'}$ représente un groupe alkyle,
et de leurs sels, caractérisé en ce qu'on fait réagir
[A] des 2-aminotétralines substituées par un groupe alkyle répondant à la formule générale (Ib)

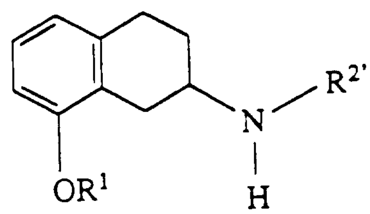

(Ib)

dans laquelle
$R^1$ et $R^{2'}$ ont la signification indiquée ci-dessus,
avec des composés halogénés répondant à la formule générale (IV)

Hal-$R^3$ (IV)

dans laquelle
$R^3$ a la signification indiquée ci-dessus
et
Hal représente un atome d'halogène, de préférence un atome de chlore, un atome de brome ou un atome d'iode,
dans des solvants inertes, en présence de bases et éventuellement en présence d'accélérateurs réactionnels,
ensuite, on transforme éventuellement des groupes fonctionnels en d'autres groupes fonctionnels par réduction, hydrolyse, oxydation ou mise en réaction avec des réactifs électrophiles,
et ensuite, dans le cas de la préparation des sels, on les fait réagir avec des acides correspondants,
ou bien en ce qu'on fait réagir
[B] des 2-aminotétralines basiques monosubstituées répondant à la formule générale (Ic)

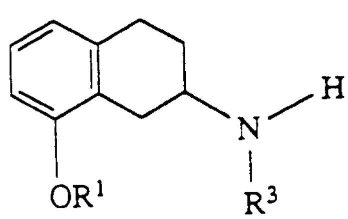

(Ic)

dans laquelle
$R^1$ et $R^3$ ont la signification indiquée ci-dessus,
avec des composés répondant à la formule générale (V)

$D-R^{2'}$ (V)

dans laquelle
$R^{2'}$ a la signification indiquée ci-dessus
et
D représente un groupe oxygène-carbonyle,
dans des solvants inertes, éventuellement en présence d'un catalyseur,
ensuite, on réduit dans des solvants inertes les produits intermédiaires obtenus,
ensuite, on transforme éventuellement des groupes fonctionnels en d'autres groupes fonctionnels par réduction, hydrolyse, oxydation ou mise en réaction avec des réactifs électrophiles et ensuite, en cas de préparation des sels, on les fait réagir avec des acides correspondants.

**12.** Médicament contenant des 2-aminotétralines basiques substituées selon la revendication 1.

**13.** Médicament selon la revendication 12, pour le traitement de maladies du système nerveux central.

**14.** Utilisation de 2-aminotétralines basiques substituées selon la revendication 1, pour la préparation de médicaments.

**15.** Utilisation de 2-aminotétralines basiques substituées selon la revendication 1, pour la préparation de médicaments pour le traitement de maladies du système nerveux central.

**Revendications pour l'Etat contractant suivant : ES**

**1.** Procédé pour la préparation de 2-aminotétralines basiques substituées répondant à la formule

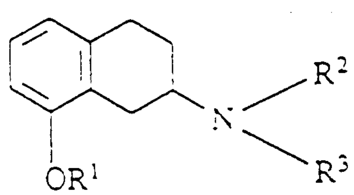

(I)

dans laquelle
$R^1$ représente un atome d'hydrogène ou un groupe méthyle,
$R^2$ représente un atome d'hydrogène, un groupe méthyle, un groupe éthyle, un groupe propyle, un groupe isopropyle, un groupe acétyle ou un groupe propionyle
et
$R^3$ représente la quinuclidine ou un groupe répondant aux formules
$-(CH_2)_a-R^4$, $-CH_2-CH=CH-(CH_2)_b-R^4$,
$-CH_2-C\equiv C-(CH_2)_b-R^4$,

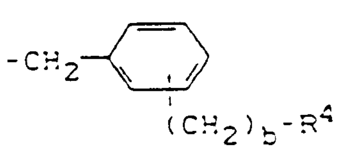

ou

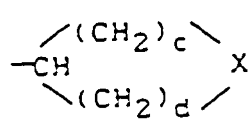

dans lesquelles
a - représente un nombre de 1 à 6,
b - représente le nombre 0, 1 ou 2,
c - représente le nombre 1 ou 2,
d - représente le nombre 2,
x - représente le groupe $NR^5$
dans lequel

$R^5$ représente un atome d'hydrogène, un groupe méthyle, un groupe éthyle, un groupe propyle, un groupe isopropyle, un groupe butyle, un groupe isobutyle, un groupe phényle, un groupe benzyle, un groupe méthoxycarbonyle, un groupe éthoxycarbonyle, un groupe propoxycarbonyle, un groupe isopropoxycarbonyle, un groupe méthylsulfonyle, un groupe éthylsulfonyle ou un groupe carbamoyle

et

$R^4$ représente un groupe cyano ou un groupe répondant aux formules
$-OR^6$, $-COOR^7$, $-CONR^8R^9$, $-SO_mR^{10}$, $-NR^{11}R^{12}$,

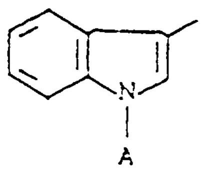

ou

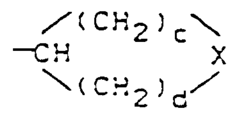

dans lesquelles
c, d et X ont la signification indiquée ci-dessus,

A représente un atome d'hydrogène, un groupe méthylsulfonyle, un groupe phénylsulfonyle, un groupe tolylsulfonyle, un groupe méthoxycarbonyle ou un groupe éthoxycarbonyle,

$R^6$ représente un atome d'hydrogène, un groupe méthyle, un groupe éthyle, un groupe propyle, un groupe isopropyle, un groupe butyle, un groupe isobutyle, un groupe méthoxycarbonyle, un groupe éthoxycarbonyle, un groupe propoxycarbonyle, un groupe isopropoxycarbonyle, un groupe butoxycarbonyle, un groupe isobutoxycarbonyle, un groupe tétrahydronaphtalén-1-yle, ou un groupe 1,2,3-benzothiadiazol-6-yle,

$R^7$ représente un atome d'hydrogène, un groupe méthyle, un groupe éthyle, un groupe propyle, un groupe isopropyle, un groupe butyle ou un groupe isobutyle,

$R^8$ et $R^9$ sont identiques ou différents et représentent un atome d'hydrogène, un groupe méthyle, un groupe éthyle, un groupe propyle, un groupe isopropyle, un groupe butyle ou un groupe isobutyle,

$R^{10}$ représente un groupe méthyle, un groupe éthyle, un groupe propyle, un groupe isopropyle, un groupe butyle, un groupe isobutyle ou
un groupe phényle éventuellement substitué par un atome de fluor, un atome de chlore, un groupe méthyle, un groupe éthyle, un groupe propyle ou un groupe isopropyle,

m représente le nombre 0, 1 ou 2,

$R^{11}$ et $R^{12}$ sont identiques ou différents et représentent un atome d'hydrogène, un groupe méthyle, un groupe éthyle, un groupe propyle, un groupe isopropyle, un groupe butyle, un groupe isobutyle ou
un groupe phényle éventuellement substitué par un atome de fluor, un atome de chlore, un groupe méthyle ou un groupe méthoxy, ou encore
un groupe $-COR^{13}$ ou $-SO_2R^{14}$,
dans lesquels

$R^{13}$ représente un groupe $NHR^{15}$ ou un groupe méthyle, un groupe éthyle, un groupe propyle, un groupe isopropyle, un groupe méthoxy, un groupe éthoxy, un groupe propoxy, un groupe isopropoxy ou encore
un groupe phényle, un groupe benzyle, un groupe benzyloxy, un groupe thiényle, un groupe furyle, un groupe pyridyle, un groupe pyrimidyle, un groupe quinolyle ou un groupe isoquinolyle éventuellement substitués par un groupe méthyle, par un groupe méthoxy, par un atome de fluor ou par un atome de chlore.

$R^{14}$ représente un groupe méthyle, un groupe éthyle, un groupe propyle, un groupe isopropyle, un groupe butyle ou un groupe isobutyle éventuellement substitué par un atome de fluor, un atome de chlore, un groupe méthoxycarbonyle, un groupe éthoxycarbonyle, un groupe propoxycarbonyle, un groupe isopropoxycarbonyle, un groupe butoxycarbonyle ou un groupe isobutoxycarbonyle, ou encore

un groupe phényle, un groupe thiényle, un groupe furyle, un groupe pyridyle, un groupe pyrimidyle, un groupe quinolyle ou un groupe isoquinolyle éventuellement substitués par un groupe méthyle, un groupe éthyle, un groupe propyle, un groupe isopropyle, un groupe méthoxy, par un atome de fluor ou par un atome de chlore ou encore

représente un groupe $NR^8R^9$

où

$R^8$ et $R^9$ ont la signification indiquée ci-dessus

et

$R^{15}$ représente un groupe méthyle, un groupe éthyle, un groupe propyle, un groupe isopropyle, un groupe butyle, un groupe isobutyle, un groupe pentyle, un groupe isopentyle, un groupe hexyle ou un groupe isohexyle éventuellement substitués par un atome de fluor ou par un atome de chlore ou encore

représente un groupe phényle qui peut être substitué par un atome de fluor, par un atome de chlore, par un groupe méthyle ou par un groupe méthoxy,

ou bien

$R^{11}$ et $R^{12}$, conjointement avec l'atome d'azote, forment un noyau de la série

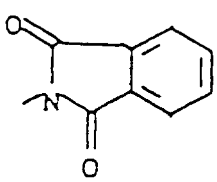,
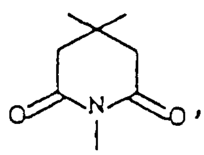,
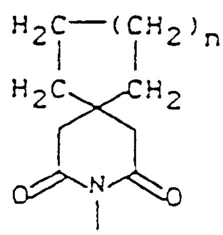,

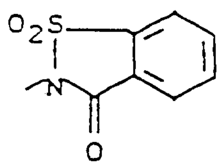,
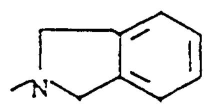,
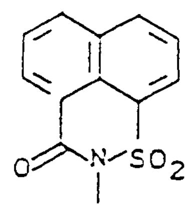

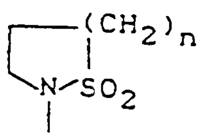,
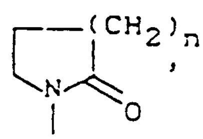,
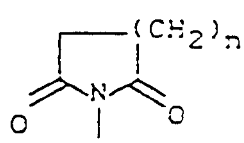

ou 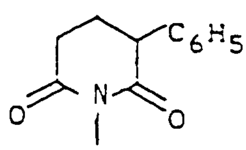

où

n représente le nombre 1 ou 2

ou bien

dans laquelle

$R^2$ et $R^3$, conjointement avec l'atome d'azote, forment un groupe de formule

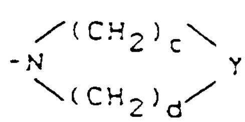

dans laquelle

c et d ont la signification indiquée ci-dessus et

Y représente un groupe de formule $NR^5$ ou $CH(CH_2)_e-NHR^5$ où

$R^5$ a la signification indiquée ci-dessus et

e représente le nombre 1 ou 2

dans laquelle, toutefois,

$R^3$ ne représente pas un groupe 3-hydroxypropyle lorsque

$R^1$ représente un groupe méthyle et $R^2$ représente un groupe propyle,

$R^3$ ne représente pas un groupe 2-méthylthioéthyle lorsque

$R^1$ représente un atome d'hydrogène ou un groupe méthyle et $R^2$ représente un atome d'hydrogène, un groupe propyle ou un groupe propionyle,

et

$R^2$ ne représente pas un atome d'hydrogène ou un groupe méthyle

lorsque

$R^1$ représente un atome d'hydrogène ou un groupe méthyle et

$R^3$ représente un radical de formule

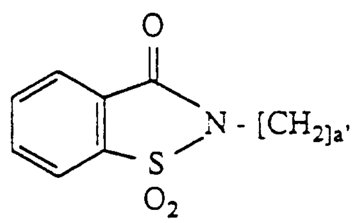

dans laquelle

a' représente un nombre de 2 à 5,

ainsi que de leurs sels, caractérisé en ce qu'on fait réagir des tétralones répondant à la formule générale

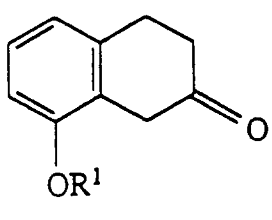

(II)

dans laquelle

$R^1$ a la signification indiquée ci-dessus,

avec des amines répondant à la formule générale (III)

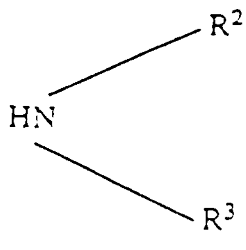

(III),

dans laquelle

$R^2$ et $R^3$ ont la signification indiquée ci-dessus,

$R^2$ ne représente toutefois pas un groupe acyle,
dans des solvants inertes, éventuellement en présence de substances auxiliaires;
ensuite, on réduit dans des solvants inertes les produits intermédiaires obtenus
puis, dans le cas de la préparation des composés acylés ($R^2$ = acyle), on les fait réagir avec un agent d'acylation,
ensuite, on transforme éventuellement des groupes fonctionnels en d'autres groupes fonctionnels par réduction, hydrolyse, oxydation ou mise en réaction avec des réactifs électrophiles,
et ensuite, dans le cas de la préparation des sels, on les fait réagir avec les acides correspondants.

**2.** Procédé selon la revendication 1, caractérisé en ce qu'on l'effectue sous forme d'un procédé en un seul pot.

**3.** Procédé pour la préparation de composés de formule (Ia) selon la revendication 1

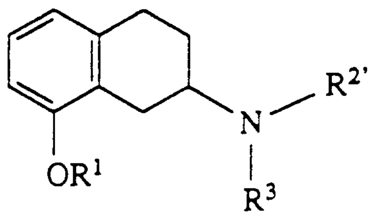
(Ia)

dans laquelle
$R^1$ et $R^3$ ont la signification indiquée à la revendication 1
et
$R^{2'}$ représente un groupe alkyle,
et de leurs sels, caractérisé en ce qu'on fait réagir
[A] des 2-aminotétralines substituées par un groupe alkyle répondant à la formule générale (Ib)

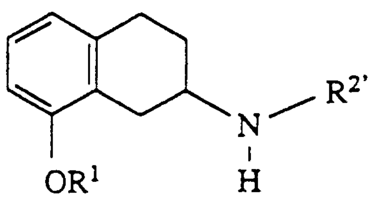
(Ib)

dans laquelle
$R^1$ et $R^{2'}$ ont la signification indiquée ci-dessus,
avec des composés halogénés répondant à la formule générale (IV)

Hal-$R^3$ (IV)

dans laquelle
$R^3$ a la signification indiquée ci-dessus
et
Hal représente un atome d'halogène, de préférence un atome de chlore, un atome de brome ou un atome d'iode,
dans des solvants inertes, en présence de bases, éventuellement en présence d'accélérateurs réactionnels,
ensuite, on transforme éventuellement des groupes fonctionnels en d'autres groupes fonctionnels par réduction, hydrolyse, oxydation ou mise en réaction avec des réactifs électrophiles,
et ensuite, dans le cas de la préparation des sels, on les fait réagir avec des acides correspondants,
ou bien en ce qu'on fait réagir
[B] des 2-aminotétralines basiques monosubstituées répondant à la formule générale (Ic)

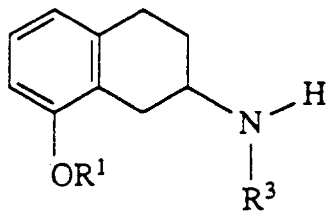

(Ic)

dans laquelle

$R^1$ et $R^3$ ont la signification indiquée ci-dessus,

avec des composés répondant à la formule générale (v)

$D-R^{2'}$ (V)

dans laquelle

$R^{2'}$ a la signification indiquée ci-dessus

et

D représente un groupe oxygène-carbonyle,

dans des solvants inertes, éventuellement en présence d'un catalyseur,

ensuite, on réduit dans des solvants inertes les produits intermédiaires obtenus,

ensuite, on transforme éventuellement des groupes fonctionnels en d'autres groupes fonctionnels par réduction, hydrolyse, oxydation ou mise en réaction avec des réactifs électrophiles et ensuite, en cas de préparation des sels, on les fait réagir avec des acides correspondants.